# EUROPEAN PATENT APPLICATION

(11) **EP 3 750 921 A1**
(43) Date of publication of application: **16.12.2020**
(21) Application number: 19747769.8
(22) Date of filing: 05.02.2019
(51) Int. Cl.: C07K 19/00, A61K 38/47, A61K 47/68, A61P 21/00, C07K 16/28, C07K 16/46, C12N 9/26, C12N 9/40, C12N 15/13, C12N 15/56, C12N 15/62

(54) **METHOD FOR DELIVERING DRUG TO MUSCLE**

(30) Priority: 05.02.2018 JP 2018018652
(71) Applicant: JCR Pharmaceuticals Co., Ltd., Ashiya-shi, Hyogo 659-0021 (JP)
(72) Inventor: TAKAHASHI, Kenichi, Kobe-shi, Hyogo 651-2241 (JP); SONODA, Hiroyuki, Kobe-shi, Hyogo 651-2241 (JP)
(74) Representative: Boult Wade Tennant LLP
(86) International application number: PCT/JP2019/004123
(87) International publication number: WO 2019/151539

(57) **Abstract**

[Problems] To provide a technique for efficiently incorporating an agent having to function in muscle tissue, which is not sufficiently incorporated into muscle tissue when administered in body, into muscle tissue, particularly muscle tissue composed of skeletal muscle or cardiac muscle. [Solution] A conjugate of an anti-human transferrin receptor antibody and an agent, wherein the agent is a biologically active agent that should function in muscle tissue, e.g., a lysosomal enzyme such as acid α-glucosidase, α-galactosidase A.

## Description

### Technical Field

The present invention relates to the delivery of a pharmaceutical agent to muscle, that agent shall exert its physiological activity in muscle, and more particularly to a conjugate comprising a desired pharmaceutical agent coupled to an anti-transferrin antibody, and a method of administering the conjugate to cause the desired agent to exert its bioactivity in muscle.

### Background Art

Recently, enzyme replacement therapy for lysosomal disease caused by functional deficiency of lysosomal enzymes has been established, and the patient's vital prognosis and quality of life (QOL) have improved. Drugs containing lysosomal enzymes as active ingredients used in enzyme replacement therapy include a drug for glycogen storage disease type 2 (Pompe disease) containing human acid α-glucosidase (hGAA) as the main agent, a drug for mucopolysaccharidosis type 2 (Hunter disease) containing human iduronate-2-sulfatase (hI2S) as the main agent, a drug for Fabry disease containing human α-galactosidase A (hα-GalA) as the main agent, and a drug for Gaucher disease containing human glucocerebrosidase as the main agent.

Lysosomal enzymes administered to patients in enzyme replacement therapy are taken up into cells and exert their functions. For example, human acid α-glucosidase (hGAA), human iduronate-2-sulfatase (hI2S), and human α-galactosidase A (hα-GalA), modified by an N-type sugar chain containing mannose-6-phosphate (M6P), are taken up into cells via binding of M6P to M6P receptor. Human glucocerebrosidase, a drug for Gaucher disease, is also modified by N-type sugar chain, whose reducing end is mannose, and is taken up into cells of the reticuloendothelial system, such as macrophages via binding of mannose to mannose receptors. Regarding the uptake into cells via binding of M6P and M6P receptors, a bis-phosphate type sugar chain containing two M6Ps have 100 times higher affinity for M6P receptor than a mono-phosphate type sugar chain containing one M6P. M6P contained in sugar chain greatly affects the efficiency of the cellular uptake of lysosomal enzymes.

The human lysosomal enzyme used in enzyme replacement therapy is a recombinant protein produced by expressing a gene encoding a human lysosomal enzyme, the gene introduced into a host cell such as a CHO cell using a gene recombination technology. Recombinant human lysosomal enzymes produced as recombinant proteins need to be appropriately modified by sugar-chain in order to exert their pharmaceutical effect. In other words, it is essential for hGAA, hα-GalA, and hI2S to be modified with the sugar chain containing mannose-6-phosphate, and for human glucocerebrosidase to be appropriately modified with the sugar chain whose reducing end is mannose, to exert their pharmaceutical effect.

In the case of recombinant human acid α-glucosidase (rhGAA), the main target organ where its pharmaceutical effects shall be exerted is muscle, particularly skeletal muscle. However, the number of M6P contained in sugar chains is 1 or less on average per molecule in rhGAA used for the enzyme replacement therapy, so that a large part of the sugar chains is mono-phosphate type, and the sugar-chain modification by M6P is not satisfactory (Non-Patent Document 1). Therefore, as rhGAA is not incorporated in the target organ inefficiently, the dose per administration is extremely high, i.e. 20mg/kg. Depending on the patient's symptoms, the dose may be increased to 40mg/kg, but still the enzyme administered is not considered to be fully functional.

A method using a ligand-receptor system other than M6P-M6P receptor has been developed as a method for efficient uptake of rhGAA into a target organ. For example, it is known that a fusion protein obtained by fusing RAP (receptor-associated protein), which is a ligand of a low-density lipoprotein receptor family (LDL receptor), and hGAA is taken up into cells in an M6P-independent manner in experiments using cultured cells (Non-Patent Document 2). According to this method, even rhGAA insufficiently modified by the sugar chain containing M6P can be taken up into cells via the binding of RAP and its receptor.

### Citation List

### Non Patent Literature

[Non-Patent Document 1] Zhu Y. et al., Biochem. J. 389, 619-28 (2005)
[Non-Patent Document 2] Prince WS. et al., J Biol. Chem. 279, 35037-46 (2004)

### Summary of Invention

### Problems to be Solved by the Invention

It is an objective of the present invention to provide a method for efficiently incorporating a pharmaceutical agent having to function in a muscle into a muscle when the pharmaceutical agent is administered in vivo, and its related art.

### Means for Solving the Problems

In the study directed to the above-mentioned purpose, as a result of intensive investigation, the present inventors have found that recombinant human acid α-glucosidase, which should exert its pharmaceutical effect in the muscle but is not sufficiently transported to the muscle as it is, is efficiently taken up into the muscle by conjugation with the anti-human transferrin receptor, and completed the present invention. Thus the present invention includes the following.
1. A conjugate of an anti-human transferrin receptor antibody and a pharmaceutical agent, wherein the agent has a physiological activity to be exerted in muscle.
2. The conjugate according to 1 above, wherein the anti-human transferrin receptor antibody is a Fab antibody, a F(ab')₂ antibody, or a F(ab') antibody.
3. The conjugate according to 1 above, wherein the anti-human transferrin receptor antibody is a single-chain antibody selected from the group consisting of scFab, scF(ab'), scF(ab')₂, and scFv.
4. The conjugate according to 3 above, wherein in the single-chain antibody a light chain and a heavy chain of the anti-human transferrin receptor antibody are linked via a linker sequence.
5. The conjugate according to 4 above, wherein the light chain and the heavy chain of the anti-human transferrin receptor antibody are linked on the C-terminal side of the light chain via the linker sequence.
6. The conjugate according to 4 above, wherein the light chain and the heavy chain of the anti-human transferrin receptor antibody are linked on the C-terminal side of the heavy chain via the linker sequence.
7. The conjugate according to any one of 4 to 6 above, wherein the linker sequence comprises 8 to 50 amino acid residues.
8. The conjugate according to 7 above, wherein the linker sequence comprises an amino acid sequence selected from the group consisting of the amino acid sequence (Gly Ser), the amino acid sequence (Gly Gly Ser), the amino acid sequence (Gly Gly Gly), the amino acid sequence set forth as SEQ ID NO: 3, and the amino acid sequence set forth as SEQ ID NO: 4.
9. The conjugate according to 8 above, wherein the linker sequence comprises 15 amino acid residues in which the amino acid sequence set forth as SEQ ID NO: 4 is consecutively repeated three times.
10. The conjugate according to any one of 1 to 9 above, wherein the anti-human transferrin receptor antibody comprises the amino acid sequence set forth as SEQ ID NO: 22 in the variable region of the light chain and the amino acid sequence set forth as SEQ ID NO: 23 in the variable region of the heavy chain.
11. The conjugate of 10 above, wherein the amino acid sequence of the variable region of the light chain has an identity not lower than 80% to the amino acid sequence set forth as SEQ ID NO: 22, and the amino acid sequence of the variable region of the heavy chain has an identity not lower than 80% to the amino acid sequence set forth as SEQ ID NO: 23.
12. The conjugate of 10 above, wherein the amino acid sequence of the variable region of the light chain has an identity not lower than 90% to the amino acid sequence set forth as SEQ ID NO: 22, and the amino acid sequence of the variable region of the heavy chain has an identity not lower than 90% to the amino acid sequence set forth as SEQ ID NO: 23.
13. The conjugate according to 10 above, wherein 1 to 10 amino acids constituting the variable region of the light chain are substituted with other amino acids relative to the amino acid sequence set forth as SEQ ID NO: 22.
14. The conjugate according to 10 above, wherein 1 to 3 amino acids constituting the variable region of the light chain are substituted with other amino acids relative to the amino acid sequence set forth as SEQ ID NO: 22.
15. The conjugate according to 10 above, wherein 1 to 10 amino acids constituting the variable region of the heavy chain are substituted with other amino acids relative to the amino acid sequence set forth as SEQ ID NO: 23.
16. The conjugate according to 10 above, wherein 1 to 3 amino acids constituting the variable region of the heavy chain are substituted with other amino acids relative to the amino acid sequence set forth as SEQ ID NO: 23.
17. The conjugate according to 10 above, wherein 1 to 10 amino acids constituting the variable region of the light chain are substituted with other amino acids relative to the amino acid sequence set forth as SEQ ID NO: 22, and 1 to 10 amino acids constituting the variable region of the heavy chain are substituted with other amino acids relative to the amino acid sequence set forth as SEQ ID NO: 23.
18. The conjugate according to 10 above, wherein 1 to 3 amino acids constituting the variable region of the light chain are substituted with other amino acids relative to the amino acid sequence set forth as SEQ ID NO: 22, and 1 to 3 amino acids constituting the variable region of the heavy chain are substituted with other amino acids relative to the amino acid sequence set forth as SEQ ID NO: 23.
19. The conjugate according to 10 above, wherein the anti-human transferrin receptor antibody comprises the light chain comprising an amino acid sequence set forth as SEQ ID NO: 24 and the heavy chain comprising an amino acid sequence set forth as SEQ ID NO: 25.
20. The conjugate according to 19 above, wherein the amino acid sequence of the light chain has an identity not lower than 80% to the amino acid sequence set forth as SEQ ID NO: 24, and the amino acid sequence of the heavy chain has an identity not lower than 80% to the amino acid sequence set forth as SEQ ID NO: 25.
21. The conjugate according to 19 above, wherein the amino acid sequence of the light chain has an identity not lower than 90% to the amino acid sequence set forth as SEQ ID NO: 24, and the amino acid sequence of the heavy chain has an identity not lower than 90% to the amino acid sequence set forth as SEQ ID NO: 25.
22. The conjugate of any of 1 to 21 above, wherein the agent is a peptide or a protein.
23. The conjugate according to 22 above selected from the group consisting of (1) to (4) below:
   (1) a conjugate in which the protein is linked to the C-terminal side of the light chain of the anti-human transferrin receptor antibody by a peptide bond,
   (2) a conjugate in which the protein is linked to the N-terminal side of the light chain of the anti-human transferrin receptor antibody by a peptide bond,
   (3) a conjugate in which the protein is linked to the C-terminal side of the heavy chain of the anti-human transferrin receptor antibody by a peptide bond, and
   (4) a conjugate in which the protein is linked to the N-terminal side of the heavy chain of the anti-human transferrin receptor antibody by a peptide bond.
24. The conjugate according to 23 above, wherein the protein is linked to the anti-human transferrin receptor antibody via a linker sequence.
25. The conjugate according to 24 above, wherein the linker sequence consists of 1 to 50 amino acid residues.
26. The conjugate according to 25 above, wherein the linker sequence comprises an amino acid sequence selected from the group consisting of a single glycine, a single serine, the amino acid sequence (Gly Ser), the amino acid sequence (Gly Gly Ser), the amino acid sequence set forth as SEQ ID NO:3, the amino acid sequence set forth as SEQ ID NO:4, and the amino acid sequence consisting of 1 to 10 amino acid sequences thereof that are linked consecutively.
27. The conjugate according to any one of 1 to 28 above, wherein the anti-human transferrin receptor antibody is a humanized anti-human transferrin receptor antibody.
28. The conjugate according to any one of 22 to 27 above, wherein the protein is a lysosomal enzyme.
29. The conjugate according to 28 above, wherein the lysosomal enzyme is human α-galactosidase A.
30. The conjugate according to 29 above, wherein the light chain of the humanized anti-hTfR antibody comprises the amino acid sequence set forth as SEQ ID NO: 24, and wherein the heavy chain of the humanized anti-hTfR antibody is linked on its C-terminal side to the human α-galactosidase A via a linker sequence (Gly Ser), and the whole linked heavy chain has the amino acid sequence set forth as SEQ ID NO: 29.
31. The conjugate according to 28 above, wherein the lysosomal enzyme is human acid α-glucosidase.
32. The conjugate according to 31 above, wherein the light chain of the humanized anti-hTfR antibody comprises the amino acid sequence set forth as SEQ ID NO: 24, and wherein the heavy chain of the humanized anti-hTfR antibody is linked on its C-terminal side to the human acid α-glucosidase via a linker sequence (Gly Ser), and the whole linked heavy chain has the amino acid sequence set forth as SEQ ID NO: 27.
33. A pharmaceutical composition for improving a muscle function comprising the conjugate according to any one of 1 to 32 above.
34. A pharmaceutical composition for ameliorating muscle dysfunction associated with lysosomal disease, comprising the conjugate according to 28 above.
35. A pharmaceutical composition for ameliorating muscle dysfunction associated with Fabry disease, comprising the conjugate according to 29 or 30 above.
36. A pharmaceutical composition for ameliorating muscle dysfunction associated with Pompe disease, comprising the conjugate according to 31 or 32 above.
37. The pharmaceutical composition according to any one of 33 to 36 above, wherein the muscle is skeletal muscle, cardiac muscle, or smooth muscle.
38. Use of the conjugate according to any one of 28 to 32 above for the manufacture of a pharmaceutical composition for ameliorating muscle dysfunction associated with lysosomal disease.
39. Use of the conjugate according to 29 or 30 above for the manufacture of a pharmaceutical composition for ameliorating muscle dysfunction associated with Fabry disease.
40. Use of the conjugate according to 31 or 32 above for the manufacture of a pharmaceutical composition for ameliorating muscle dysfunction associated with Pompe disease.
41. Use of the conjugate according to any one of 1 to 32 above as a pharmaceutical composition for improving muscle function.
42. A method for delivering a pharmaceutical agent to muscle, comprising a step of producing the agent as a conjugate in which the agent is linked to an anti-human transferrin receptor antibody and a step of administering the conjugate to an individual.
43. The method according to 42 above, wherein the conjugate is the conjugate according to any one of 1 to 32 above.
44. The method according to 42 or 43 above, further comprising a step of improving muscle function of the individual by administration of the conjugate.
45. The method according to any one of 42 to 44 above, wherein the individual has a muscle dysfunction.
46. The method according to any one of 42 to 44 above, wherein the individual has a muscle dysfunction associated with lysosomal disease.
47. The method of 46 above, wherein the lysosomal disease is Fabry disease or Pompe disease.

### Effects of the Invention

According to the present invention, a pharmaceutical agent exerting its function in a muscle, in particular, a peptide or a protein having a physiological activity can be efficiently delivered to a muscle when the agent is administered in a body by let the agent to be conjugated with an anti-human transferrin receptor. In other words, by conjugating the agent with the human transferrin receptor, it is possible to provide a more effective therapeutic agent for diseases associated with muscle dysfunction.

### Brief Description of Drawings

[Figure 1] A figure showing the quantitative results of glycogen contained in the heart. The quantitative results of the wild-type control group, (1) shows the quantitative results of wild-type control group, KO-control group, hGAA administration group, and hGAA-anti-hTfR antibody administration group are shown in (1), (2), (3), and (4), respectively. Vertical bars indicate SD values. The vertical axis represents glycogen concentration (mg/g wet tissue weight).
[Figure 2] A figure showing the quantitative results of glycogen contained in the diaphragm. The same are represented in (1)∼(4), vertical bars, and the vertical axis as in Fig. 1.
[Figure 3] A figure showing the quantitative results of glycogen contained in the soleus muscle. The same are represented in (1)∼(4), vertical bars, and the vertical axis as in Fig. 1.
[Figure 4] A figure showing the quantitative results of glycogen contained in the tibialis anterior muscle. The same are represented in (1) ∼(4), vertical bars, and the vertical axis as in Fig. 1.
[Figure 5] A figure showing the quantitative results of glycogen contained in the quadriceps femoris muscle. The same are represented in (1)∼(4), vertical bars, and the vertical axis as in Fig. 1.

### Embodiments for Carrying Out the Invention

In the present invention, the term "muscle" means a tissue mainly composed of skeletal muscle, cardiac muscle, or smooth muscle, and in particular, a tissue mainly composed of skeletal muscle or a tissue mainly composed of cardiac muscle. And the term "muscle tissue" is understood to be synonymous with muscle.

In the present invention, a pharmaceutical agent that should exert physiological activity in a muscle is the agent that cannot exert its physiological activity sufficiently in a muscle for the inefficient transport to the muscle when the agent is administered alone into the blood. Such agents may be small molecule compounds, peptides, or proteins. Here, the peptide is that to be potentially capable of exerting physiological activity in muscle (bioactive peptide), but alone not to be efficiently transported to muscle when administered in a body. Such a peptide preferably consists of 2 to 100 amino acid residues, e.g., 2 to 50, 5 to 100 amino acid residues. Further, such a protein is potentially capable of exerting physiological activity in muscle (bioactive protein), but it alone is not efficiently transported to muscle when administered in a body, and includes lysosomal enzymes such as acid α-glucosidase (GAA) and α-galactosidase A (α-GalA), and so on. Such peptides and proteins may have artificial amino acid sequences or may have amino acid sequences encoded by animal genes. Here, the animal species of the gene encoding the peptide or protein is not particularly limited, but is preferably a mammal, more preferably human, monkey, mouse, domestic animal (horse, pig, sheep, etc.), or pet animal (cat, dog, etc.), and more preferably a human.

In addition to GAA and α GalA, lysosomal enzymes that need to exhibit functions as medicaments for improving the malfunction of muscle associated with lysosomal diseases include includeα-L-iduronidase (IDUA), iduronate-2-sulfatase (IDS), glucocerebrosidase (GBA), β-galactosidase, GM2 activator protein, β-hexosaminidase A, β-hexosaminidase B, N-acetylglucosamine-1-phosphotransferase, α-mannosidase (LAMAN), β-mannosidase, galactosylceramidase (GALC), Saposin C, arylsulfatase A (ARSA), α-L-fucosidase (FUCA1), aspartylglucosaminidase, α-N-acetylgalactosaminidase, acid sphingomyelinase (ASM), β-glucuronidase (GUSB), heparan N-sulfatase (SGSH),α-N-acetylglucosaminidase (NAGLU), acetyl-CoA: α-glucosaminide N-acetyltransferase, N-acetylglucosamine-6-sulfate sulfatase, acid ceramidase (AC), amylo-1, 6-glucosidase, sialidase, aspartylglucosaminidase, palmitoyl protein thioesterase-1 (PPT-1), tripeptidyl peptidase-1 (TPP-1), hyaluronidase-1, CLN1, CLN2, and a like. These enzymes can be more efficiently delivered to muscle when administered in a body as a form of a conjugate with an anti-human transferrin receptor antibody. Thus, these conjugates can be used as a medicament to ameliorate muscle dysfunction associated with lysosomal disease.

Iduronate-2-sulfatase (IDS) fused with anti-hTfR antibody can be used as a therapeutic agent for muscle dysfunction in Hunter syndrome, α-galactosidase A fused with anti-hTfR antibody can be used as a therapeutic agent for muscle dysfunction in Fabry disease, α-L-iduronidase (IDUA) fused with anti-hTfR antibody can be used as a therapeutic agent for muscle dysfunction in Hurler syndrome or Hurler-Scheie syndrome, glucocerebrosidase (GBA) fused with anti-hTfR antibody can be used as a therapeutic agent for muscle dysfunction in Gaucher disease, β-galactosidase fused with anti-hTfR antibody can be used as a therapeutic agent for muscle dysfunction in GM1-gangliosidosis types 1-3, GM2 activator protein fused with anti-hTfR antibody can be used as a therapeutic agent for muscle dysfunction in GM2-gangliosidosis AB variant, β-hexosaminidase A fused with anti-hTfR antibody can be used as a therapeutic agent for muscle dysfunction in Sandhoff disease and Tay-Sachs disease, β-hexosaminidase B fused with anti-hTfR antibody can be used as a therapeutic agent for muscle dysfunction in Sandhoff disease, N-acetylglucosamine-1-phosphotransferase fused with anti-hTfR antibody can be used as a therapeutic agent for muscle dysfunction in I-cell disease, α-mannosidase (LAMAN) fused with anti-hTfR antibody can be used as a therapeutic agent for muscle dysfunction in α-mannosidosis, galactosylceramidase (GALC) fused with anti-hTfR antibody can be used as a therapeutic agent for muscle dysfunction in Krabbe disease, saposin C fused with anti-hTfR antibody can be used as a therapeutic agent for muscle dysfunction in Gaucher-like disease, and arylsulfatase A (ARSA) fused with anti-hTfR antibody can be used as a therapeutic agent for muscle dysfunction in metachromatic leukodystrophy, α-L-fucosidase (FUCA1) fused with anti-hTfR antibody can be used as a therapeutic agent for muscle dysfunction in fucosidosis, aspartylglucosaminidase fused with anti-hTfR antibody can be used as a therapeutic agent for muscle dysfunction in aspartylglucosamiuria, α-N-acetylgalactosaminidase fused with anti-hTfR antibody can be used as a therapeutic agent for muscle dysfunction in Sindler disease and Kanzaki disease, acid sphingomyelinase (ASM) fused with anti-hTfR antibody can be used as a therapeutic agent for muscle dysfunction in Niemann-Pick disease, β-glucuronidase (GUSB) fused with anti-hTfR antibody can be used as a therapeutic agent for muscle dysfunction in Sly syndrome, heparan N-sulfatase (SGSH), α-N-acetylglucosaminidase (NAGLU), acetyl-CoA: α-glucosaminide N-acetyltransferase, and N-acetylglucosamine-6-sulfate sulfatase fused with anti-hTfR antibody can be used as therapeutic agents for muscle dysfunction in Sanfilippo syndrome, acid ceramidase (AC) fused with anti-hTfR antibody can be used as a therapeutic agent for muscle dysfunction in Farber disease, amylo-1,6-glucosidase fused with anti-hTfR antibody can be used as a therapeutic agent for muscle dysfunction in Cori disease (Forbes/Cori disease), sialidase fused with anti-hTfR antibody can be used as a therapeutic agent for muscle dysfunction in sialidase deficiency, palmitoyl protein thioesterase-1 (PPT-1) fused with anti-hTfR antibody can be used as a therapeutic agent for muscle dysfunction in neuroceroid lipofuscinosis or Santavuori-Haltia disease, tripeptidyl peptidase-1 (TPP-1) fused with anti-hTfR antibody can be used as a therapeutic agent for muscle dysfunction in neuroceroid lipofuscinosis or Jansky-Bielschowsky disease, hyaluronidase-1 fused with anti-hTfR antibody can be used as a therapeutic agent for muscle dysfunction in hyaluronidase deficiency, acid α-glucosidase (GAA) fused with anti-hTfR antibody can be used as a therapeutic agent for muscle dysfunction in Pompe disease, and CLN1 and CLN2 fused with anti-hTfR antibody can be used as therapeutic agents for muscle dysfunction in Batten disease. In particular, the anti-hTfR antibody of the present invention is expected to reach the cerebral parenchyma, hippocampal nerve-like cells, cerebellar Purkinje cells, etc. after crossing the blood-brain barrier, and also to reach nerve-like cells of the cerebral striatum and nigra of the midbrain, so that the pharmaceutical efficacy of the protein can be enhanced by fusing it with a protein to exert its function in these tissues or cells. However, the pharmaceutical use is not limited to use for these diseases.

In the present invention, the term "lysosomal enzyme" or the term indicating an individual lysosomal enzyme means, in particular, that having a natural amino acid sequence, but is not limited thereto, and as long as it has a lysosomal enzyme, lysosomal enzymes in which mutations such as substitutions, deletions, additions, and a like are introduced to the amino acid sequence of the natural lysosomal enzyme are also included in the lysosomal enzyme. When the amino acids of the amino acid sequence of the lysosomal enzyme are substituted with other amino acids, the number of amino acids to be substituted is preferably from 1 to 10, more preferably from 1 to 5, even more preferably from 1 to 3, and still more preferably from 1 to 2. When the amino acids of the amino acid sequence of the lysosomal enzyme are deleted, the number of amino acids to be deleted is preferably from 1 to 10, more preferably from 1 to 5, even more preferably from 1 to 3, and still more preferably from 1 to 2. A mutation combining such substitution and deletion of amino acids can also be introduced to the lysosomal enzyme. When the amino acids are added to the amino acid, preferably 1 to 10, more preferably 1 to 5, even more preferably 1 to 3, and still more preferably 1 to 2 amino acids are added to the amino acid sequence of the lysosomal enzyme or to N-terminal or C-terminal side of the lysosomal enzyme. A mutation combining such addition, substitution and deletion of amino acids can also be introduced to the lysosomal enzyme. The amino acid sequence of the mutated lysosomal enzyme preferably has an identity not lower than 80%, more preferably 90%, and even more preferably 95% to the amino acid sequence of the original lysosomal enzyme. The same applies to proteins and peptides other than lysosomal enzymes.

Here, when it is referred to as "lysosomal enzyme has lysosomal enzyme activity", it means that the lysosomal enzyme has an activity not less than 3% relative to the activity inherent in the wild type lysosomal enzyme when conjugated with anti-hTfR antibody. However, the activity is preferably not less than 10%, more preferably not less than 20%, even more preferably not less than 50%, and still more preferably not less than 80% relative to the activity inherent in the wild type lysosomal enzyme. The same is applied when lysosomal enzymes conjugated to anti-hTfR antibody are mutated. The same applies to proteins and peptides other than lysosomal enzymes.

Substitutions of amino acids in the amino acid sequence of peptides or proteins (including lysosomal enzymes and antibodies) with other amino acids include, for example, substitutions between amino acids in the same group, such as aromatic amino acids (Phe, Trp, Tyr), aliphatic amino acids (Gly, Ala, Leu, Ile, Val), amide-type amino acids (Gln, Asn), basic amino acids (Lys, Arg, His), acidic amino acids (Glu, Asp), amino acids with hydroxyl groups (Ser, Thr), branched amino acids (Val, Leu, Ile), amino acids with small side chains (Gly, Ala, Ser, Thr, Met), and amino acids with nonpolar side chains (Ala, Val, Leu, Ile, Pro, Phe, Met). Substitutions with such similar amino acids are predicted not to result in a change in the phenotype of the protein (i.e., conservative amino acid substitutions). Examples of conservative amino acid substitutions are well known in the art and described in various references (see, e.g., Bowie et al., Science, 247:1306-1310 (1990)).

In the present invention, "homology" or "identity" means the ratio (%) of homologous amino acid residues to total amino acid residues in the optimal alignment when two amino acid sequences are compared using a homology calculation algorithm. It has been well known in the art of the invention to compare the two amino acid sequences by a homology (identity) shown by such a ratio and may be readily understood by those skilled in the art. In the present invention, as a homology calculation algorithm for calculating homology between the amino acid sequence of the original protein (including an antibody) and the amino acid sequence of the mutated protein, BLAST (Altschul SF. J Mol. Biol. 215. 403-10 (1990)), Pearson and Lipman's similar searching method (Proc. Natl. Acad. Sci. USA. 85. 2444 (1988)), Smith and Waterman's local homology algorithm (Adv. Appl. Math. 2. 482-9 (1981)), and so on have been well known. Further, blastp, one of the BLAST programs provided on the Internet by the National Institutes of Health, has been well known as a means to calculate the homology of two amino acid sequences.

The lysosomal enzymes are described in detail by taking acid α-glucosidase and α-galactosidase A as an example in detail below.

Acid α-glucosidase (GAA) is a lysosomal enzyme that has the activity of degrading glycogen. Pompe disease (glycogen storage disease type II) is a disease in which glycogen accumulates in large amounts in the lysosome of cells by genetically lacking most or all of the acid α-glucosidase activity, and the dysfunction of cardiac muscle and skeletal muscle is a main symptom. Pompe disease is classified into infant form, childhood form and adult form. The infant form develops mainly in the early infancy and shows cardiovascular symptoms due to concentric cardiac hypertrophy, muscle weakness due to skeletal muscle invasion, and hypotonia due to glycogen accumulation in the myocardium, and is characterized by congenital myopathic symptoms. In the childhood form, skeletal muscle symptoms are predominant, and they show motor developmental delay and progressive muscle weakness from the late infancy. Muscle weakness is the main symptom in the adult form.

α-Galactosidase A (a-GalA) is a lysosomal enzyme that has the activity of hydrolyzing terminal α-galactosyl bonds of glycolipids and glycoproteins. Trihexosylceramide is one of the substrates for α-galactosidase A and undergoes hydrolysis by the enzyme at its terminal hexose moiety. Fabry disease is a disease caused by a genetic loss of most or all of the α-GalA activity. The clinical features of Fabry disease are renal failure, angiokeratoma, and cardiovascular abnormalities, such as ventricular hypertrophy and mitral valve regurgitation. An atypical type of Fabry disease, in which the heart is primarily impaired, could be distinguished, particularly as cardiac Fabry disease.

As a therapy for the lysosomal disease patient lacking the lysosomal enzyme, the enzyme replacement therapy in which the lysosomal enzyme produced using the gene recombination technology is administered to the patient as a drug is carried out. For example, enzyme replacement therapy using Myozyme (registered trademark), a recombinant human acid α-glucosidase produced by gene recombination technology, is carried out on Pompe disease patients. In addition, enzyme replacement therapy using Fabrazyme or Ripregal (registered trademarks), a recombinant human α-galactosidase A produced by the gene recombination technology, is carried out for the Fabry disease patient. One of tissues in which lysosomal enzymes used as medicaments in these enzyme replacement therapies should exert their effect is muscle. Thus, by increasing the supply of lysosomal enzymes to the muscle, it may be possible to further ameliorate the muscle dysfunction associated with lysosomal disease.

In the present invention, the term "human acid α-glucosidase" or "hGAA" particularly means a hGAA having an amino acid sequence set forth as SEQ ID NO: 1 which is the same as that of a native hGAA, but the present invention is not limited thereto, and as long as it has hGAA activity, hGAA includes a hGAA to which mutations such as substitutions, deletions, additions, and the like are introduced to the amino acid sequence of a native hGAA. When the amino acids of the amino acid sequence of hGAA are substituted with other amino acids, the number of amino acids to be substituted is preferably from 1 to 10, more preferably from 1 to 5, even more preferably from 1 to 3, and still more preferably from 1 to 2. When the amino acids of the amino acid sequence of a hGAA, the number of amino acids to be deleted is preferably from 1 to 10, more preferably from 1 to 5, even more preferably from 1 to 3, and still more preferably from 1 to 2. A mutation combining such substitution and deletion of amino acids can also be introduced to hGAA. When amino acids are added to a hGAA, preferably 1 to 10, more preferably 1 to 5, even more preferably 1 to 3, and still more preferably 1 to 2 amino acids are added in the amino acid sequence of hGAA or on the N-terminal or C-terminal side. A mutation combining such addition, substitution and deletion of amino acids can also be introduced to hGAA. The amino acid sequence of the mutated hGAA preferably has 80% or more identity, more preferably 90% or more identity, and even more preferably 95% or more identity to the amino acid sequence of the original hGAA.

Here, the term "hGAA has hGAA activity" means that it has not less than 3% activity relative to the activity inherent in the natural type hGAA when conjugated to anti-hTfR antibody. However, the activity is preferably not less than 10%, more preferably 20% or more, even more preferably not less than 50%, and still more preferably not less than 80% relative to the activity inherent in the natural type hGAA. The same is true when hGAA conjugated with the anti-hTfR antibody is mutated.

In the present invention, the term "human α-galactosidase A" or "hα-GalA" means, in particular, hα-GalA having the amino acid sequence set forth as SEQ ID NO: 2 which is the same as that of a native hα-GalA, but is not limited thereto, and as long as the hα-GalA has the hα-GalA activity, the hα-GalA also includes mutations such as substitutions, deletions, additions, etc. in the amino acid sequence of the native hα-GalA. When amino acids of the amino acid sequence of the hα-GalA are substituted with other amino acids, the number of amino acids to be substituted is preferably from 1 to 10, more preferably from 1 to 5, even more preferably from 1 to 3, and still more preferably from 1 to 2. When amino acids in the amino acid sequence of the hα-GalA are deleted, the number of amino acids to be deleted is preferably from 1 to 10, more preferably from 1 to 5, even more preferably from 1 to 3, and still more preferably from 1 to 2. A mutation combining such substitution and deletion of amino acids can also be introduced to the hα-GalA. When amino acids are added to the hα-GalA, preferably 1 to 10, more preferably 1 to 5, even more preferably 1 to 3, still more preferably 1 to 2 amino acids are added in the amino acid sequence of the hα-GalA or on the N-terminal or C-terminal side. A mutation combining such addition, substitution and deletion of amino acids may also be introduced to the hα-GalA. The amino acid sequence of the mutated hα-GalA preferably has not less than 80% identity, more preferably not less than 90% identity, and even more preferably not less than 95% identity to the amino acid sequence of the original hα-GalA.

Here, when hα-GalA has hα-GalA activity, it means that it has an not less than 3% activity relative to the activity inherent in the native hα-GalA when conjugated with anti-hTfR antibody. However, the activity is preferably not less than 10%, more preferably not less than 20%, even more preferably not less than 50%, and still more preferably not less than 80% relative to the activity inherent in the native hα-GalA. The same is true when the hα-GalA conjugated with anti-hTfR antibody is mutated.

In the present invention, the term "antibody" refers primarily to human antibody, mouse antibody, humanized antibody, chimeric antibody of human antibody and other mammalian antibody, and chimeric antibody of mouse antibody and other mammalian antibody, but as long as they have the property of specifically binding to a particular antigen, they are not limited thereto, and there are not any particular limitations to the animal species of the antibody.

In the present invention, the term "human antibody" refers to an antibody whose entire protein is encoded by a gene originating from human. The term "human antibody", however, also includes an antibody encoded by a gene obtained by introducing a mutation into an original human gene for a purpose of enhancing expression efficacy of the gene, for example, without modifying the original amino acid sequence. The term "human antibody" also includes an antibody in which certain part of the human antibody is replaced with part of another human antibody by combining two or more genes encoding human antibodies. A human antibody includes three complementarity determining regions (CDRs: abbreviation of complementary determining region) in the light chain of the immunoglobulin and three complementarity determining regions (CDRs) in the heavy chain of the immunoglobulin. The three CDRs in the light chain of the immunoglobulin are called, from the N-terminal side, CDR1, CDR2 and CDR3, respectively. The three CDRs in the heavy chain of the immunoglobulin are also called, from the N-terminal side, CDR1, CDR2 and CDR3, respectively. The term "human antibody" also includes a human antibody produced by replacing a CDR of a human antibody with a CDR of another human antibody to modify such properties as the antigen specificity and the affinity of the original human antibodies, etc.

In the present invention, the term "human antibody" also includes an antibody which is produced through modification of the gene of the original human antibody by introducing a mutation, such as substitution, deletion, addition, to the amino acid sequence of the original antibody. When replacing one or more amino acids of the amino acid sequence of the original antibody with other amino acids, the number of amino acid replaced may preferably be 1-20, more preferably 1-5, and still more preferably 1-3. When deleting one or more amino acids of the amino acid sequence of the original antibody, the number of amino acids deleted may preferably be 1-20, more preferably 1-5, and still more preferably 1-3. An antibody produced by a combined mutation of these substitution and deletion of amino acids is also a "human antibody". In some cases, one or more amino acids, preferably 1-20, more preferably 1-5, and still more preferably 1-3 amino acids may be added inside the amino acid sequence of the original antibody or on its N- or C-terminal side. An antibody produced by a combined mutation of addition, substitution, and deletion of amino acids is also a "human antibody". The amino acid sequence of such a mutated antibody has a homology of preferably not lower than 80%, more preferably not lower than 90%, still more preferably not lower than 95%, and even more preferably not lower than 98%, to the amino acid sequence of the original antibody. Thus, in the present invention, the term "gene originating from human" includes not only the unmutated gene originating from human but also a gene produced by modifying this.

The term "mouse antibody" refers to an antibody whose entire protein consists of an amino acid sequence which is the same as an antibody encoded by a gene originating from a mouse. Therefore, the term "mouse antibody" also includes an antibody that is encoded by a gene produced by introducing a mutation into the original mouse gene without causing a change in its amino acid sequence but in order, for example, to improve the expression efficiency of the gene. Further, the term "mouse antibody" also includes an antibody produced through combining two or more genes encoding mouse antibodies by replacing part of a mouse antibody with part of another mouse antibody. A mouse antibody has three complementarity determining regions (CDRs) in the light chain of the immunoglobulin and three complementarity determining regions (CDRs) in the heavy chain of the immunoglobulin. The three CDRs in the light chain of the immunoglobulin are called, from the N-terminal side, CDR1, CDR2 and CDR3, respectively. The three CDRs in the heavy chain of the immunoglobulin are also called, from the N-terminal side, CDR1, CDR2 and CDR3, respectively. For example, the term "mouse antibody" also includes an antibody produced by replacing a CDR of a mouse antibody with a CDR of another mouse antibody to modify the specificity and affinity of the original mouse antibodies.

In the present invention, the term "mouse antibody" also includes an antibody which is produced through modification of the gene of the original mouse antibody by introducing a mutation, such as substitution, deletion, addition, to the amino acid sequence of the original antibody. When replacing one or more amino acids of the amino acid sequence of the original antibody with other amino acids, the number of amino acid replaced may preferably be 1-20, more preferably 1-5, and still more preferably 1-3. When deleting one or more amino acids of the amino acid sequence of the original antibody, the number of amino acids deleted may preferably be 1-20, more preferably 1-5, and still more preferably 1-3. An antibody produced by a combined mutation of these substitution and deletion of amino acids is also included in a "mouse antibody". When adding one or more amino acids, they may be added inside the amino acid sequence of the original antibody or on its N- or C-terminal side, preferably 1-20, more preferably 1-5, and still more preferably 1-3, in number. An antibody produced by a combined mutation of addition, substitution, and deletion of amino acids is also included in a "mouse antibody". The amino acid sequence of such a mutated antibody has a homology of preferably not lower than 80%, more preferably not lower than 90%, still more preferably not lower than 95%, and even more preferably not lower than 98%, to the amino acid sequence of the original antibody. Thus, in the present invention, the term "gene originating from mouse" includes not only the unmutated gene originating from mouse but also a gene produced by modifying this.

In the present invention, the term "humanized antibody" refers to an antibody in which part of the amino acid sequence of its variable region (e.g., especially the whole or part of its CDRs) originates from a non-human mammal while the rest originates from human. An example of humanized antibody is an antibody produced by replacing the three complementarity determining regions (CDRs) of the light chain of the immunoglobulin and the three complementarity determining regions (CDRs) of the heavy chain of the immunoglobulin constituting a human antibody, with CDRs from a non-human mammal. There is no particular limitation as to the biological species from which those CDRs grafted into a proper position of the human antibody originate, insofar as it originates from a non-human mammal. Though the species are preferably mouse, rat, rabbit, horse or non-human primate, and more preferably mouse or rat, for example mouse.

In the present invention, the term "chimeric antibody" refers to an antibody produced by connecting fragments of two or more different antibodies originating from two or more different species.

A chimeric antibody comprising a human antibody and a non-human mammalian antibody is an antibody provided by replacing part of a human antibody with part of a non-human mammalian antibody. An antibody is made of an Fc region, a Fab region and a hinge region. A specific example of such chimeric antibodies is a chimeric antibody whose Fc region originates from a human antibody while its Fab region originates from a non-human mammalian antibody. The hinge region either originates from a human antibody or from a non-human mammalian antibody. On the contrary, the term chimeric antibody also includes one whose Fc region originates from a non-human mammalian antibody while its Fab region originates from a human antibody. In such a case also, the hinge region either originates from a human antibody or from a non-human mammalian antibody.

An antibody can be viewed as composed of a variable region and a constant region. Additional examples of chimeric antibodies include an antibody in which the heavy chain constant region (C_{H}) and the light chain constant region (C_{L}) both originate from a human antibody while the heavy chain variable region (V_{H}) and the light chain variable region (V_{L}) both originate from an antibody of a non-human mammal, and conversely, an antibody in which the heavy chain constant region (C_{H}) and the light chain constant region (C_{L}) both originate from an antibody of a non-human mammal, while the heavy chain variable region (V_{H}) and the light chain variable region (V_{L}) both originate from a human antibody. In these, there is no particular limitation as to the biological species of the non-human mammal, insofar as it is a non-human mammal, though the species are preferably mouse, rat, rabbit, horse or non-human primate, more preferably mouse.

A chimeric antibody of a mouse antibody and another mammalian antibody is an antibody in which a part of the mouse antibody is replaced by a part of an antibody of a mammal other than a mouse. An example of such a chimeric antibody includes a chimeric antibody in which the Fc region is derived from a mouse antibody while the Fab region is derived from an antibody of another mammal, and conversely, a chimeric antibody in which the Fc region is derived from another mammal while the Fab region is derived from a mouse antibody. Here, the species of another mammal is not particularly limited as long as it is a mammal other than a mouse, but preferably is a rat, a rabbit, a horse, and a non-human primate.

A chimeric antibody comprising a human antibody and a mouse antibody is designated in particular "human/mouse chimeric antibody". Examples of human/mouse chimeric antibodies include a chimeric antibody in which the Fc region originates from a human antibody while the Fab region originates from a mouse antibody, and conversely, a chimeric antibody whose Fc region originates from mouse antibody, while its Fab region originates from a human antibody. A hinge region either originates from a human antibody or a mouse antibody. Additional specific examples of human/mouse chimeric antibodies include those whose heavy chain constant region (C_{H}) and light chain constant region (C_{L}) originate from a human antibody while its heavy chain variable region (V_{H}) and light chain variable region (V_{L}) originate from a mouse antibody, and conversely, those whose heavy chain constant region (C_{H}) and light chain constant region (C_{L}) originate from a mouse antibody while its heavy chain variable region (V_{H}) and light chain variable region (V_{L}) originate from a human antibody.

Originally, an antibody is of the basic structure having four polypeptide chains in total, consisting of two immunoglobulin light chains and two immunoglobulin heavy chains. However, in the present invention the term "antibody" refers, besides to an antibody having this basic structure, also to;
(1) an antibody consisting of two polypeptide chains comprising a single immunoglobulin light chain and a single immunoglobulin heavy chain, and, as explained later,
(2) a single-chain antibody consisting of an immunoglobulin light chain which is linked, on the C-terminal side thereof, to a linker sequence which in turn is linked, on the C-terminal side thereof, to an immunoglobulin heavy chain,
(3) single-chain antibodies consisting of an immunoglobulin heavy chain which is linked, on the C-terminal side thereof, to a linker sequence which in turn is linked, on the C-terminal side thereof, to an immunoglobulin light chain, and
(4) one consisting of a Fab region, i.e., a structure left behind by removal of the Fc region from an antibody having the basic structure as the original meaning, and one consisting of the Fab region and the whole or part of the hinge region (including Fab, F(ab'), and F(ab')₂).

Here, the term "Fab" refers to a molecule consisting of a single light chain comprising the variable region and the C_{L} region (light chain constant region) and a single heavy chain comprising the variable region and the C_{H}1 region (portion 1 of heavy chain constant region) which are combined by a disulfide bond between their respective cysteine residues. While the heavy chain in a Fab can include part of the hinge region in addition to the variable region and the C_{H}1 region (portion 1 of heavy chain constant region), the hinge region in such a case lacks the cysteine residue that otherwise is present in the hinge region and would serve to link two heavy chains of an antibody together. In Fab, the light chain and the heavy chain are connected by a disulfide bond formed between the cysteine residue present in the light chain constant region (C_{L} region) and the cysteine residue located in the heavy chain constant region (C_{H}1 region) or the hinge region. As it lacks the cysteine residue in the hinge region which serves to bind two heavy chains of an antibody, Fab consists of a single light chain and a single heavy chain. The light chain of Fab includes a variable region and a C_{L} region. The heavy chain as a component of Fab may either consist of a variable region and a C_{H}1 region or also of part of the hinge region in addition to the variable region and the C_{H}1 region. However, in this case, the hinge region is so selected as not to include the cysteine residue that could bind two heavy chains, in order to avoid the formation of a disulfide bond between two heavy chains at their hinge regions. In F(ab'), the heavy chain includes, in addition to a variable region and a C_{H}1 region, the whole or part of a hinge region containing a cysteine residue that could bind two heavy chains. F(ab')₂ is a molecule consisting of two F(ab')s bound together through a disulfide bond formed between the cysteine residues present in their respective hinge regions. Further, a polymer such as a dimer and a trimer, which consists of two or more antibodies connected with each other, directly or via a linker, is also included in the term "antibody". Moreover, in addition to the aforementioned, any molecule that includes part of an immunoglobulin molecule and has a property to specifically bind to the antigen is also included in the term "antibody" in the present invention. Thus, in the present invention, the term "immunoglobulin light chain" includes a molecule that is derived from an original immunoglobulin light chain and having the amino acid sequence of the whole or part of its variable region. Likewise, the term "immunoglobulin heavy chain" includes a molecule that is derived from an original immunoglobulin heavy chain and having the amino acid sequence of the whole or part of its variable region. Therefore, insofar as having the whole or part of the amino acid sequence of the variable region, a molecule is included in the term "immunoglobulin heavy chain", even if it lacks its Fc region, for example.

Also, in the above, the Fc or Fc region refers to a region including fragments consisting of a C_{H}2 region (portion 2 of the constant region of the heavy chain) and a C_{H}3 region (portion 3 of the constant region of the heavy chain) in the antibody molecule.

Furthermore, in the present invention, the term "antibody" also includes:

(5) scFab, scF(ab'), and scF(ab')₂, which are single-chain antibodies produced by binding the light chain to the heavy chain that form, respectively, the Fab, F(ab') and F(ab')₂ mentioned in (4) above, via a linker sequence. Such scFab, scF(ab') and scF(ab')₂ may be a molecule in which either the light chain is linked, on the C-terminal side thereof, to a linker sequence, which in turn is linked, on the C-terminal side thereof, to the heavy chain, or the heavy chain is linked, on the C-terminal side thereof, to a linker sequence, which in turn is linked, on the C-terminal side thereof, to the light chain. Furthermore, a scFv, which is a single-chain antibody provided by binding the light chain variable region to the heavy chain variable region, via a linker sequence between them, is also included in the term "antibody" in the present invention. Such scFv may be a molecule in which either the light chain variable region is linked, on the C-terminal side thereof, to a linker sequence, which in turn is linked, on the C-terminal side thereof, to the heavy chain variable region, or the heavy chain variable region is linked, on the C-terminal side thereof, to a linker sequence, which in turn is linked, on the C-terminal side thereof, to the light chain variable region.

In the present invention, the term "single-chain antibody" refers to a protein in which an amino acid sequence comprising the whole or part of an immunoglobulin light chain variable region linked, on the C-terminal side thereof, to a linker sequence, which in turn is linked, on the C-terminal side thereof, to the amino acid sequence of the whole or part of an immunoglobulin heavy chain variable region, and having an ability to specifically bind a certain antigen. For example, those shown in (2), (3), and (5) above are included in the single-chain antibody. Further, a protein in which an amino acid sequence comprising the whole or part of an immunoglobulin heavy chain variable region is linked, on the C-terminal side thereof, to a linker sequence, which in turn is further linked, on the C-terminal side thereof, to the amino acid sequence of the whole or part of an immunoglobulin light chain variable region, and which has an ability to specifically bind to a certain antigen, is also included in the term "single-chain antibody" in the present invention. In a single-chain antibody in which an immunoglobulin heavy chain is linked, on the C-terminal side thereof and via a linker sequence, to an immunoglobulin light chain, the immunoglobulin heavy chain generally lacks the Fc region. An immunoglobulin light chain variable region has three complementarity determining regions (CDRs) which participate in determining the antigen specificity of an antibody. Likewise, an immunoglobulin heavy chain variable region also has three CDRs. Those CDRs are the primary regions that determine the antigen specificity of an antibody. Therefore, a single-chain antibody preferably contains all the three CDRs of the immunoglobulin heavy chain and all the three CDRs of the immunoglobulin light chain. However, it is also possible to provide a single-chain antibody in which one or more of those CDRs are deleted, insofar as the antigen-specific affinity of the antibody is retained.

In a single-chain antibody, the linker sequence placed between the light chain and the heavy chain of the immunoglobulin is a peptide chain consisting of preferably 2 to 50, more preferably 8 to 50, still more preferably 10 to 30, even more preferably 12 to 18, or 15 to 25, for example 15 or 25 amino acid residues. While there is no particular limitation as to the specific amino acid sequence of such a linker sequence insofar as the anti-hTfR antibody comprising the both chains linked thereby retains the affinity to hTfR, it is preferably made of glycine only, or of glycine and serine. For example, there are the amino acid sequence Gly-Ser, the amino acid sequence Gly-Gly-Ser, the amino acid sequence Gly-Gly-Gly, the amino acid sequence Gly-Gly-Gly-Gly-Ser (SEQ ID NO:3), the amino acid sequence Gly-Gly-Gly-Gly-Gly-Ser (SEQ ID NO:4), the amino acid sequence Ser-Gly-Gly-Gly-Gly (SEQ ID NO:21), or a sequence which includes a sequence corresponding to 2 to 10 or 2 to 5 of any of those amino acid sequences consecutively linked. For example, in linking the amino acid sequence of the entire immunoglobulin heavy chain variable region on the C-terminal side thereof and via a linker sequence, to immunoglobulin light chain variable region, a preferable linker sequence is a linker sequence consisting of a total of 15 amino acids corresponding to three of the amino acid sequence Gly-Gly-Gly-Gly-Ser (SEQ ID NO:3) consecutively linked.

In the present invention, the term "human transferrin receptor" or "hTfR" refers to a membrane protein having the amino acid sequence set forth as SEQ ID NO:5. The anti-hTfR antibody of the present invention is, in one of its embodiments, that which binds to the region from the cysteine residue at the position 89th from the N-terminus to the phenylalanine at the C-terminus in the amino acid sequence set forth as SEQ ID NO:5 (the extracellular region of hTfR), though it is not limited to this embodiment. Further in the present invention, the term "monkey transferrin receptor" or "monkey TfR" refers to, in particular, a membrane protein having the amino acid sequence set forth as SEQ ID NO: 6 derived from a crab-eating macaque (Macaca fascicularis). The anti-hTfR antibody of one embodiment of the present invention, also binds to the region from the cysteine residue at the position 89th from the N-terminus to the phenylalanine at the C-terminus in the amino acid sequence set forth as SEQ ID NO:6 (the extracellular region of monkey TfR).

As a method for producing antibody against hTfR, it is common practice to generate recombinant human transferrin receptor (rhTfR) using cells transduced with expression vectors incorporating hTfR genes and immunize an animal such as a mouse with this rhTfR. Hybridoma cells capable of producing the antibody can be produced by collecting antibody-producing cells against hTfR from the animal after immunization and fusing the cells with myeloma cells.

Further, cells producing an antibody to hTfR can also be obtained by collecting immunocompetent cells from an animal such as mouse, and immunizing them with rhTfR by in vitro immunization. In conducting immunization by in vitro immunization, there is no particular limitation as to the animal species from which the immunocompetent cells are derived, though preferred are mouse, rat, rabbit, guinea pig, dog, cat, horse, and primates including human, and more preferred are mouse, rat and human, and still more preferably mouse and human. As mouse immunocompetent cells, spleen cells prepared from mouse spleen may be used, for example. As human immunocompetent cells, such cells can be used as prepared from human peripheral blood, bone marrow, spleen, and the like. By immunizing human immunocompetent cells according to in vitro immunization, a human antibody to hTfR can be obtained.

After immunizing the immune system cells by in vitro immunization, the cells are fused with myeloma cells, whereby hybridoma cells capable of producing the antibody can be produced. In addition, it is also possible to extract mRNA from the cells after immunization to synthesize cDNA, amplify DNA fragments containing genes encoding the light and heavy chain of immunoglobulin by PCR-reaction using this cDNA as a template, and artificially reconstruct antibody genes using the amplified DNA fragments.

Hybridoma cells as obtained by the above method also include cells producing antibodies that recognize proteins other than hTfR as antigens. Also, all of the hybridoma cells producing the anti-hTfR antibody do not necessarily produce an anti-hTfR antibody that exhibits the desired affinity for hTfR.

Likewise, artificially reconstructed antibody genes include such genes as encode antibodies recognizing other proteins than hTfR as antigens. Moreover, not all the genes encoding anti-hTfR antibodies necessarily have desired properties such as encoding an anti-hTfR antibody exhibiting high affinity to hTfR.

Therefore, a selection step is necessary to select hybridoma cells producing an antibody having desired properties (such as high affinity to hTfR) from the hybridoma cells freshly obtained above. Further, in the case where antibody genes are artificially reconstructed, a selection step is necessary to select from the antibody genes a gene encoding an antibody having desired properties (such as high affinities to hTfR).

For example, for selecting hybridoma cells which produce high affinity antibodies to anti-hTfR antibody, a method is employed in which recombinant hTfR is added to a plate and held by it, then the culture supernatant of the hybridoma cells is added, and after removing antibody unbound to the recombinant hTfR from the plate, the amount of the antibody held by the plate is measured. According to this method, the higher the affinity to hTfR of the antibody contained in the culture supernatant of the hybridoma cells added to the plate is, the greater the amount of antibody held by the plate becomes. Therefore, by measuring the amount of the antibody held by the plate, it is possible to select those hybridoma cells corresponding to the plates where the antibody is held in the greater amount as cell lines producing an anti-hTfR antibody having the relatively higher affinity to hTfR. It is also possible to isolate the gene encoding the high-affinity antibody by extracting mRNAs from each cell line selected in this manner, synthesizing cDNAs, and amplifying a DNA fragment containing the gene encoding the anti-hTfR antibody by PCR using the cDNA as a template.

In order to select the gene encoding the high-affinity anti-hTfR antibody from the above artificially reconstructed antibody genes, the artificially reconstructed antibody genes are once incorporated into an expression vector, and the expression vector then is introduced into host cells. Although there is no particular limitation as to the cells to be employed as host cells, even whether they are prokaryotic eukaryotic, insofar as they can express the antibody gene after introduction of an expression vector having the incorporated artificially reconstructed antibody gene, preferred are cells originating mammals such as human, mouse, Chinese hamster, and the like, and particularly preferred are CHO cells originating from Chinese hamster ovary cells, or NS/0 cells originating from mouse myeloma. Further, there is no particular limitation as to an expression vector to be employed for incorporation of the antibody encoding gene and expression of it, and any expression vector may be used as far as it can express the gene when incorporated into mammalian cells. The gene incorporated into an expression vector is located downstream of a DNA sequence that can regulate the frequency of transcription of a gene in mammalian cells (gene expression regulatory site). Examples of gene expression regulatory sites that may be employed in the present invention include cytomegalovirus-derived promoter, SV40 early promoter, human elongation factor-1α (EF-1α) promoter, human ubiquitin C promoter.

Mammalian cells having such an introduced expression vector come to express the antibody encoded by artificially reconstructed gene above incorporated in the expression vector. In order to select those cells which produce a high-affinity antibody to anti-hTfR antibody from the above obtained cells expressing the artificially reconstructed antibody, a method is employed in which the recombinant hTfR is added to a plate and held by it, then the recombinant hTfR is contacted by the culture supernatant of the cells, and after the removal of antibody unbound to the recombinant hTfR from the plate, the amount of the antibody held by the plate is measured. According to this method, the higher the affinity to hTfR of the antibody contained in the cells culture supernatant is, the greater the amount of antibody held by the plate becomes. Therefore, by measuring the amount of the antibody held by the plate, one can select those cells corresponding to the plate where the antibody is held in the greater amount, as a cell line producing an anti-hTfR antibody having relatively the high-affinity anti-hTfR antibody, and eventually can select a gene encoding an anti-hTfR antibody having a high-affinity anti-hTfR antibody to hTfR. Using cell line selected in this manner, one can perform PCR to amplify a DNA fragment containing the gene encoding the anti-hTfR antibody to isolate the gene encoding the high-affinity antibody.

Selection of the gene encoding a high affinity anti-hTfR antibody from the above artificially reconstructed antibody genes can also be carried out by incorporating the artificially reconstructed antibody genes into an expression vector, introducing the expression vector into E. coli cells, culturing the E. coli cells, and selecting the E. coli cells having the desired gene, in the same manner as in the above selection of hybridoma cells, using the culture supernatant of the E. coli cells or an antibody-containing solution prepared by lysing the E. coli cells. E. coli cells thus selected express the gene encoding an anti-hTfR antibody having a relatively high affinity to hTfR. From this cell line, the gene encoding the anti-hTfR antibody having a relatively the high-affinity anti-hTfR antibody to hTfR can be isolated. In order to allow the antibody to be secreted into the E. coli culture supernatant, the antibody gene may be incorporated into the expression vector so that a secretion signal sequence is attached on the N-terminal side of the gene.

Another method for selection of the gene encoding a high-affinity anti-hTfR antibody is a method in which the antibody encoded by the above artificially reconstructed antibody gene is expressed and retained on phage particles. For this, the antibody gene is reconstructed as a gene encoding a single-chain antibody. A method for retaining phage particles to retain an antibody on their surface is disclosed in international publications WO 1997/09436 and WO1995/11317, and the like, and thus well known. In order to select phages retaining the high-affinity antibody to anti-hTfR antibody from the phages retaining the antibodies encoded by the artificially reconstructed antibody genes, a method is employed in which a recombinant hTfR from the plate is added to a plate and held by, contacted by the phages, and after removal of the phages unbound to the recombinant hTfR, the amount of the phages held by the plate is measured. According to this method, the higher the affinity to hTfR of the antibody retained on the phage particles is, the greater the amount of the phage held by the plate becomes. Therefore, by measuring the amount of the phage held by the plate, one can select the phage particles corresponding to the plate where the phages' were held in the greater amount, as the phage particles producing anti-hTfR antibody having a relatively the high-affinity anti-hTfR antibody to hTfR, and eventually can select the gene encoding the high-affinity anti-hTfR antibody to hTfR. Using the phage particles thus selected, PCR can be performed to amplify a DNA fragment containing the gene encoding the anti-hTfR antibody and isolate the gene encoding the high-affinity antibody.

It is possible to prepare cDNA or phage DNA from the above cells such as the hybridoma cells producing the high-affinity antibody to anti-hTfR, or from the above phage particles retaining high-affinity antibody to anti-hTfR, and perform PCR or the like using it as a template to amplify and isolate a DNA fragment containing the gene encoding the whole or part of the anti-hTfR antibody light chain, the anti-hTfR antibody heavy chain, or a single-chain antibody, as an anti-hTfR antibody. In the same manner, it is also possible to perform PCR or the like to amplify and isolate a DNA fragment containing the gene encoding the whole or part of the light chain variable region of the anti-hTfR antibody, or a DNA fragment containing the gene encoding the whole or part of the heavy chain variable region of the anti-hTfR antibody.

A high-affinity anti-hTfR antibody can be obtained by incorporating the whole or part of the gene encoding the light chain and the heavy chain of this high-affinity anti-hTfR antibody into an expression vector, transforming host cells such as mammalian cells with this expression vector, and culturing the obtained transformant cells. Using the nucleotide sequence of the isolated gene encoding the anti-hTfR antibody, it is also possible to translate the amino acid sequence of the anti-hTfR antibody, and artificially synthesize a DNA fragment encoding the same amino acid sequence. In artificially synthesizing a DNA fragment, the expression level of the anti-hTfR antibody in the host cells can be enhanced by proper selection of the codons.

In order to introduce a mutation such as substitution, deletion, addition and the like into the amino acid sequence of the original anti-hTfR antibody, a mutation may be introduced as desired into the gene encoding the anti-hTfR antibody contained in the isolated DNA fragment. Though the gene encoding the mutated anti-hTfR antibody has a homology preferably not lower than 80%, more preferably not lower than 90%, to the original gene, there is no particular limitation as to the level of homology as far as the affinity for hTfR is not lost. By introducing a mutation into the amino acid sequence so as to modify the number or the type of sugar chains bound to the anti-hTfR antibody, it is also possible to enhance the stability of the anti-hTfR antibody in the body.

When introducing a mutation into the gene encoding the whole or part of the light chain variable region of the anti-hTfR antibody, the gene thus mutated has a homology that is preferably not lower than 80%, more preferably not lower than 90%, to the original gene, though there is no particular limitation as to the level of homology as far as the affinity for hTfR is not lost. When replacing one or more amino acids of the amino acid sequence of the light chain variable region with other amino acids, the number of amino acids to be replaced is preferably 1 to 10, more preferably 1 to 5, still more preferably 1 to 3, and even more preferably 1 or 2. When deleting one or more amino acids of the amino acid sequence of the light chain variable region, the number of amino acid to be deleted is preferably 1 to 10, more preferably 1 to 5, still more preferably 1 to 3, even more preferably 1 or 2. A combined mutation of these substitution and deletion of amino acids can also be carried out. When adding one or more amino acids to the light chain variable region, they may be added inside, or on the N-terminal side or C-terminal side of, the amino acid sequence of the light chain variable region, and the number of amino acids added is preferably 1 to 10, more preferably 1 to 5, still more preferably 1 to 3, and even more preferably 1 or 2. A combined mutation of these addition, substitution, and deletion of amino acids can also be carried out. The amino acid sequence of the light chain variable region thus mutated has a homology that is preferably not lower than 80%, more preferably not lower than 90%, still more preferably not lower than 95% to the amino acid sequence of the original light chain variable region. In particular, when replacing one or more amino acids of the amino acid sequence of CDR with other amino acids, the number of amino acid replaced is preferably 1 to 5, more preferable 1 to 3, still more preferably 1 or 2. When deleting one or more amino acid of the amino acid sequence of CDR, the number of amino acids to be deleted is preferably 1 to 5, more preferably 1 to 3, still more preferably 1 or 2. A combined mutation of these substitution and deletion of the amino acid can also be carried out. When adding one or more amino acids, they may be added inside, or on the N-terminal side or C-terminal side of, the amino acid sequence, and the number of amino acids added is preferably 1 to 5, more preferably 1 to 3, still more preferably 1 or 2. A combined mutation of these addition, substitution, and deletion of amino acids can also be carried out. The amino acid sequence of respective mutated CDR has a homology that is preferably not lower than 80%, more preferably not lower than 90%, and still more preferably not lower than 95% to the amino acid sequence of the original CDR.

When introducing mutation into the gene encoding the whole or part of the heavy chain variable region of the anti-hTfR antibody, the gene thus mutated has a homology that is preferably not lower than 80%, more preferably not lower than 90%, to the original gene, though there is no particular limitation as to the level of homology as far as the affinity for hTfR is not lost. When replacing one or more amino acids of the amino acid sequence of the heavy chain variable region with other amino acids, the number of amino acids to be replaced is preferably 1 to 10, more preferably 1 to 5, still more preferably 1 to 3, and even more preferably 1 or 2. When deleting one or more amino acids of the amino acid sequence of the heavy chain variable region, the number of amino acids to be deleted is preferably 1 to 10, more preferably 1 to 5, still more preferably 1 to 3, even more preferably 1 or 2. A combined mutation of these substitution and deletion of amino acids can also be carried out. When adding one or more amino acid to the heavy chain variable region, they may be added inside, or on the N-terminal side or C-terminal side of, the amino acid sequence of the heavy chain variable region, and the number of amino acids added is preferably 1 to 10, more preferably 1 to 5, still more preferably 1 to 3, and even more preferably 1 or 2. A combined mutation of these addition, substitution, and deletion of amino acids can also be carried out. The amino acid sequence of the heavy chain variable region thus mutated has a homology that is preferably not lower than 80%, more preferably not lower than 90%, still more preferably not lower than 95% to the amino acid sequence of the original heavy chain variable region. In particular, when replacing one or more amino acids of the amino acid sequence of CDR with other amino acids, the number of amino acid replaced is preferably 1 to 5, more preferable 1 to 3, still more preferably 1 or 2. When deleting one or more amino acid of the amino acid sequence of CDR, the number of amino acids to be deleted is preferably 1 to 5, more preferably 1 to 3, still more preferably 1 or 2. A combined mutation of these substitution and deletion of the amino acid can also be carried out. When adding one or more amino acids, they may be added inside, or on the N-terminal side or C-terminal side of, the amino acid sequence, and the number of amino acids added is preferably 1 to 5, more preferably 1 to 3, still more preferably 1 or 2. A combined mutation of these addition, substitution, and deletion of amino acids can also be carried out. The amino acid sequence of respective mutated CDR has a homology that is preferably not lower than 80%, more preferably not lower than 90%, and still more preferably not lower than 95% to the amino acid sequence of the original CDR.

A mutation may be introduced into both the variable regions of the light chain and the heavy chain of the anti-hTfR antibody, by combining the above mutation into the light chain variable region of the anti-hTfR antibody and the above mutation into the heavy chain variable region of the anti-hTfR antibody.

Substitutions of amino acids in the amino acid sequences of the light and heavy chain of the above anti-hTfR antibody to other amino acids include a substitution between amino acids classified into the same group, such as aromatic amino acids (Phe, Trp, Tyr), aliphatic amino acids (Gly, Ala, Leu, Ile, Val), amide-type amino acids (Gln, Asn), basic amino acids (Lys, Arg, His), acidic amino acids (Glu, Asp), amino acids with hydroxyl groups (Ser, Thr), branched amino acids (Val, Leu, Ile), amino acids with smaller side chains (Gly, Ala, Ser, Thr, Met), amino acids with nonpolar side chains (Ala, Val, Leu, Leu, Ile, Pro, Phe, Met). It is expected that the substitution between such analogous amino acids does not bring about any change to the phenotype of the protein (i.e., it is conservative amino acid substitution). Examples of conservative amino acid substitutions are well known in the art and described in various references (see, e.g., Bowie et al., Science, 247:1306-1310 (1990)).

When amino acids are added to the C-terminal or N-terminal end of an anti-hTfR antibody by mutation and the anti-hTfR antibody and a drug (including a physiologically active protein) to exert its function in muscle are linked via the amino acids, the amino acids shall constitute a part of the anti-hTfR antibody in the conjugate.

The anti-hTfR antibody obtained by culturing the cells selected by the above methods and the like, as producing an anti-hTfR antibody that has a relatively the high-affinity anti-hTfR antibody to hTfR, and the anti-hTfR antibody obtained by expression of the gene encoding a high-affinity anti-hTfR antibody, may be modified by introducing a mutation into their amino acid sequences, such as substitution, deletion, addition to give them desired properties. Introduction of a mutation into the amino acid sequence of the anti-hTfR antibody may be performed by introducing a mutation into the gene corresponding to the amino acid sequence.

The affinity of an anti-hTfR antibody to hTfR can be adjusted as desired by introduction of a mutation, such as substitution, deletion, and addition, into the amino acid sequence of a variable region of the antibody. For example, if an antibody has such a high affinity to its antigen that leads to too low a dissociation constant in water, there is a possibility that the antibody could, after administered to the body, fail to dissociate from the antigen, thereby leading to a functional disadvantage. In such a case, a most preferable antibody suitable to a given purpose can be obtained by introducing a mutation into the variable region of the antibody so as to adjust its dissociation constant stepwise to 2 to 5 times, 5 to 10 times, 10 to 100 times, and so on, that of the original antibody. Conversely, the dissociation constant can be adjusted stepwise to 1/2 to 1/5 times, 1/5 to 1/10 times, 1/10 to 1/100 times, and so on, that of the original antibody, by introducing a mutation.

Introduction of a mutation such as substitution, deletion and addition to the amino acid sequence of the anti-hTfR antibody can be performed by introducing a mutation into certain positions of the nucleotide sequence of the gene either, for example, by PCR or the like using the gene encoding the anti-hTfR antibody as a template, or by random introduction of a mutation.

Introduction of a mutation into the amino acid sequence of the anti-hTfR antibody for adjusting the affinity of the antibody to hTfR can be carried out by, for example, incorporating a gene encoding the anti-hTfR antibody as a single-chain antibody into a phagemid, preparing with this phagemid a phage with expressed single-chain antibody on the surface of its capsid, letting the phage multiply while introducing a mutation into the gene encoding the single-chain antibody by application of a mutagen or the like, and selecting, from the multiplied phage, a phage expressing a single-chain antibody having a desired dissociation constant either by the method described above or by purification using an antigen column under a certain condition.

If an antibody having a relatively high-affinity to hTfR and obtained by the above method in which those cells producing a high affinity antibody were selected, is an antibody of a non-human animal, it may be converted to a humanized antibody. A humanized antibody is an antibody produced by using an amino acid sequence of part of the variable region (e.g., the whole or part of the CDRs) of a non-human animal antibody, and replacing a proper region of a human antibody with the sequence (implantation) while maintaining the specificity to the antigen. Examples of humanized antibodies include an antibody produced by replacing the three complementarity determining regions (CDRs) in the immunoglobulin light chain and the three complementarity determining regions (CDRs) in the immunoglobulin heavy chain, both constituting a human antibody, with CDRs of a non-human mammal. Though there is no particular limitation as to the biological species from which the CDRs to be incorporated into the human antibody are derived so long as it is a non-human mammal, it preferably is a mouse, rat, rabbit, horse, and non-human primate, more preferably a mouse and rat, and still more preferably a mouse. Here, it is well known that it may be necessary to reproduce the original activity of the donor antibody by replacing the corresponding part of the human antibody as the acceptor by the amino acid sequence of the region outside the CDR which is involved in the structure retention of the CDR or the binding with the antigen, as well as the CDR of the antibody of the non-human mammal. The region outside the CDR is also referred to as a framework region (FR). Thus, the production of the humanized antibody involves transplanting CDRs (and optionally surrounding FRs) of the non-human mammalian antibody in place of the CDRs (and optionally surrounding FRs) of the variable regions of the human antibody.

As aforementioned, in a humanized antibody, the regions of a non-human mammalian animal antibody to be implanted into the variable regions of the original human antibody generally include CDRs themselves, or CDRs and their neighboring part of FRs. However, such FRs implanted together with CDRs also play a role either in maintaining the structure of the CDRs or in binding to the antigen, thus having a substantial function in determining the complementarity of an antibody, and the term "CDR" in the present invention, therefore, refers to such regions that are, or could be, taken from a non-human mammalian animal antibody and implanted into a humanized antibody, in preparing a humanized antibody. Thus, a region generally considered to be in a FR region is included in a CDR in the present invention as far as it takes part either in maintaining the structure of the CDR or in binding to the antigen, and is thus considered to have a substantial function in determining the complementarity of the antigen.

A detailed explanation will be given below regarding the case where the anti-hTfR antibody is a humanized antibody or human antibody. In human antibody light chain, there are λ and κ chains. The light chain constituting the human antibody may either be λ and κ chain. And in human heavy chain, there are γ, µ, α, σ, and ε chains, which correspond to IgG, IgM, IgA, IgD and IgE, respectively. Though the heavy chain constituting the anti-hTfR antibody may be any of γ, µ, α, σ, and ε chains, preferred is a γ chain. Further, in γ chain of human heavy chain, there are γ1, γ2, γ3 and γ4 chains, which correspond to IgG1, IgG2, IgG3 and IgG4, respectively. Where the heavy chain constituting the anti-hTfR antibody is a γ chain, though the γ chain may be any of γ1, γ2, γ3 and γ4 chains, preferred is a γ1 or γ4 chain. In the case where the anti-hTfR antibody is a humanized antibody or human antibody and IgG, the human antibody light chain may either be λ chain or κ chain, and though the human antibody heavy chain may either be γ1, γ2, γ3 and γ4 chains, preferred is a γ1 or γ4 chain. For example, a preferable embodiment of anti-hTfR antibody includes one whose light chain is a λ chain and heavy chain is a γ4 chain, and one whose light chain is a λ chain and heavy chain is a γ1.

In the present invention, the pharmaceutical agent to be conjugated to the anti-hTfR antibody, including a biologically active protein, is the agent to exert its function in muscle. Methods of conjugating the anti-hTfR antibody to such an agent include conjugation via a non-peptide linker or a peptide linker. As the non-peptide linker, polyethylene glycol, polypropylene glycol, copolymer of ethylene glycol and propylene glycol, polyoxyethylated polyol, polyvinyl alcohol, polysaccharide, dextran, polyvinyl ether, biodegradable polymer, lipid polymer, chitin, and hyaluronic acid, a derivative thereof, or a combination thereof can be used. A peptide linker is a peptide chain composed of peptide-bonded 1 to 50 amino acids, or a derivative thereof, wherein the N-terminal and C-terminal ends of the peptide linker form covalent bonds with either an anti-hTfR antibody or the agent, respectively, thereby linking the anti-hTfR antibody and the agent.

The conjugate of an anti-hTfR antibody in the present invention and a pharmaceutical agent using PEG as a non-peptide linker is particularly referred to as an anti-hTfR antibody-PEG-agent. The anti-hTfR antibody-PEG-agent can be produced by conjugating anti-hTfR antibody and PEG to produce anti-hTfR antibody-PEG, followed by conjugation of anti-hTfR antibody-PEG with the agent. Alternatively, the anti-hTfR antibody-PEG-agent can be produced by conjugating a pharmaceutical agent with PEG to produce an agent-PEG, and then conjugating the agent-PEG with an anti-hTfR antibody. PEG modified with a functional group such as carbonate, carbonylimidazole, active ester of carboxylic acid, azlactone, cyclic imidothion, isocyanate, isothiocyanate, imidate, or aldehyde is used to conjugate PEG to anti-hTfR antibody and the agent. The functional group introduced into the PEG reacts mainly with the anti-hTfR antibody and the amino group in the agent molecule, whereby the PEG and hTfR antibody and the agent are covalently bound. There is no particular limitation on the molecular weight and shape of the PEG used at this time, but the average molecular weight (MW) thereof is preferably from MW=500 to 60000, more preferably from MW=500 to 20000. For example, the PEG having an average molecular weight of about 300, about 500, about 1000, about 2000, about 4000, about 10000, about 20000, or the like can be suitably used as a non-peptide linker.

For example, "anti-hTfR antibody-PEG" can be prepared by mixing the anti-hTfR antibody with a polyethylene glycol having aldehyde groups as functional groups (ALD-PEG-ALD) so that the molar ratio of ALD-PEG-ALD to the antibody is 11, 12.5, 15, 110, 120 and the like, and then adding to the mixture a reducing agent such as NaCNBH₃ to let a reaction take place. The anti-hTfR antibody-PEG is then reacted with a pharmaceutical agent in the presence of a reducing agent, such as NaCNBH₃, to obtain an anti-hTfR antibody-PEG-agent. Conversely, an anti-hTfR antibody-PEG-agent can also be obtained by first conjugating the agent with ALD-PEG-ALD to produce an agent-PEG, and then conjugating the agent-PEG with an anti-hTfR antibody.

When the pharmaceutical agent is a biologically active protein (target protein), the anti-hTfR antibody and the target protein can be conjugated to the C-terminal side or the N-terminal side of the heavy or light chain of the anti-hTfR antibody via a linker sequence or directly, respectively, to the N-terminal side or the C-terminal side of the target protein by a peptide bond. The fusion protein obtained by conjugating the anti-hTfR antibody and the target protein in this manner can be obtained by integrating a DNA fragment in which a cDNA encoding the target protein is placed in frame into an expression vector for a mammalian cell in series or by interposing a DNA sequence encoding a linker sequence on the 3' end side or the 5' end side of a cDNA encoding a heavy or light chain of an anti-hTfR antibody, and culturing a mammalian cell into which the expression vector has been introduced. In this mammalian cell, when a DNA fragment encoding a target protein is linked to a heavy chain, an expression vector for a mammalian cell incorporating a cDNA fragment encoding a light chain of an anti-hTfR antibody is also introduced into the same host cell, and when a DNA fragment encoding a target protein is linked to a light chain, an expression vector for a mammalian cell incorporating a cDNA fragment encoding a heavy chain of an anti-hTfR antibody is also introduced into the same host cell. When the anti-hTfR antibody is a single-chain antibody, a fusion protein in which an anti-hTfR antibody and a target protein are conjugated can be obtained by integrating a DNA fragment, in which a cDNA encoding a single-chain anti-hTfR antibody is linked on the 5' end side or the 3' end side of a cDNA encoding the target protein directly or with a DNA sequence encoding a linker sequence interposed therebetween, into an expression vector (for an eukaryotic cell such as a mammalian cell, a yeast, or a prokaryotic cell such as E. coli), and expressing the fusion protein in the cell into which the expression vector is introduced. The fusion protein is to be understood as one form of a conjugate.

In a fusion protein of the type in which a pharmaceutical agent is linked to the anti-hTfR antibody light chain on the C-terminal side thereof, the anti-human transferrin receptor antibody comprises an amino acid sequence including the whole or part of the light chain variable region and an amino acid sequence including the whole or part of the heavy chain variable region, and the agent is linked to the light chain of this anti-human transferrin receptor antibody on the C-terminal side thereof. Here, the anti-hTfR antibody light chain and the agent may be linked together, directly or via a linker.

In a fusion protein of the type in which a pharmaceutical agent is linked to the anti-hTfR antibody heavy chain on the C-terminal side thereof, the anti-human transferrin receptor antibody comprises an amino acid sequence including the whole or part of the light chain variable region and an amino acid sequence including the whole or part of the heavy chain variable region, and the agent is linked to the heavy chain of this anti-human transferrin receptor antibody on the C-terminal side thereof. Here, the anti-hTfR antibody heavy chain and the agent may be linked together, directly or via a linker.

In a fusion protein of the type in which a pharmaceutical agent is linked to the anti-hTfR antibody light chain on the N-terminal side thereof, the anti-human transferrin receptor antibody comprises an amino acid sequence including the whole or part of the light chain variable region and an amino acid sequence including the whole or part of the heavy chain variable region, and the agent is linked to the light chain of this anti-human transferrin receptor antibody on the N-terminal side thereof. Here, the anti-hTfR antibody light chain and the agent may be linked together, directly or via a linker.

In a fusion protein of the type in which a pharmaceutical agent is linked to the anti-hTfR antibody heavy chain on the N-terminal side thereof, the anti-human transferrin receptor antibody comprises an amino acid sequence including the whole or part of the light chain variable region and an amino acid sequence including the whole or part of the heavy chain variable region, and the agent is linked to the heavy chain of this anti-human transferrin receptor antibody on the N-terminal side thereof. Here, the anti-hTfR antibody heavy chain and the agent may be linked together, directly or via a linker.

In the above, the linker sequence placed between the anti-hTfR antibody and the agent may be a peptide chain consisting preferably of 1 to 50, more preferably of 1 to 17, still more preferably of 1 to 10, even more preferably of 1 to 5 amino acids, and in accordance with the agent to be linked to the anti-hTfR antibody, the number of amino acids of the linker sequence may be adjusted to 1, 2, 3, 1-17, 1-10, 10-40, 20-34, 23-31, 25-29, etc., as desired. Though there is no particular limitation as to amino acid sequence of the linker sequence insofar as the anti-hTfR antibody linked by it retains the affinity to hTfR and the agent linked by the linker sequence also exhibit the protein's own physiological activity under a physiological condition, the linker may preferably be composed of glycine and serine. Such linker sequences are preferably, but not limited to, those composed of glycine and serine, for example, amino acid sequence Gly-Ser, amino acid sequence Gly-Gly-Ser, amino acid sequence Gly-Gly-Gly-Gly-Ser (SEQ ID NO: 3), amino acid sequence Gly-Gly-Gly-Gly-Gly-Ser (SEQ ID NO: 4), or a sequence composed of 1 to 50 amino acids in which these amino acid sequences are repeated for 1 to 10 or 2 to 5 times, or a sequence in which these amino acid sequences are repeated for 2 to 17, 2 to 10, 10 to 40, 20 to 34, 23 to 31, or 25 to 29 times, etc., so long as the linked anti-hTfR antibody retain affinity for hTfR and the target protein linked by the linker sequences can exert physiological activity of the protein under physiological conditions. For example, that having the amino acid sequence Gly-Ser can be suitably used as a linker sequence.

In the case where the anti-hTfR antibody is a humanized antibody or a human antibody, the anti-hTfR antibody and a target protein may be bound together by linking the anti-hTfR antibody, at the N-terminus (or the C-terminus) of the heavy chain or light chain, via a linker sequence or directly, to the C-terminus (or the N-terminus), respectively, of the target protein, by peptide bonds. When linking the target protein to the anti-hTfR antibody heavy chain on the N-terminal side (or to the C-terminal side) thereof, the C-terminus (or the N-terminus), respectively, of the target protein is linked to the N-terminus (or the C-terminus) of the γ, µ**,** α, σ or ε chain of anti-hTfR antibody, via a linker sequence or directly, by peptide bonds. When linking the target protein to the anti-hTfR antibody light chain on the N-terminal side (or the C-terminal side) thereof, the C-terminus (or the N-terminus), respectively, of the target protein in linked to the N-terminus (or the C-terminus) of the λ chain and κ chain of anti-hTfR antibody, via a linker sequence or directly, by peptide bonds. However, in the case where the anti-hTfR antibody consists of the Fab region, or of the Fab region and the whole or part of the hinge region (Fab, F(ab')₂, and F(ab')), the target protein may be linked at the C-terminus (or the N-terminus) thereof and via a linker sequence or directly, to the N-terminus (or the C-terminus), respectively, of the heavy chain or light chain that constitutes the Fab, F(ab')₂ and F(ab'), by peptide bonds.

In a fusion protein of the anti-hTfR antibody and a target protein, where the anti-hTfR antibody is a single-chain antibody, the amino acid sequence including the whole or part of the immunoglobulin light chain variable region and the amino acid sequence including the whole or part of the immunoglobulin heavy chain variable region are linked, generally via a linker sequence. Insofar as the affinity of the anti-hTfR antibody to hTfR is retained, the amino acid sequence derived from the light chain may be linked, on the C-terminal side thereof, to a linker sequence which in turn being linked, on the C-terminal side thereof, to the amino acid sequence derived from the heavy chain or, conversely, the amino acid sequence derived from the heavy chain may be linked, on the C-terminal side thereof, to a linker sequence which in turn being linked, on the C-terminal side thereof, to the amino acid sequence derived from the light chain. The same can be established when the single-chain antibody is scFv.

In a single-chain antibody, the linker sequence placed between the light chain and the heavy chain of the immunoglobulin is preferably a peptide chain consisting of preferably 2 to 50, more preferably 8 to 50, still more preferably 10 to 30, even more preferably 12 to 18, or 15 to 25, for example 15 or 25 amino acid residues. Such a linker sequence is preferably, but not limited to, that made of glycine only or of glycine and serine, for example, amino acid sequence Gly-Ser, amino acid sequence Gly-Gly-Ser, amino acid sequence Gly-Gly-Gly, amino acid sequence Gly-Gly-Gly-Gly-Ser (SEQ ID NO: 3), amino acid sequence Gly-Gly-Gly-Gly-Gly-Ser (SEQ ID NO: 4), or a sequence in which these amino acid sequences are repeated for 2 to 10 or 2 to 5 times , as far as the anti-hTfR antibody to which both chains are linked retains affinity for hTfR and the target protein bound to the antibody exhibits its physiological activity under physiological conditions. As a preferable embodiment of the linker sequence, there is a sequence consisting of 15 amino acids in which the amino acid sequence Gly-Gly-Gly-Gly-Ser (SEQ ID NO: 3) is repeated for three times.

Where the anti-hTfR antibody is a single-chain antibody, such a fusion protein can be produced by, for example, transforming host cells such as mammalian cells with an expression vector having an incorporated DNA fragment containing a nucleotide sequence encoding the fusion protein, and then culturing the host cells.

Besides, in the present invention, when a peptide chain includes a plurality of linker sequences, each of those linker sequences is designated, from the N-terminal side, the first linker sequence, the second linker sequence, and so on, for convenience.

The target protein to be fused with the anti-hTfR antibody is not particularly limited insofar as the protein is to function in muscle. Preferred target protein includes, for example, a lysosomal enzyme encoded by a gene responsible for lysosomal diseases that cause muscle dysfunction. Such a lysosomal enzyme includes human acid α-glucosidase (hGAA) and human α-galactosidase A, but is not limited thereto, also includes α-L-iduronidase (IDUA), iduronate-2-sulfatase (IDS), glucocerebrosidase (GBA), β-galactosidase, GM2 activator protein, β-hexosaminidase A, β-hexosaminidase B, N-acetylglucosamine-1-phospholansferase, α-mannosidase (LAMAN), β-mannosidase, galactosylceramidase (GALC), saposin C, arylsulufatase A (ARSA), α-L-fucosidase (FUCA1), aspartylglucosaminidase, α-N-acetylgalactosaminidase, acid sphingomyelinase (ASM), β-glucuronidase (GUSB), heparan N-sulfatase (SGSH), α-N-acetylglucosaminidase (NAGLU), acetyl-CoA: α-glucosaminide N-acetyltransferase, N-acetylglucosamine-6-sulfate sulfatase, acid ceramidase (AC), amylo-1,6-glucosidase, sialidase, aspartyl glucosaminidase, palmitoyl protein thioesterase-1 (PPT-1), tripeptidyl peptidase-1 (TPP-1), hyaluronidase-1, CLN1, CLN2, and the like.

Hereinafter, a fusion protein of an anti-hTfR antibody and a lysosomal enzyme will be described in detail by taking acid α-glucosidase and α-galactosidase A as examples.

When administered to a Pompe disease patient, the fusion protein of the anti-hTfR antibody and human acid α-glucosidase (hGAA) is incorporated into the patient's muscle cells and transported to lysosomes. Thus, it degrades glycogen accumulated in lysosomes of muscle cells of the patient with Pompe disease. In the case of Pompe disease, the target is the muscles consisting mainly of skeletal muscle cells and the muscles consisting mainly of heart muscle cells. In Pompe disease, the functional impairment of the heart and skeletal muscles is the main symptom, but by administering this fusion protein, the glycogen accumulated in the skeletal muscle cells and heart muscle cells is decomposed, which results in the improvement of symptoms such as forcibly expand, muscle weakness based on skeletal muscle invasion, and weakness of muscle tension. Thus, the fusion protein of the anti-hTfR antibody and human acid α-glucosidase can be used as a therapeutic agent for skeletal muscular dysfunction or/and cardiac disorders in Pompe disease.

Suitable examples of fusion proteins of anti-hTfR antibody and human acid α-glucosidase include a fusion protein of human acid α-glucosidase and anti-hTfR antibody, wherein the light chain of the humanized anti-hTfR antibody has the amino acid sequence set forth as SEQ ID NO: 24, and wherein the heavy chain of the humanized anti-hTfR antibody is linked to human acid α-glucosidase on its C-terminal side via a linker sequence Gly-Ser, and the whole has the amino acid set forth as SEQ ID NO: 27.

Another example is a fusion protein of a human acid α-glucosidase and an anti-hTfR antibody, the fusion protein comprising a light chain of an anti-hTfR antibody having the amino acid sequence set forth as SEQ ID NO: 24, and a heavy chain of an anti-hTfR antibody having the amino acid sequence set forth as SEQ ID NO: 25 and linked on its C-terminal side to the human acid α-glucosidase set forth as SEQ ID NO: 1 via a linker sequence (Gly Ser).

When administered to a Fabry disease patient, the fusion protein of the anti-hTfR antibody and human α-galactosidase A (ha-GalA) is incorporated into the patient's muscle cells and transported to lysosomes. Thus, it degrades trihexosylceramide accumulated in lysosomes of muscle cells of the patient with Fabry disease. In the case of Fabry disease, the target is the muscles consisting mainly of heart muscle cells. In Fabry disease, cardiovascular abnormalities are found, but by administering this fusion protein, the trihexosylceramide accumulated in heart muscle cells is decomposed, which can improve various symptoms of cardiovascular abnormalities such as ventricular expansion. This fusion protein is particularly effective for cardiac Fabry disease, an atypical type of Fabry disease in which the heart is primarily damaged. Thus, the fusion protein of the anti-hTfR antibody and human α-galactosidase A can be used as a therapeutic agent for cardiac disorders in Fabry disease, particularly cardiac Fabry disease.

Suitable examples of fusion proteins of anti-hTfR antibody and human α-galactosidase A include a fusion protein of human α-galactosidase A and anti-hTfR antibody, wherein the light chain of the humanized anti-hTfR antibody has the amino acid sequence set forth as SEQ ID NO: 24, and wherein the heavy chain of the humanized anti-hTfR antibody is linked to human α-galactosidase A on its C-terminal side via linker sequence Gly-Ser, and the whole has the amino acid sequence set forth as SEQ ID NO: 29.

Another example is a fusion protein of a human acid α-glucosidase and an anti-hTfR antibody, the fusion protein comprising a light chain of an anti-hTfR antibody having the amino acid sequence set forth as SEQ ID NO: 24, and a heavy chain of an anti-hTfR antibody having the amino acid sequence set forth as SEQ ID NO: 25 and linked on its C-terminal side to the human α-galactosidase A set forth as SEQ ID NO: 2 via a linker sequence (Gly Ser).

The conjugate of an anti-hTfR antibody and a pharmaceutical agent can be used as a pharmaceutical composition to be administered into the blood to exert a therapeutic effect in muscle. Such a drug is generally administered to a patient by intravenous injection such as intravenous drip injection, subcutaneous injection, intramuscular injection, or the like, but the route of administration is not particularly limited.

The conjugate of the anti-hTfR antibody and the agent of the present invention can be provided to a medical institution as a pharmaceutical composition in the form of a lyophilized product, an aqueous solution, or the like. In the case of the aqueous solution, the pharmaceutical composition may be provided as a formulation sealed in a vial or syringe, pre-dissolved in a solution containing a stabilizer, buffer, isotonic agent. The formulation sealed in a syringe is commonly referred to as a prefilled syringe formulation. The prefilled syringe formulation can simplify the administration of the drug by the patient himself.

Where an aqueous preparation is provided, the concentration of the conjugate in the aqueous preparation is, e.g., 1-4 mg/mL, though it is to be adjusted as desired in accordance with the dosage. Where there is no particular limitation as to stabilizers to be contained in the aqueous preparation insofar as they are pharmaceutically available, nonionic surfactants may preferably be used. Examples of such nonionic surfactants include polysorbate and poloxamer, either of which may be used alone or in combination. Among polysorbates, polysorbate 20 and polysorbate 80 are preferably used. As poloxamer, poloxamer 188 (polyoxyethylene (160) polyoxypropylene (30) glycol) is particularly preferred. Further, the concentration of nonionic surfactant contained in the aqueous preparation is preferably 0.01-1 mg/mL, more preferably, 0.01-0.5 mg/mL, and still more preferably 0.1-0.5 mg/mL. As stabilizers, amino acids such as histidine, arginine, methionine, and glycine may also be used. Where employed as a stabilizer, the concentration of an amino acid in the aqueous preparation is preferably 0.1-40 mg/mL, more preferably 0.2-5 mg/mL, and still more preferably 0.5-4 mg/mL. While there is no particular limitation as to a buffer to be contained in the aqueous preparation insofar as it is pharmaceutically available, phosphate buffer is preferred, and more preferred is sodium phosphate buffer. Where used as a buffer, the concentration of sodium phosphate is preferably 0.01-0.04 M. The pH of the aqueous preparation adjusted with a buffer is preferably 5.5-7.2. While there is no particular limitation as to an isotonizer to be contained in the aqueous preparation insofar as it is pharmaceutically available, sodium chloride or mannitol may be preferably used alone or in combination as an isotonizer.

### Examples

Though the present invention is described in further detail below with reference to examples, it is not intended that the present invention be limited to those examples.

### [Example 1] Construction of hTfR expression vector

Employing human spleen Quick Clone cDNA (Clontech Inc.) as a template and using primer hTfR5' (SEQ ID NO:7) and primer hTfR3' (SEQ ID NO:8), PCR was performed to amplify the gene fragment encoding human transferrin receptor (hTfR). The amplified fragment encoding hTfR was digested with MluI and NotI, and then inserted between MluI and NotI sites of vector pCI-neo (Promega Inc.). The vector thus prepared was designated pCI-neo(hTfR). This vector then was digested with MluI and NotI to cut out the gene fragment encoding hTfR, and this fragment was inserted between MluI and NotI sites of pE-mIRES-GS-puro, an expression vector disclosed in an international publication WO 2012/063799 to construct an hTfR expression vector, pE-mIRES-GS-puro(hTfR).

### [Example 2] Preparation of recombinant hTfR

Into CHO-K1 cells was introduced pE-mIRES-GS-puro(hTfR) by electroporation, and the cells then were subjected to selection culture in a CD OptiCHO™ medium (Invitrogen Inc.) containing methionine sulfoximine (MSX) and puromycin to prepare recombinant hTfR expressing cells. The recombinant hTfR expressing cells were cultured, and recombinant hTfR was prepared.

### [Example 3] Immunization of mouse with recombinant hTfR

Mice were immunized with recombinant hTfR prepared in Example 2 as antigen. Immunization was carried out by intravenously or intraperitoneally injecting the mice with the antigen.

### [Example 4] Preparation of hybridomas

About one week after the last injection, the spleens of the mice were excised and homogenized to isolate spleen cells. The spleen cells thus obtained were fused with cells of mouse myeloma cell line (P3.X63.Ag8.653) by the polyethylene glycol method. After cell fusion, the cells were suspended in a RPMI 1640 medium containing (1X) HAT supplement (Life Technologies Inc.) and 10% Ultra low IgG fetal bovine serum (Life Technologies Inc.), and the cell suspension was dispensed to 20 of 96-well plates, 200 µL/well. After the cells were cultured for 10 days in a carbon dioxide gas incubator (37°C, 5% CO₂), each well was examined under a microscope, and the wells that contain a single colony were selected. When the cells in each well reached near confluence, the culture supernatant was collected as a culture supernatant of hybridoma, and subjected to the following screening process.

### [Example 5] Screening of high affinity antibody producing cell line

The recombinant hTfR solution (Sino Biologics Inc.) was diluted with 50 mM sodium phosphate buffer (pH 9.5-9.6) to 5 µg/mL to prepare a solid phase solution. After 50 µL of the solid phase solution was added to each well of a Nunc MaxiSorp™ flat-bottom 96-well plate (substrate: polystyrene, mfd. by Nunc Inc.), the plate was left to stand for one hour at room temperature to let the recombinant hTfR adhere to the plate and become immobilized. The solid phase solution was discarded, each well was washed three times with 250 µL of washing solution (PBS containing PBS-T: 0.05% Tween20), 200 µL of a blocking solution (PBS containing 1% BSA) then was added to each well, and the plate was left to stand for one hour at room temperature.

The blocking solution was discarded, and each well was washed three times with 250 µL of PBS-T. To each well was added 50 µL of the hybridoma culture supernatant, and the plate was left to stand for one hour at room temperature to let the mouse anti-hTfR antibody contained in the culture supernatant bind to the recombinant hTfR. At the same time, to some wells was added 50 µL of culture supernatant of a hybridoma that did not produce mouse anti-hTfR antibody, as a control. In addition, 50 µL of the medium for hybridoma culture was added to the wells, as mock wells, beside those wells to which the culture supernatant was added. Measurement was conducted in an n=2 fashion. Then, the solution was discarded, and each well was washed three times with 250 µL of PBS-T.

To each of the above wells was added 100 µL of HRP-labeled goat anti-mouse immunoglobulin antibody solution (Promega Inc.), and the plate was left to stand for one minute at room temperature. The solution then was discarded, and each well was washed three times with 250 µL of PBS-T. To each well as added 50 µL of a chromogenic substrate solution, TMB Stabilized Substrate for Horseradish Peroxidase (Promega Inc.), and the wells were left to stand for 10 to 20 minutes at room temperature. Then, following addition of 100 µL of a stop solution (2N sulfuric acid), the absorbance of each well was measured on a plate reader at 450 nm. Of the two wells for each of the culture supernatant and control, the mean values were taken, respectively, and from each of the mean values, the respective mean value for the two mock wells placed corresponding to each of the culture supernatant and the control, was subtracted, giving the measurement.

Fourteen types of hybridoma cells corresponding to culture supernatants added to the wells which exhibited the higher measurements were selected as the cell lines (high affinity antibody producing cell line) that produce antibodies exhibiting high affinities to hTfR (high affinity anti-hTfR antibody). These fourteen types of cell lines were designated as Clone 1 line to Clone 14 line. Anti-hTfR antibodies produced by clonal cell lines 1 to 14 were designated as anti-hTfR antibody No. 1 to anti-hTfR antibody No. 14, respectively.

### [Example 6] Analysis of the variable-region amino acid sequence of the high affinity anti-hTfR antibodies

Further clone 3 line was selected from clone 1 line to clone 14 line selected in Example 5. A cDNA of clone 3 line was prepared, and the gene encoding the light chain and heavy chain of an antibody was amplified using this cDNA as a template. The nucleic acid sequence of the amplified gene was translated to determine the amino acid sequence of the variable region of the light chain and heavy chain of the anti-hTfR antibody No. 3 produced by this clone line.

The anti-hTfR antibody No.3 was found to include the amino acid sequence set forth as SEQ ID NO:9 as the light chain variable region, and the amino acid sequence set forth as SEQ ID NO: 10 as the heavy chain variable region. The light chain variable region was found to include the amino acid sequence set forth as SEQ ID NO: 11 or 12 as CDR1; SEQ ID NO: 13 or 14 as CDR2, and SEQ ID NO:15 as CDR3; and the heavy chain variable region to include the amino acid sequence set forth as SEQ ID NO: 16 or 17 as CDR1, SEQ ID NO: 18 or 19 as CDR2, and SEQ ID NO:20 or 21 as CDR3. However, it was also considered that CDRs are not limited to those which consist of these amino acid sequences, but they can also either be regions of amino acid sequences that include any of the above sequences, or amino acid sequences consisting of not less than three consecutive amino acids containing part of the above sequences.

### [Example 7] Measurement of the affinity of anti-hTfR antibody to human and monkey TfRs

The affinity of the anti-hTfR antibody to human and monkey TfRs were measured on Octet RED96 (ForteBio Inc., a division of Pall Corporation), a system for analysis of interactions between biomolecules utilizing bio-layer interferometry (BLI). The basic principles of bio-layer interferometry are briefly explained below. When a layer of a biomolecule immobilized on the surface of a sensor tip is irradiated with light of a certain wavelength, the light is reflected from two of the surfaces, the one of the biomolecule and the other of inner, reference layer, producing interfering light waves. A molecule in the sample being measured binds to the biomolecule on the surface of the sensor tip and thus increases the thickness of the layers on the sensor tip, which results in a shift between the interfering waves. By measuring the variations of this shift between the interfering waves, determination of the number of the molecules bound to the layer of the biomolecules immobilized to the sensor tip surface and kinetic analysis of it can be performed in real time. The measurement was performed according generally to the operating manual attached to Octet RED96. As a human TfR, a recombinant human TfR (r human TfR: Sino Biological Inc.) was used, which had the amino acid sequence of the hTfR extracellular region, i.e., the cysteine residue at position 89 from the N-terminal side to the phenylalanine at the C-terminus, of the amino acid sequence set forth as SEQ ID NO: 1, with a histidine tag attached to the N-terminus. As a monkey TfR, a recombinant monkey TfR (r monkey TfR: Sino Biological Inc.) was used, which had the amino acid sequence of TfR extracellular region of Macaca fascicularis, i.e., the cysteine residue at position 89 from the N-terminal side to the phenylalanine at the C-terminus, of the amino acid sequence set forth as SEQ ID NO:2, with a histidine tag attached to the N-terminus.

Clone 3 line selected in Example 6 was diluted with a RPMI 1640 medium containing (IX) HAT Supplement (Life Technologies Inc.) and 10% Ultra low IgG fetal bovine serum (Life Technologies Inc.) so as to adjust the cell density to approximately 2×10⁵ cells/mL. To a 1 L conical flask were added 200 mL of each cell suspension, and the culture was performed for 6 to 7 days in a wet environment at 37°C, 5% CO₂ and 95% air, with stirring at a rate of about 70 rpm. The culture supernatant was centrifuged, and then filtered through a 0.22 µm filter (Millipore Inc.) to collect a culture supernatant. The culture supernatant thus collected was loaded onto a Protein G column (column volume: 1 mL, GE Healthcare Inc.) that had been equilibrated in advance with three column volumes of 20 mM Tris buffer (pH 8.0) containing 150 mM NaCl. After the column was washed with 5 column volumes of the same buffer, adsorbed antibody was eluted with 4 column volumes of 50 mM glycine buffer (pH 2.8) containing 150 mM NaCl, and eluted fractions were collected. The fractions eluted were adjusted to pH 7.0 by addition of 1 M Tris buffer (pH 8.0). These were used as purified products of anti-hTfR antibody No. 3 in the experiments described below.

Purified anti-hTfR antibody No. 3 was diluted in two steps with HBS-P + (10mM HEPES containing 150 mM NaCl, 50 µM EDTA and 0.05% Surfactant P20), respectively, to prepare antibody solutions of 7 different concentrations, 0.78125 to 50 nM (0.117 to 7.5 µg/mL). These antibody solutions were used as sample solutions. The r human and r monkey TfRs were respectively diluted with HBS-P+ to prepare 25 µg/mL solutions, which were used as r human TfR-ECD (Histag) solution and r monkey TfR-ECD (Histag) solution, respectively.

Each of the sample solutions prepared above by 2-fold dilution steps was added, 200 µL/well, to a 96-well plate, black (Greiner Bio-One Inc.). Each of the r human TfR-ECD (Histag) solution and the r monkey TfR-ECD (Histag) solutions prepared above was added, 200 µL/well, to predetermined wells. To respective wells for baseline, dissociation and washing were added HBS-P+, 200 µL/well. To wells for regeneration were added 10 mM Glycine-HCl (pH 1.7), 200 µL/well. To wells for activation was added 0.5 mM NiCl₂ solution, 200 µL/well. The plate and biosensor (Biosensor/Ni-NTA: ForteBio Inc., a division of Pall Corporation) were set in the prescribed positions of Octet RED96.

Octet RED96 was run under the conditions shown in Table 1 below to collect data, on which then, using the analyzing software attached to Octet RED96, and fitting the binding reaction curve to 1:1 binding model or 2:1 binding model, the association rate constant (kₒₙ) and dissociation rate constant (k_{off}) of anti-hTfR antibody to r human TfR and r monkey TfR were measured and the dissociation constant (K_{D}) was calculated. The measurement was performed at 25 to 30°C.

### [Table 1]

**Table 1 Operating Conditions of OctetRED96**

| | Step | Sensor contact time (sec) | Rate (rpm) | Threshold |
|---|---|---|---|---|
| 1 | Base line 1 | 60 | 1000 | - |
| 2 | Loading | 600 | 1000 | 1.5 - 2.0 |
| 3 | Base line 2 | 60 | 1000 | - |
| 4 | Association | 180 | 1000 | - |
| 5 | Dissociation | 540 | 1000 | - |
| 6 | Regeneration | 5 | 1000 | - |
| 7 | Washing | 5 | 1000 | - |
| The steps 6 to 7 above are repeated for 6 to 7 times. | | | | |
| 8 | Activation | 60 | 1000 | - |
| The steps 1 to 8 are repeated until all samples have been measured. | | | | |

The dissociation constant (K_{D}) with human TfR of anti-hTfR No. 3 was not more than 1X10⁻¹² M, and the dissociation constant (K_{D}) with monkey TfR was not more than 1X10⁻¹² M. These results indicate that anti-hTfR antibody No. 3 is an antibody with higher affinity not only for human TfR but also for monkey TfR.

### [Example 8] Preparation of humanized anti-hTfR antibodies

Humanization was tried of the amino acid sequence included in the light chain and the heavy chain variable regions of anti-hTfR antibody No. 3. Thus the variable region of the humanized light chain having the amino acid sequence set forth as SEQ ID NO: 22 and the variable region of the humanized heavy chain having the amino acid sequence set forth as SEQ ID NO: 23 were obtained. Further, for the light chain, a light chain having the amino acid sequence set forth as SEQ ID NO: 24 and having the amino acid sequence set forth as SEQ ID NO: 22 in the variable region was obtained. As for the heavy chain, a heavy chain having the amino acid sequence set forth as SEQ ID NO: 25 and having the amino acid sequence set forth as SEQ ID NO: 23 in the variable region was obtained. The heavy chain of the amino acid sequence set forth as SEQ ID NO: 25 is of IgG4 type. The dissociation factor (K_{D}) of the humanized anti-hTfR antibody with human TfR thus obtained was measured by the method described in Example 7. The results show that the dissociation constant (K_{D}) of humanized anti-hTfR antibody with human TfR was not more than IX10⁻¹² M, and the dissociation constant (K_{D}) with monkey TfR was about 1X10⁻⁹ M. These results indicate that the obtained humanized antibody of anti-hTfR antibody No. 3 has high affinity not only for human TfR but also for monkey TfR.

### [Example 9] Construction of a gene encoding the fusion protein of anti-TfR antibody and hGAA

A DNA fragment encoding the light chain of the amino acid sequence set forth as SEQ ID NO: 24 (SEQ ID NO: 26) was synthesized. The fragment has, on the 5' side, in order from the 5' end, a MluI sequence and a sequence encoding a leader peptide which functions as a secretory signal, and a NotI sequence on the 3' side.

In addition, a DNA fragment (SEQ ID NO: 28) encoding a protein of the amino acid sequence set forth as SEQ ID NO: 27 as a whole was synthesized in that protein a human acid α-glucosidase of the amino acid sequence set forth as SEQ ID NO: 1 was linked on the C-terminal side of the heavy chain of the amino acid sequence set forth as SEQ ID NO: 25 via a linker sequence consisting of Gly-Ser. The DNA fragment has, on the 5' side, in order from the 5' end, a MluI sequence and a sequence encoding a leader peptide which functions as a secretory signal, and a NotI sequence on the 3' side.

### [Example 10] Construction of an expression vector for fusion protein of anti-TfR antibody and hGAA

A pEF/myc/nuc vector (Invitrogen Inc.) was digested with KpnI and Ncol, the region containing the EF-1α promoter and its first intron was excised and blunt-ended with T4 DNA polymerase. A pCI-neo vector (Invitrogen Inc.) was digested with Bg1II and EcoRI to excise the region containing CMV enhancer/promoter and intron, followed by blunt-end treatment with T4 DNA polymerase. Into this, the region containing the EF-1α promoter and its first intron described above was inserted to construct a pE-neo vector. A pE-neo vector was digested with SfiI and BstXI, and approximately 1 kbp region containing neomycin resistance gene was excised. Using PcDNA3.1/Hygro(+) (Invitrogen Inc.) as a template, Hygromycin resistance gene was amplified by PCR-reaction using primer Hyg-Sfi5' (SEQ. NO. 30) and primer Hyg-BstX3' (SEQ. NO. 31). The amplified hygromycin resistance gene was digested with SfiI and BstXI, and the neomycin resistance gene was inserted into the excised pE-neo vector to construct a pE-hygr vector.

A pE-hygr vector and a pE-neo vector were digested with MluI and NotI, respectively. The DNA fragment encoding the light chain of the anti-hTfR antibody synthesized in Example 9 (SEQ ID NO: 26) and the DNA fragment encoding the protein in which the heavy chain of the anti-hTfR antibody and human acid α-glucosidase were linked (SEQ ID NO: 28) were digested with MluI and NotI, and inserted between MluI-NotI of pE-hygr vector and pE-neo vector, respectively. The obtained vectors were used as a pE-hygr (LC), a vector for expression of light chain of a humanized anti-hTfR antibody, and a pE-neo(HC-hGAA, a vector for expression of protein in which a heavy chain of an anti-hTfR antibody and human acid α-glucosidase were linked, respectively.

### [Example 11] Preparation of cells for expressing hGAA-humanized anti-hTfR antibody fusion protein

CHO cells (CHO-K1: obtained from American Type Culture Collection) we transformed with pE-hygr (LC) and pE-neo(HC-hGAA) constructed in Example 10 using GenePulser(Bio-Rad, Inc.) according to the method described below. The transformation of the cell was generally carried out in the following manner. 5X10⁶ of CHO-K1 cells were seeded in a 3.5 cm culture dish with CD OptiCHO™ medium (Life Technologies Inc.) added, and were grown overnight under the conditions of 37°C and 5% CO₂. The medium was exchanged for Opti-MEM™ I medium (Life Technologies Inc.) and the cells were suspended at the density of 5X10⁶ cells/mL. 100 µL of the cell suspension was collected, and 5 µL each of the pE-hygr(LC1) plasmid DNA solution and the pE-neo(HC1) plasmid DNA solution, both diluted to 100 µg/mL in Opti-MEM medium™ I medium, were added to the cell suspension. Electroporation was performed using GenePulser (Bio-Rad Inc.) and plasmids were introduced into the cells. The cells then were cultured overnight under the condition of 37°C, 5% CO₂, and subjected to selection culture in CD OptiCHO™ medium supplemented with 0.5 mg/mL of hygromycin and 0.8 mg/mL of G418.

Then, the cells selected above through the selection culture were seeded on 96-well plates so that not more than one cell was seeded per well by limiting dilution. The cells then were cultured for about 10 days so that monoclonal colonies were formed. Respective culture supernatants of the wells in which monoclonal colony was formed were collected, the amount of the humanized antibody contained in culture supernatants was determined by ELISA, and hGAA-humanized anti-hTfR antibody-fusion protein high-expressing cell lines were selected.

The ELISA above was conducted as follows in general. To each well of 96-well microtiter plates (Nunc Inc.) were added 100 µL of a goat anti-human IgG polyclonal antibody solution diluted with 0.05 M sodium bicarbonate buffer (pH 9.6) to 4 µg/mL, and the plate was left to stand for at least one hour at room temperature so as to allow the antibody to be adsorbed by the plates. Then, after the well were washed three times with PBS-T, 200 µL of Starting Block (PBS) Blocking Buffer (Thermo Fisher Scientific Inc.) was added to each well, and the plates were left to stand for 30 minutes at room temperature. After each well was washed with PBS-T three times, the culture supernatant or the human IgG standard product which had been diluted with a PBS-BT (PBS supplemented with 0.5% BSA and 0.05% Tween20) to appropriate concentrations, was added to each well, in the amount of 100 µL, and the plates were left to stand for at least one hour at room temperature. After the plates were washed three times with PBS-T, 100 µL of HRP-labeled anti-human IgG polyclonal antibody solution which had been diluted with PBS-BT, was added to each well, and the plates were left to stand for at least one hour at room temperature. After the wells were washed three times with PBS-T, 0.4 mg/mL o-phenylenediamine in citrate-phosphate buffer (pH 5.0) was added to each well, in the amount of 100 µL, and the wells were left to stand for 8 to 20 minutes at room temperature. Then, 1 mol/L sulfuric acid was added to each well, in the amount of 100 µL to terminate the reaction, and the absorbance for each well was measured at 490 nm using a 96-well plate reader. The cells corresponding to the wells which exhibited the higher measurements were regarded as a hGAA-humanized anti-hTfR antibody-fusion protein high-expressing cell line. The hGAA-humanized anti-hTfR antibody-fusion protein produced by this cell line was designated as hGAA-anti-hTfR antibody.

### [Example 12] Production of the hGAA-Anti-hTfR Antibody

The hGAA-anti-hTfR antibody was produced in the following manner. The high-expressing cell line of the hGAA-anti-hTfR antibody obtained in Example 11 was diluted in CD OptiCHO™ medium to a cell concentration of about 2X10⁵ cells/mL, 200 mL of cell suspension was added to 1 L Erlenmeyer flask, and incubated at 37°C in a wet environment consisting of 5% CO₂ and 95% air at a stirring rate of about 70 rpm for 6-7 days. The culture supernatant was collected by centrifugation and filtered through a 0.22 µm filter (Millipore Inc.) to obtain a culture supernatant. To the harvested culture supernatant was added 5 column volumes of 20 mM Tris buffer (pH 8.0) containing 150 mM NaCl and it was loaded onto a Protein A column (column volume: 1 mL, Bio-Rad Inc.) that had been pre-equilibrated with 3 column volumes of 20 mM Tris buffer (pH 8.0) containing 150 mM NaCl. The column was then washed with 5 column volumes of the same buffer and the adsorbed hGAA-anti-hTfR antibody was eluted with 4 column volumes of 50 mM glycine buffer (pH 2.8) containing 150 mM NaCl. The pH of this eluate containing the hGAA-anti-hTfR antibody was adjusted to pH 7.0 by adding 1 M Tris buffer (pH 8.0), and then buffer-exchanged to PBS using an Amicon Utra 30 kDa membrane (Millipore Co.). Thus obtained was used as a purified product of the hGAA-anti-hTfR antibody.

### [Example 13] Measurement of incorporation of hGAA into cells by using human skeletal muscle

Human skeletal myocytes (PromoCell Inc.) are diluted to 8.0X10⁴ cells/mL by Skeletal Muscle Cell Growth Medium (Ready-to-use) (PromoCell Inc.) and seeded into the wells of a 96 well plate coated with Cellnest™ (Fujifilm Inc.) in 250 µL/well. After culturing at 37°C in 5% CO₂ for 3 days, the medium is replaced with medium for inducing skeletal myocyte differentiation, Skeletal Muscle Cell Differentiation Medium (Ready-to-use) (PromoCell Inc.). Then, every 2 days, the medium is changed with the medium for induction of differentiation, and differentiation is induced for 6 days. After removing the medium, 100 µL of each of serially diluted solutions of the hGAA-anti-hTfR antibody and hGAA (0.005-20.0 µg/mL), diluted with the medium for inducing differentiation, is added to the well as a test substance, and the cells is further incubated at 37°C and 5% CO₂ for an additional 20 hours.

After incubation, the medium is removed and each well is washed three times with 300 µL of PBS. 100 µL of M-PER™ (with 0.5 % Protease Inhibitor Cocktail, Thermo Fisher Scientific Inc.) is added to each well, and the plate is shaken by a plate shaker at room temperature for 25 minutes to prepare a cellular extract. 20 µL of the extract from each well is collected and the protein contained in the extract is determined by using BCA protein assay kit (Pierce Inc.). In addition, 80 µL of PBS-BT is added to the wells after collecting 20 µL of the extract, and the plate is shaken for 10 minutes at room temperature on a plate shaker to prepare a cellular extract to be subjected to ELISA measurement.

A cell extract is prepared in the same manner from cells in wells to which the test substance is not added, and this extract is used as a pool extract. The hGAA-anti-hTfR antibody and commercial available hGAA are serially diluted with pooled extracts to prepare calibration curve samples. The test substance can be quantitated by the following ELISA method.

Rabbit anti-hGAA polyclonal antibody obtained by immunizing a rabbit with hGAA are diluted to 5 µg/mL with 20 mmol/L sodium carbonate buffer (pH 9.0), added to each well of a 96 well plate (Maxisorp™) in 100 µL /well, and shaken for 1 hour at room temperature on a plate shaker. The antibody solution is removed and 300 µL of blocking buffer (PBS with 1% BSA) is added to each well and left to stand for 1 hour at room temperature. The wells are then washed three times with 300 µL of PBS-T. And 100 µL of each of the standard curve sample and cell extract is added to the well, and the plate is shaken for 1 hour at room temperature on a plate shaker. Each well is washed three times with 300 µL of wash solution, 100 µL of Biotin labeled rabbit anti-hGAA antibody (prepared in-house) in PBS-BT is added to each well, and the plate is shaken on a plate shaker at room temperature for 1 hour. After washing each well three times with 300 µL of wash solution, 100 µL of NeutrAvidin-HRP conjugate (Pierce Inc.) diluted with 0.5% BSA/PBS (with 0.05% Tween20) is added to each well, the plate is shaken for 15 minutes at room temperature on a plate shaker, and then each well is washed three times with 300 µL of wash solution. The mixed solution of TMB Peroxidase Substrate Solution B of TMB Microwell Peroxidase Substrate (2-Component System, KPL Inc.) and TMB Peroxidase Substrate in equal amounts is added to each well in 100 µL/well and a color reaction is developed at room temperature. After adequate color development is obtained, 100 µl of 1 mol/L phosphate is added to each well and the reaction is stopped. The absorbance at 450 nanometers is measured with a microplate reader, a calibration curve is prepared by using SoftMax Pro (Molecular Device Inc.), and the concentrations of each test substance are calculated by interpolating the measured values of each test substance to the calibration curve. The concentration of the test substance in each cell extract is divided by the protein concentration to obtain the amount of the test substance taken into the cell (ng/mg protein).

### [Example 14] Preparation of CD71-KI/GAA-KO mice

CD71-KI/GAA-KO mice knocking out the acid α-glucosidase gene (GAA gene) and knocking in the human transferrin receptor gene (CD71 gene) were generated by common genetic engineering techniques. The production method was generally as follows. A targeting vector (GAA knockout vector) capable of knocking out the mouse GAA gene was constructed by inserting a stop codon and a neomycin resistance gene into BamHI site present in exon 6 of the mouse GAA gene. The targeting vector comprises the nucleic acid sequence set forth as SEQ ID NO: 32. After linearization of the targeting vector, the vector was introduced into the ES cells of C57BL/6 lineage by electroporation. After the introduction, selective culture using neomycin drug resistance as an index was carried out, and drug-resistant ES clones were obtained. The obtained drug-resistant ES clones were screened by genomic PCR and Southern blot, and clones in which specific homologous recombination was achieved (homologous recombination ES clones) were selected. Using homologous recombination ES clones and 8-cell stage embryos of ICR lineage, chimeric embryos were produced by aggregation method. Recipient mouse was implanted with chimeric embryos to deliver chimeric mice.

The chimeric mice were naturally mated to produce GAA-KO hetero mice, and GAA-KO homo mice were produced through repeated mating. CD71-KI hetero mice and GAA-KO homo mice were mated and the resulting mice were screened to produce CD71-KI/GAA-KO mice. CD71-KI hetero mice were prepared according to the method described in Sonoda H., et al. Molecular Therapy 26. 1366-74 (2018).

### [Example 15] Measurement of hGAA incorporation into cells in mice

Purified product of hGAA-anti-hTfR antibody prepared in Example 12 and commercial available hGAA for medical use were each prepared as 4 mg/mL solutions using physiological saline. Using these solutions, CD71-KI/GAA-KO mice were administered hGAA-anti-hTfR antibody or hGAA via the tail veins, respectively. Both hGAA-anti-hTfR antibody and hGAA were administered at a dose of 20 mg/dose for a total of 4 doses every other week. Two weeks after the last dose, the mice were exsanguinated to death, and the heart, diaphragm, soleus, tibialis anterior, and quadriceps muscles were collected. The collected tissue was washed with saline. The concentrations of glycogens in these muscular tissues, mg/g wet tissue weights, were measured as described in Example 16. The group that received hGAA-anti-hTfR antibody was designated as the hGAA-anti-hTfR antibody-administered group and the group that received commercially available medical hGAA was designated as the hGAA-administered group. CD71-KI/GAA-KO mice administered with an equal volume of saline were used as the KO-control group. In addition, wild-type mice that received an equal volume of saline were used as the wild-type control group. Each group consisted of 5 mice.

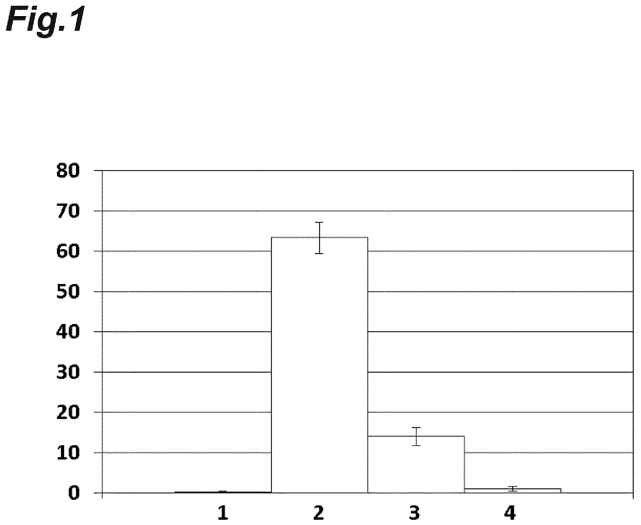

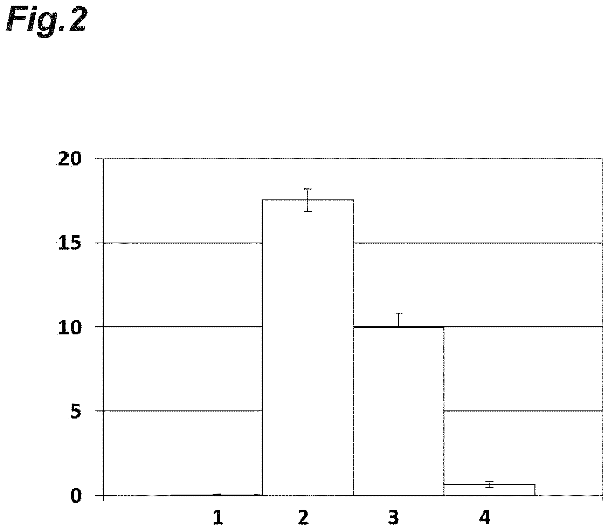

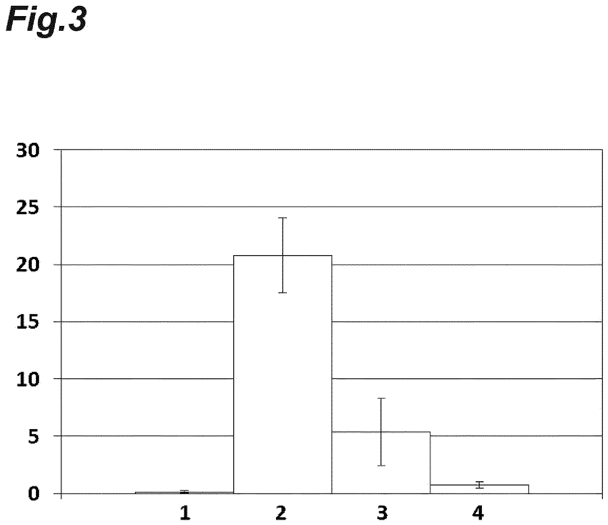

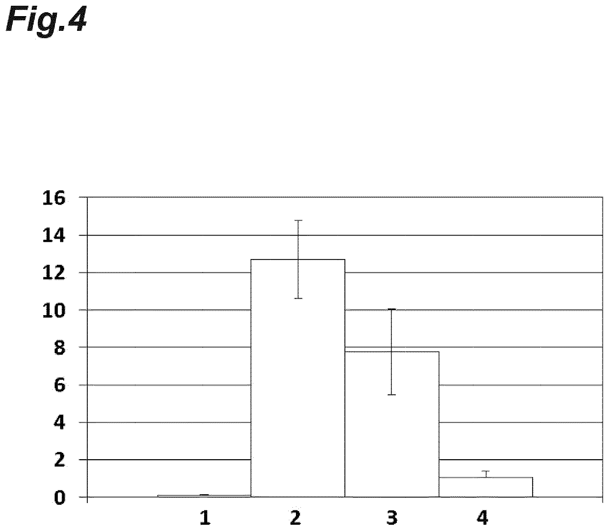

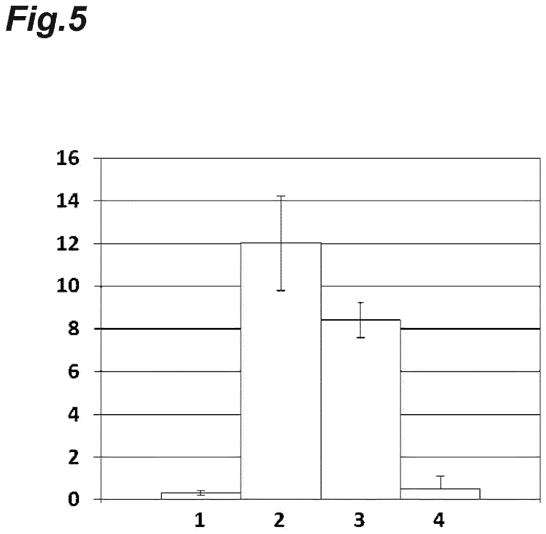

The results of the measurement of the amount of glycogen contained in each muscle tissue are shown in figures 1 to 5. Figure 1, 2, 3, 4, and 5 show the result of the measurement of the heart, the diaphragm, the soleus muscle, anterior tibial muscle, and the quadriceps muscle. When the amount of glycogen contained in each tissue in hGAA-administered group was set as 100%, the amount of glycogen contained in each tissue in hGAA-anti-hTfR antibody-administered group decreased to approximately 7.5% in the heart, 6.5% in the diaphragm, 13.5% in the soleus, 13.5% in the anterior tibial muscle, and 6% in the quadriceps muscle. These results demonstrate that hGAA-anti-hTfR antibody can efficiently degrade the glycogen accumulated in muscular tissue compared to commercially available medical-grade hGAA. Pompe disease (glycogen storage disease type II) is a disease in which glycogen accumulates in large amounts in lysosomes in cells by genetically lacking most or all of the acid α-glucosidase activity, and the main symptom is dysfunction of cardiac and skeletal muscles. That is, hGAA-anti-hTfR antibody is promising as a therapeutic agent in enzymatic replacement therapy of Pompe disease patients because they can efficiently degrade glycogens accumulated in cardiac and skeletal muscles of Pompe disease patients compared to hGAA.

### [Example 16] Method for determining the amount of glycogen contained in tissues

The tissue obtained in Example 15 and 15 pieces of 0.5 mm-diameter SUS beads were placed in a holder set in a bead crusher (BEADS CRUSHER µT-12, Titec Inc.), and water for injection was further added to the holder. The bead crusher was operated to crush the tissue. The obtained tissue crushed material was centrifuged at 15,000 rpm for 10 minutes at 4°C, and the supernatant was collected as a sample solution and stored frozen until the measurement.

Glycogen concentration was measured using Glycogen Colorimetric/Fluorometric Assay Kit (Biovision Inc.) in the following steps as a whole. The calibration curve sample (Glycogen Standard) was diluted stepwise with pure water, and the 50µL of diluted solutions were added to the wells of F96 Black plate (Nunc Inc.). In likewise, 50 µL of the sample solution was added to each well. At this time, each of the calibration curve sample diluted solutions and the sample solutions was added to two wells, respectively. 1 µl of Hydrolysis Enzyme Mix was added to two wells to which a calibration curve sample was added and to one well to which a sample was added each, mixed, and reacted at room temperature for 30 minutes to degrade glycogen into glucose. Then, 50 µl of a Reaction Mix containing a fluorescent reagent was added to all the wells and reacted for 30 minutes at room temperature in a light-proof manner. Fluorescence intensities were measured (Ex/Em = 535/587 nm) using a fluorescent plate reader (SPECTRA max GEMINI XPS, Molecular Devices Inc.) and the glucose concentration of the sample solution to which Hydrolysis Enzyme Mix was added and that of the sample solution to which Hydrolysis Enzyme Mix was not added were calculated from a calibration curve prepared by Linear fit curve from the theoretical concentration (X) and the signal mean (Y) of the calibration curve samples. The glucose concentration measurement of the sample solution to which Hydrolysis Enzyme Mix was not added was then subtracted from the glucose concentration measurement of the sample solution to which Hydrolysis Enzyme Mix was added, and this value was taken as the glycogen concentration of the sample solution. Glycogen concentrations (mg/g wet tissue weights) of the tissues were determined from the weights of the tissues used to prepare the sample solutions and the determined glycogen concentrations.

### Industrial Applicability

According to the present invention, a pharmaceutical agent having to function in muscle, which is not sufficiently taken up into muscle as it is, can be efficiently taken up into muscle by making it as a conjugate coupled to an anti-transferrin receptor antibody. Therefore it can be applied to the development of a new therapeutic drug for muscle disease.

### Sequence Listing Free Text

SEQ ID NO:3: Amino acid sequence of an exemplified linker 1
SEQ ID NO:4: Amino acid sequence of an exemplified linker 2
SEQ ID NO:7: Primer hTfR5', synthetic sequence
SEQ ID NO:8: Primer hTfR3', synthetic sequence
SEQ ID NO:9: Amino acid sequence of the light-chain variable region of mouse anti-hTfR antibody No. 3
SEQ ID NO: 10: Amino acid sequence of the heavy-chain variable region of mouse anti-hTfR antibody No. 3
SEQ ID NO: 11: Amino acid sequence 1 of CDR1 in the light chain of mouse anti-hTfR antibody No. 3
SEQ ID NO: 12: Amino acid sequence 2 of CDR1 in the light chain of mouse anti-hTfR antibody No. 3
SEQ ID NO: 13: Amino acid sequence 1 of CDR2 in the light chain of mouse anti-hTfR antibody No. 3
SEQ ID NO: 14: Amino acid sequence 2 of CDR2 in the light chain of mouse anti-hTfR antibody No. 3
SEQ ID NO:15: Amino acid sequence of CDR3 in the light chain of mouse anti-hTfR antibody No. 3
SEQ ID NO: 16: Amino acid sequence 1 of CDR1 in the heavy chain of mouse anti-hTfR antibody No. 3
SEQ ID NO: 17: Amino acid sequence 2 of CDR1 in the heavy chain of mouse anti-hTfR antibody No. 3
SEQ ID NO: 18: Amino acid sequence 1 of CDR2 in the heavy chain of mouse anti-hTfR antibody No. 3
SEQ ID NO: 19: Amino acid sequence 2 of CDR2 in the heavy chain of mouse anti-hTfR antibody No. 3
SEQ ID NO:20: Amino acid sequence 1 of CDR3 in the heavy chain of mouse anti-hTfR antibody No. 3
SEQ ID NO:21: Amino acid sequence 2 of CDR3 in the heavy chain of mouse anti-hTfR antibody No. 3
SEQ ID NO:22: Amino acid sequence of the light-chain variable region of humanized anti-hTfR antibody No. 3
SEQ ID NO:23: Amino acid sequence of the heavy-chain variable region of humanized anti-hTfR antibody No. 3
SEQ ID NO:24: Amino acid sequence of the light-chain of humanized anti-hTfR antibody No. 3
SEQ ID NO:25: Amino acid sequence of the heavy-chain of humanized anti-hTfR antibody No. 3
SEQ ID NO:26: Nucleic acid sequence encoding the amino acid sequence of the light-chain of humanized anti-hTfR antibody No. 3, synthetic sequence
SEQ ID NO:27: Amino acid sequence of the fusion protein of the heavy chain of humanized anti-hTfR antibody No. 3 and hGAA
SEQ ID NO:28: Nucleic acid sequence encoding the amino acid sequence of the fusion protein of the heavy chain of humanized anti-hTfR antibody No. 3 and hGAA, synthetic sequence
SEQ ID NO:29: Amino acid sequence of the fusion protein of the heavy chain of humanized anti-hTfR antibody No. 3 and h alfa-galA
SEQ ID NO:30: Primer Hyg-Sfi5', synthetic sequence
SEQ ID NO:31: Primer Hyg-BstX3', synthetic sequence
SEQ ID NO:32: Nucelic acid sequence of GAA knockout vector, synthetic sequence

## Claims

1. A conjugate of an anti-human transferrin receptor antibody and an agent, wherein the agent has a physiological activity to be exerted in muscle.

2. The conjugate according to claim 1, wherein the anti-human transferrin receptor antibody is a Fab antibody, a F(ab')₂ antibody, or a F(ab') antibody.

3. The conjugate according to claim 1, wherein the anti-human transferrin receptor antibody is a single-chain antibody selected from the group consisting of scFab, scF(ab'), scF(ab')₂, and scFv.

4. The conjugate according to claim 3, wherein in the single-chain antibody a light chain and a heavy chain of the anti-human transferrin receptor antibody are linked via a linker sequence.

5. The conjugate according to claim 4, wherein the light chain and the heavy chain of the anti-human transferrin receptor antibody are linked on the C-terminal side of the light chain via the linker sequence.

6. The conjugate according to claim 4, wherein the light chain and the heavy chain of the anti-human transferrin receptor antibody are linked on the C-terminal side of the heavy chain via the linker sequence.

7. The conjugate according to any one of claims 4 to 6, wherein the linker sequence comprises 8 to 50 amino acid residues.

8. The conjugate according to claim 7, wherein the linker sequence comprises an amino acid sequence selected from the group consisting of the amino acid sequence (Gly Ser), the amino acid sequence (Gly Gly Ser), the amino acid sequence (Gly Gly Gly), the amino acid sequence set forth as SEQ ID NO: 3, and the amino acid sequence set forth as SEQ ID NO: 4.

9. The conjugate according to claim 8, wherein the linker sequence comprises 15 amino acid residues in which the amino acid sequence set forth as SEQ ID NO: 4 is consecutively repeated three times.

10. The conjugate according to any one of claims 1 to 9, wherein the anti-human transferrin receptor antibody comprises the amino acid sequence set forth as SEQ ID NO: 22 in the variable region of the light chain and the amino acid sequence set forth as SEQ ID NO: 23 in the variable region of the heavy chain.

11. The conjugate according to claim 10, wherein the amino acid sequence of the variable region of the light chain has an identity not lower than 80% to the amino acid sequence set forth as SEQ ID NO: 22, and the amino acid sequence of the variable region of the heavy chain has an identity not lower than 80% to the amino acid sequence set forth as SEQ ID NO: 23.

12. The conjugate according to claim 10, wherein the amino acid sequence of the variable region of the light chain has an identity not lower than 90% to the amino acid sequence set forth as SEQ ID NO: 22, and the amino acid sequence of the variable region of the heavy chain has an identity not lower than 90% to the amino acid sequence set forth as SEQ ID NO: 23.

13. The conjugate according to claim 10, wherein 1 to 10 amino acids constituting the variable region of the light chain are substituted with other amino acids relative to the amino acid sequence set forth as SEQ ID NO: 22.

14. The conjugate according to claim 10, wherein 1 to 3 amino acids constituting the variable region of the light chain are substituted with other amino acids relative to the amino acid sequence set forth as SEQ ID NO: 22.

15. The conjugate according to claim 10, wherein 1 to 10 amino acids constituting the variable region of the heavy chain are substituted with other amino acids relative to the amino acid sequence set forth as SEQ ID NO: 23.

16. The conjugate according to claim 10, wherein 1 to 3 amino acids constituting the variable region of the heavy chain are substituted with other amino acids relative to the amino acid sequence set forth as SEQ ID NO: 23.

17. The conjugate according to claim 10, wherein 1 to 10 amino acids constituting the variable region of the light chain are substituted with other amino acids relative to the amino acid sequence set forth as SEQ ID NO: 22, and 1 to 10 amino acids constituting the variable region of the heavy chain are substituted with other amino acids relative to the amino acid sequence set forth as SEQ ID NO: 23.

18. The conjugate according to claim 10, wherein 1 to 3 amino acids constituting the variable region of the light chain are substituted with other amino acids relative to the amino acid sequence set forth as SEQ ID NO: 22, and 1 to 3 amino acids constituting the variable region of the heavy chain are substituted with other amino acids relative to the amino acid sequence set forth as SEQ ID NO: 23.

19. The conjugate according to claim 10, wherein the anti-human transferrin receptor antibody comprises the light chain comprising an amino acid sequence set forth as SEQ ID NO: 24 and the heavy chain comprising an amino acid sequence set forth as SEQ ID NO: 25.

20. The conjugate according to claim 19, wherein the amino acid sequence of the light chain has an identity not lower than 80% to the amino acid sequence set forth as SEQ ID NO: 24, and the amino acid sequence of the heavy chain has an identity not lower than 80% to the amino acid sequence set forth as SEQ ID NO: 25.

21. The conjugate according to claim 19, wherein the amino acid sequence of the light chain has an identity not lower than 90% to the amino acid sequence set forth as SEQ ID NO: 24, and the amino acid sequence of the heavy chain has an identity not lower than 90% to the amino acid sequence set forth as SEQ ID NO: 25.

22. The conjugate according to any of claims 1 to 21, wherein the agent is a peptide or a protein.

23. The conjugate according to claim 22, selected from the group consisting of (1) to (4) below:
(1) a conjugate in which the protein is linked to the C-terminal side of the light chain of the anti-human transferrin receptor antibody by a peptide bond,
(2) a conjugate in which the protein is linked to the N-terminal side of the light chain of the anti-human transferrin receptor antibody by a peptide bond,
(3) a conjugate in which the protein is linked to the C-terminal side of the heavy chain of the anti-human transferrin receptor antibody by a peptide bond, and
(4) a conjugate in which the protein is linked to the N-terminal side of the heavy chain of the anti-human transferrin receptor antibody by a peptide bond.

24. The conjugate according to claim 23, wherein the protein is linked to the anti-human transferrin receptor antibody via a linker sequence.

25. The conjugate according to claim 24, wherein the linker sequence consists of 1 to 50 amino acid residues.

26. The conjugate according to claim 25, wherein the linker sequence comprises an amino acid sequence selected from the group consisting of a single glycine, a single serine, the amino acid sequence (Gly Ser), the amino acid sequence (Gly Gly Ser), the amino acid sequence set forth as SEQ ID NO:3, the amino acid sequence set forth as SEQ ID NO:4, and the amino acid sequence consisting of 1 to 10 amino acid sequences thereof that are linked consecutively.

27. The conjugate according to any one of claims 1 to 28, wherein the anti-human transferrin receptor antibody is a humanized anti-human transferrin receptor antibody.

28. The conjugate according to any one of claims 22 to 27, wherein the protein is a lysosomal enzyme.

29. The conjugate according to claim 28, wherein the lysosomal enzyme is human α-galactosidase A.

30. The conjugate according to claim 29, wherein the light chain of the humanized anti-hTfR antibody comprises the amino acid sequence set forth as SEQ ID NO: 24, and wherein the heavy chain of the humanized anti-hTfR antibody is linked on its C-terminal side to the human α-galactosidase A via a linker sequence (Gly Ser), and the whole linked heavy chain has the amino acid sequence set forth as SEQ ID NO: 29.

31. The conjugate according to claim 28, wherein the lysosomal enzyme is human acid α-glucosidase.

32. The conjugate according to claim 31, wherein the light chain of the humanized anti-hTfR antibody comprises the amino acid sequence set forth as SEQ ID NO: 24, and wherein the heavy chain of the humanized anti-hTfR antibody is linked on its C-terminal side to the human acid α-glucosidase via a linker sequence (Gly Ser), and the whole linked heavy chain has the amino acid sequence set forth as SEQ ID NO: 27.

33. A pharmaceutical composition for improving a muscle function, comprising the conjugate according to any one of claims 1 to 32.

34. A pharmaceutical composition for ameliorating muscle dysfunction associated with lysosomal disease, comprising the conjugate according to claim 28.

35. A pharmaceutical composition for ameliorating muscle dysfunction associated with Fabry disease, comprising the conjugate according to claim 29 or 30.

36. A pharmaceutical composition for ameliorating muscle dysfunction associated with Pompe disease, comprising the conjugate according to claim 31 or 32.

37. The pharmaceutical composition according to any one of claims 33 to 36, wherein the muscle is skeletal muscle, cardiac muscle, or smooth muscle.

38. Use of the conjugate according to any one of claims 28 to 32 for the manufacture of a pharmaceutical composition for ameliorating muscle dysfunction associated with lysosomal disease.

39. Use of the conjugate according to claim 29 or 30 for the manufacture of a pharmaceutical composition for ameliorating muscle dysfunction associated with Fabry disease.

40. Use of the conjugate according to claim 31 or 32 for the manufacture of a pharmaceutical composition for ameliorating muscle dysfunction associated with Pompe disease.

41. Use of the conjugate according to any one of claims 1 to 32 as a pharmaceutical composition for improving muscle function.

42. A method for delivering an agent to muscle, comprising a step of producing the agent as a conjugate in which the agent is linked to an anti-human transferrin receptor antibody and a step of administering the conjugate to an individual.

43. The method according to claim 41, wherein the conjugate is the conjugate according to any one of claims 1 to 32.

44. The method according to claim 42 or 43, further comprising a step of improving muscle function of the individual by administration of the conjugate.

45. The method according to any one of claims 42 to 44, wherein the individual has a muscle dysfunction.

46. The method according to any one of claims 42 to 44, wherein the individual has a muscle dysfunction associated with lysosomal disease.

47. The method of claim 46, wherein the lysosomal disease is Fabry disease or Pompe disease.


SEQUENCE LISTING

<110>  JCR Pharmaceuticals Co., Ltd.

<120>  Method for sorting a protein to muscle tissue

<130>  1210P

<150>  JP2018-018652
<151>  2018-02-05

<160>  32

<170>  PatentIn version 3.5

<210>  1
<211>  883
<212>  PRT
<213>  Homo sapiens

<400>  1

Ala His Pro Gly Arg Pro Arg Ala Val Pro Thr Gln Cys Asp Val Pro
1               5                   10                  15

Pro Asn Ser Arg Phe Asp Cys Ala Pro Asp Lys Ala Ile Thr Gln Glu
            20                  25                  30

Gln Cys Glu Ala Arg Gly Cys Cys Tyr Ile Pro Ala Lys Gln Gly Leu
        35                  40                  45

Gln Gly Ala Gln Met Gly Gln Pro Trp Cys Phe Phe Pro Pro Ser Tyr
    50                  55                  60

Pro Ser Tyr Lys Leu Glu Asn Leu Ser Ser Ser Glu Met Gly Tyr Thr
65                  70                  75                  80

Ala Thr Leu Thr Arg Thr Thr Pro Thr Phe Phe Pro Lys Asp Ile Leu
                85                  90                  95

Thr Leu Arg Leu Asp Val Met Met Glu Thr Glu Asn Arg Leu His Phe
            100                 105                 110

Thr Ile Lys Asp Pro Ala Asn Arg Arg Tyr Glu Val Pro Leu Glu Thr
        115                 120                 125

Pro Arg Val His Ser Arg Ala Pro Ser Pro Leu Tyr Ser Val Glu Phe
    130                 135                 140

Ser Glu Glu Pro Phe Gly Val Ile Val His Arg Gln Leu Asp Gly Arg
145                 150                 155                 160

Val Leu Leu Asn Thr Thr Val Ala Pro Leu Phe Phe Ala Asp Gln Phe

                165                 170                 175

Leu Gln Leu Ser Thr Ser Leu Pro Ser Gln Tyr Ile Thr Gly Leu Ala
            180                 185                 190

Glu His Leu Ser Pro Leu Met Leu Ser Thr Ser Trp Thr Arg Ile Thr
        195                 200                 205

Leu Trp Asn Arg Asp Leu Ala Pro Thr Pro Gly Ala Asn Leu Tyr Gly
    210                 215                 220

Ser His Pro Phe Tyr Leu Ala Leu Glu Asp Gly Gly Ser Ala His Gly
225                 230                 235                 240

Val Phe Leu Leu Asn Ser Asn Ala Met Asp Val Val Leu Gln Pro Ser
                245                 250                 255

Pro Ala Leu Ser Trp Arg Ser Thr Gly Gly Ile Leu Asp Val Tyr Ile
            260                 265                 270

Phe Leu Gly Pro Glu Pro Lys Ser Val Val Gln Gln Tyr Leu Asp Val
        275                 280                 285

Val Gly Tyr Pro Phe Met Pro Pro Tyr Trp Gly Leu Gly Phe His Leu
    290                 295                 300

Cys Arg Trp Gly Tyr Ser Ser Thr Ala Ile Thr Arg Gln Val Val Glu
305                 310                 315                 320

Asn Met Thr Arg Ala His Phe Pro Leu Asp Val Gln Trp Asn Asp Leu
                325                 330                 335

Asp Tyr Met Asp Ser Arg Arg Asp Phe Thr Phe Asn Lys Asp Gly Phe
            340                 345                 350

Arg Asp Phe Pro Ala Met Val Gln Glu Leu His Gln Gly Gly Arg Arg
        355                 360                 365

Tyr Met Met Ile Val Asp Pro Ala Ile Ser Ser Ser Gly Pro Ala Gly
    370                 375                 380

Ser Tyr Arg Pro Tyr Asp Glu Gly Leu Arg Arg Gly Val Phe Ile Thr
385                 390                 395                 400

Asn Glu Thr Gly Gln Pro Leu Ile Gly Lys Val Trp Pro Gly Ser Thr
                405                 410                 415

Ala Phe Pro Asp Phe Thr Asn Pro Thr Ala Leu Ala Trp Trp Glu Asp
            420                 425                 430

Met Val Ala Glu Phe His Asp Gln Val Pro Phe Asp Gly Met Trp Ile
        435                 440                 445

Asp Met Asn Glu Pro Ser Asn Phe Ile Arg Gly Ser Glu Asp Gly Cys
    450                 455                 460

Pro Asn Asn Glu Leu Glu Asn Pro Pro Tyr Val Pro Gly Val Val Gly
465                 470                 475                 480

Gly Thr Leu Gln Ala Ala Thr Ile Cys Ala Ser Ser His Gln Phe Leu
                485                 490                 495

Ser Thr His Tyr Asn Leu His Asn Leu Tyr Gly Leu Thr Glu Ala Ile
            500                 505                 510

Ala Ser His Arg Ala Leu Val Lys Ala Arg Gly Thr Arg Pro Phe Val
        515                 520                 525

Ile Ser Arg Ser Thr Phe Ala Gly His Gly Arg Tyr Ala Gly His Trp
    530                 535                 540

Thr Gly Asp Val Trp Ser Ser Trp Glu Gln Leu Ala Ser Ser Val Pro
545                 550                 555                 560

Glu Ile Leu Gln Phe Asn Leu Leu Gly Val Pro Leu Val Gly Ala Asp
                565                 570                 575

Val Cys Gly Phe Leu Gly Asn Thr Ser Glu Glu Leu Cys Val Arg Trp
            580                 585                 590

Thr Gln Leu Gly Ala Phe Tyr Pro Phe Met Arg Asn His Asn Ser Leu
        595                 600                 605

Leu Ser Leu Pro Gln Glu Pro Tyr Ser Phe Ser Glu Pro Ala Gln Gln
    610                 615                 620

Ala Met Arg Lys Ala Leu Thr Leu Arg Tyr Ala Leu Leu Pro His Leu
625                 630                 635                 640

Tyr Thr Leu Phe His Gln Ala His Val Ala Gly Glu Thr Val Ala Arg
                645                 650                 655

Pro Leu Phe Leu Glu Phe Pro Lys Asp Ser Ser Thr Trp Thr Val Asp
            660                 665                 670

His Gln Leu Leu Trp Gly Glu Ala Leu Leu Ile Thr Pro Val Leu Gln
        675                 680                 685

Ala Gly Lys Ala Glu Val Thr Gly Tyr Phe Pro Leu Gly Thr Trp Tyr
    690                 695                 700

Asp Leu Gln Thr Val Pro Ile Glu Ala Leu Gly Ser Leu Pro Pro Pro
705                 710                 715                 720

Pro Ala Ala Pro Arg Glu Pro Ala Ile His Ser Glu Gly Gln Trp Val
                725                 730                 735

Thr Leu Pro Ala Pro Leu Asp Thr Ile Asn Val His Leu Arg Ala Gly
            740                 745                 750

Tyr Ile Ile Pro Leu Gln Gly Pro Gly Leu Thr Thr Thr Glu Ser Arg
        755                 760                 765

Gln Gln Pro Met Ala Leu Ala Val Ala Leu Thr Lys Gly Gly Glu Ala
    770                 775                 780

Arg Gly Glu Leu Phe Trp Asp Asp Gly Glu Ser Leu Glu Val Leu Glu
785                 790                 795                 800

Arg Gly Ala Tyr Thr Gln Val Ile Phe Leu Ala Arg Asn Asn Thr Ile
                805                 810                 815

Val Asn Glu Leu Val Arg Val Thr Ser Glu Gly Ala Gly Leu Gln Leu
            820                 825                 830

Gln Lys Val Thr Val Leu Gly Val Ala Thr Ala Pro Gln Gln Val Leu
        835                 840                 845

Ser Asn Gly Val Pro Val Ser Asn Phe Thr Tyr Ser Pro Asp Thr Lys
    850                 855                 860

Val Leu Asp Ile Cys Val Ser Leu Leu Met Gly Glu Gln Phe Leu Val
865                 870                 875                 880

Ser Trp Cys

<210> 2
<211> 398
<212> PRT
<213> Homo sapiens

<400> 2

Leu Asp Asn Gly Leu Ala Arg Thr Pro Thr Met Gly Trp Leu His Trp
1               5                   10                  15

Glu Arg Phe Met Cys Asn Leu Asp Cys Gln Glu Glu Pro Asp Ser Cys
            20                  25                  30

Ile Ser Glu Lys Leu Phe Met Glu Met Ala Glu Leu Met Val Ser Glu
        35                  40                  45

Gly Trp Lys Asp Ala Gly Tyr Glu Tyr Leu Cys Ile Asp Asp Cys Trp
    50                  55                  60

Met Ala Pro Gln Arg Asp Ser Glu Gly Arg Leu Gln Ala Asp Pro Gln
65                  70                  75                  80

Arg Phe Pro His Gly Ile Arg Gln Leu Ala Asn Tyr Val His Ser Lys
                85                  90                  95

Gly Leu Lys Leu Gly Ile Tyr Ala Asp Val Gly Asn Lys Thr Cys Ala
            100                 105                 110

Gly Phe Pro Gly Ser Phe Gly Tyr Tyr Asp Ile Asp Ala Gln Thr Phe
        115                 120                 125

Ala Asp Trp Gly Val Asp Leu Leu Lys Phe Asp Gly Cys Tyr Cys Asp
    130                 135                 140

Ser Leu Glu Asn Leu Ala Asp Gly Tyr Lys His Met Ser Leu Ala Leu
145                 150                 155                 160

Asn Arg Thr Gly Arg Ser Ile Val Tyr Ser Cys Glu Trp Pro Leu Tyr
                165                 170                 175

Met Trp Pro Phe Gln Lys Pro Asn Tyr Thr Glu Ile Arg Gln Tyr Cys
            180                 185                 190

Asn His Trp Arg Asn Phe Ala Asp Ile Asp Asp Ser Trp Lys Ser Ile
        195                 200                 205

Lys Ser Ile Leu Asp Trp Thr Ser Phe Asn Gln Glu Arg Ile Val Asp
    210                 215                 220

Val Ala Gly Pro Gly Gly Trp Asn Asp Pro Asp Met Leu Val Ile Gly
225                 230                 235                 240

Asn Phe Gly Leu Ser Trp Asn Gln Gln Val Thr Gln Met Ala Leu Trp
                245                 250                 255

Ala Ile Met Ala Ala Pro Leu Phe Met Ser Asn Asp Leu Arg His Ile
                260                 265                 270

Ser Pro Gln Ala Lys Ala Leu Leu Gln Asp Lys Asp Val Ile Ala Ile
            275                 280                 285

Asn Gln Asp Pro Leu Gly Lys Gln Gly Tyr Gln Leu Arg Gln Gly Asp
    290                 295                 300

Asn Phe Glu Val Trp Glu Arg Pro Leu Ser Gly Leu Ala Trp Ala Val
305                 310                 315                 320

Ala Met Ile Asn Arg Gln Glu Ile Gly Gly Pro Arg Ser Tyr Thr Ile
                325                 330                 335

Ala Val Ala Ser Leu Gly Lys Gly Val Ala Cys Asn Pro Ala Cys Phe
                340                 345                 350

Ile Thr Gln Leu Leu Pro Val Lys Arg Lys Leu Gly Phe Tyr Glu Trp
            355                 360                 365

Thr Ser Arg Leu Arg Ser His Ile Asn Pro Thr Gly Thr Val Leu Leu
    370                 375                 380

Gln Leu Glu Asn Thr Met Gln Met Ser Leu Lys Asp Leu Leu
385                 390                 395

<210>  3
<211>  5
<212>  PRT
<213>  Artificial Sequence

<220>
<223>  Amino acid sequence of an exemplified linker 1

<400>  3

Gly Gly Gly Gly Ser
1               5

<210>  4
<211>  6
<212>  PRT
<213>  Artificial Sequence

<220>
<223>  Amino acid sequence of an exemplified linker 2

<400>  4

Gly Gly Gly Gly Gly Ser

1               5


<210>  5
<211>  760
<212>  PRT
<213>  Homo sapiens

<400>  5

Met Met Asp Gln Ala Arg Ser Ala Phe Ser Asn Leu Phe Gly Gly Glu
1               5                   10                  15


Pro Leu Ser Tyr Thr Arg Phe Ser Leu Ala Arg Gln Val Asp Gly Asp
            20                  25                  30


Asn Ser His Val Glu Met Lys Leu Ala Val Asp Glu Glu Glu Asn Ala
        35                  40                  45


Asp Asn Asn Thr Lys Ala Asn Val Thr Lys Pro Lys Arg Cys Ser Gly
    50                  55                  60


Ser Ile Cys Tyr Gly Thr Ile Ala Val Ile Val Phe Phe Leu Ile Gly
65                  70                  75                  80


Phe Met Ile Gly Tyr Leu Gly Tyr Cys Lys Gly Val Glu Pro Lys Thr
                85                  90                  95


Glu Cys Glu Arg Leu Ala Gly Thr Glu Ser Pro Val Arg Glu Glu Pro
            100                 105                 110


Gly Glu Asp Phe Pro Ala Ala Arg Arg Leu Tyr Trp Asp Asp Leu Lys
        115                 120                 125


Arg Lys Leu Ser Glu Lys Leu Asp Ser Thr Asp Phe Thr Gly Thr Ile
    130                 135                 140


Lys Leu Leu Asn Glu Asn Ser Tyr Val Pro Arg Glu Ala Gly Ser Gln
145                 150                 155                 160


Lys Asp Glu Asn Leu Ala Leu Tyr Val Glu Asn Gln Phe Arg Glu Phe
                165                 170                 175


Lys Leu Ser Lys Val Trp Arg Asp Gln His Phe Val Lys Ile Gln Val
            180                 185                 190


Lys Asp Ser Ala Gln Asn Ser Val Ile Ile Val Asp Lys Asn Gly Arg
        195                 200                 205


Leu Val Tyr Leu Val Glu Asn Pro Gly Gly Tyr Val Ala Tyr Ser Lys

210                 215                 220

Ala Ala Thr Val Thr Gly Lys Leu Val His Ala Asn Phe Gly Thr Lys
225                 230                 235                 240

Lys Asp Phe Glu Asp Leu Tyr Thr Pro Val Asn Gly Ser Ile Val Ile
                245                 250                 255

Val Arg Ala Gly Lys Ile Thr Phe Ala Glu Lys Val Ala Asn Ala Glu
            260                 265                 270

Ser Leu Asn Ala Ile Gly Val Leu Ile Tyr Met Asp Gln Thr Lys Phe
        275                 280                 285

Pro Ile Val Asn Ala Glu Leu Ser Phe Phe Gly His Ala His Leu Gly
    290                 295                 300

Thr Gly Asp Pro Tyr Thr Pro Gly Phe Pro Ser Phe Asn His Thr Gln
305                 310                 315                 320

Phe Pro Pro Ser Arg Ser Ser Gly Leu Pro Asn Ile Pro Val Gln Thr
                325                 330                 335

Ile Ser Arg Ala Ala Ala Glu Lys Leu Phe Gly Asn Met Glu Gly Asp
            340                 345                 350

Cys Pro Ser Asp Trp Lys Thr Asp Ser Thr Cys Arg Met Val Thr Ser
        355                 360                 365

Glu Ser Lys Asn Val Lys Leu Thr Val Ser Asn Val Leu Lys Glu Ile
    370                 375                 380

Lys Ile Leu Asn Ile Phe Gly Val Ile Lys Gly Phe Val Glu Pro Asp
385                 390                 395                 400

His Tyr Val Val Val Gly Ala Gln Arg Asp Ala Trp Gly Pro Gly Ala
                405                 410                 415

Ala Lys Ser Gly Val Gly Thr Ala Leu Leu Leu Lys Leu Ala Gln Met
            420                 425                 430

Phe Ser Asp Met Val Leu Lys Asp Gly Phe Gln Pro Ser Arg Ser Ile
        435                 440                 445

Ile Phe Ala Ser Trp Ser Ala Gly Asp Phe Gly Ser Val Gly Ala Thr
    450                 455                 460

Glu Trp Leu Glu Gly Tyr Leu Ser Ser Leu His Leu Lys Ala Phe Thr
465                 470                 475                 480

Tyr Ile Asn Leu Asp Lys Ala Val Leu Gly Thr Ser Asn Phe Lys Val
                485                 490                 495

Ser Ala Ser Pro Leu Leu Tyr Thr Leu Ile Glu Lys Thr Met Gln Asn
            500                 505                 510

Val Lys His Pro Val Thr Gly Gln Phe Leu Tyr Gln Asp Ser Asn Trp
        515                 520                 525

Ala Ser Lys Val Glu Lys Leu Thr Leu Asp Asn Ala Ala Phe Pro Phe
    530                 535                 540

Leu Ala Tyr Ser Gly Ile Pro Ala Val Ser Phe Cys Phe Cys Glu Asp
545                 550                 555                 560

Thr Asp Tyr Pro Tyr Leu Gly Thr Thr Met Asp Thr Tyr Lys Glu Leu
                565                 570                 575

Ile Glu Arg Ile Pro Glu Leu Asn Lys Val Ala Arg Ala Ala Ala Glu
            580                 585                 590

Val Ala Gly Gln Phe Val Ile Lys Leu Thr His Asp Val Glu Leu Asn
        595                 600                 605

Leu Asp Tyr Glu Arg Tyr Asn Ser Gln Leu Leu Ser Phe Val Arg Asp
    610                 615                 620

Leu Asn Gln Tyr Arg Ala Asp Ile Lys Glu Met Gly Leu Ser Leu Gln
625                 630                 635                 640

Trp Leu Tyr Ser Ala Arg Gly Asp Phe Phe Arg Ala Thr Ser Arg Leu
                645                 650                 655

Thr Thr Asp Phe Gly Asn Ala Glu Lys Thr Asp Arg Phe Val Met Lys
            660                 665                 670

Lys Leu Asn Asp Arg Val Met Arg Val Glu Tyr His Phe Leu Ser Pro
        675                 680                 685

Tyr Val Ser Pro Lys Glu Ser Pro Phe Arg His Val Phe Trp Gly Ser
    690                 695                 700

Gly Ser His Thr Leu Pro Ala Leu Leu Glu Asn Leu Lys Leu Arg Lys
705                 710                 715                 720

Gln Asn Asn Gly Ala Phe Asn Glu Thr Leu Phe Arg Asn Gln Leu Ala
                725                 730                 735

Leu Ala Thr Trp Thr Ile Gln Gly Ala Ala Asn Ala Leu Ser Gly Asp
            740                 745                 750

Val Trp Asp Ile Asp Asn Glu Phe
        755                 760

<210>  6
<211>  760
<212>  PRT
<213>  Macaca fascicularis

<400>  6

Met Met Asp Gln Ala Arg Ser Ala Phe Ser Asn Leu Phe Gly Gly Glu
1               5                   10                  15

Pro Leu Ser Tyr Thr Arg Phe Ser Leu Ala Arg Gln Val Asp Gly Asp
            20                  25                  30

Asn Ser His Val Glu Met Lys Leu Ala Val Asp Asp Glu Glu Asn Ala
        35                  40                  45

Asp Asn Asn Thr Lys Ala Asn Gly Thr Lys Pro Lys Arg Cys Gly Gly
    50                  55                  60

Asn Ile Cys Tyr Gly Thr Ile Ala Val Ile Ile Phe Phe Leu Ile Gly
65                  70                  75                  80

Phe Met Ile Gly Tyr Leu Gly Tyr Cys Lys Gly Val Glu Pro Lys Thr
                85                  90                  95

Glu Cys Glu Arg Leu Ala Gly Thr Glu Ser Pro Ala Arg Glu Glu Pro
            100                 105                 110

Glu Glu Asp Phe Pro Ala Ala Pro Arg Leu Tyr Trp Asp Asp Leu Lys
        115                 120                 125

Arg Lys Leu Ser Glu Lys Leu Asp Thr Thr Asp Phe Thr Ser Thr Ile
    130                 135                 140

Lys Leu Leu Asn Glu Asn Leu Tyr Val Pro Arg Glu Ala Gly Ser Gln
145                 150                 155                 160

Lys Asp Glu Asn Leu Ala Leu Tyr Ile Glu Asn Gln Phe Arg Glu Phe
                165                 170                 175

Lys Leu Ser Lys Val Trp Arg Asp Gln His Phe Val Lys Ile Gln Val
            180                 185                 190

Lys Asp Ser Ala Gln Asn Ser Val Ile Ile Val Asp Lys Asn Gly Gly
        195                 200                 205

Leu Val Tyr Leu Val Glu Asn Pro Gly Gly Tyr Val Ala Tyr Ser Lys
    210                 215                 220

Ala Ala Thr Val Thr Gly Lys Leu Val His Ala Asn Phe Gly Thr Lys
225                 230                 235                 240

Lys Asp Phe Glu Asp Leu Asp Ser Pro Val Asn Gly Ser Ile Val Ile
                245                 250                 255

Val Arg Ala Gly Lys Ile Thr Phe Ala Glu Lys Val Ala Asn Ala Glu
            260                 265                 270

Ser Leu Asn Ala Ile Gly Val Leu Ile Tyr Met Asp Gln Thr Lys Phe
        275                 280                 285

Pro Ile Val Lys Ala Asp Leu Ser Phe Phe Gly His Ala His Leu Gly
    290                 295                 300

Thr Gly Asp Pro Tyr Thr Pro Gly Phe Pro Ser Phe Asn His Thr Gln
305                 310                 315                 320

Phe Pro Pro Ser Gln Ser Ser Gly Leu Pro Asn Ile Pro Val Gln Thr
                325                 330                 335

Ile Ser Arg Ala Ala Ala Glu Lys Leu Phe Gly Asn Met Glu Gly Asp
            340                 345                 350

Cys Pro Ser Asp Trp Lys Thr Asp Ser Thr Cys Lys Met Val Thr Ser
        355                 360                 365

Glu Asn Lys Ser Val Lys Leu Thr Val Ser Asn Val Leu Lys Glu Thr
    370                 375                 380

Lys Ile Leu Asn Ile Phe Gly Val Ile Lys Gly Phe Val Glu Pro Asp
385                 390                 395                 400

His Tyr Val Val Val Gly Ala Gln Arg Asp Ala Trp Gly Pro Gly Ala
                405                 410                 415

Ala Lys Ser Ser Val Gly Thr Ala Leu Leu Leu Lys Leu Ala Gln Met
            420                 425                 430

Phe Ser Asp Met Val Leu Lys Asp Gly Phe Gln Pro Ser Arg Ser Ile
        435                 440                 445

Ile Phe Ala Ser Trp Ser Ala Gly Asp Phe Gly Ser Val Gly Ala Thr
    450                 455                 460

Glu Trp Leu Glu Gly Tyr Leu Ser Ser Leu His Leu Lys Ala Phe Thr
465                 470                 475                 480

Tyr Ile Asn Leu Asp Lys Ala Val Leu Gly Thr Ser Asn Phe Lys Val
                485                 490                 495

Ser Ala Ser Pro Leu Leu Tyr Thr Leu Ile Glu Lys Thr Met Gln Asp
            500                 505                 510

Val Lys His Pro Val Thr Gly Arg Ser Leu Tyr Gln Asp Ser Asn Trp
        515                 520                 525

Ala Ser Lys Val Glu Lys Leu Thr Leu Asp Asn Ala Ala Phe Pro Phe
    530                 535                 540

Leu Ala Tyr Ser Gly Ile Pro Ala Val Ser Phe Cys Phe Cys Glu Asp
545                 550                 555                 560

Thr Asp Tyr Pro Tyr Leu Gly Thr Thr Met Asp Thr Tyr Lys Glu Leu
                565                 570                 575

Val Glu Arg Ile Pro Glu Leu Asn Lys Val Ala Arg Ala Ala Ala Glu
            580                 585                 590

Val Ala Gly Gln Phe Val Ile Lys Leu Thr His Asp Thr Glu Leu Asn
        595                 600                 605

Leu Asp Tyr Glu Arg Tyr Asn Ser Gln Leu Leu Leu Phe Leu Arg Asp
    610                 615                 620

Leu Asn Gln Tyr Arg Ala Asp Val Lys Glu Met Gly Leu Ser Leu Gln
625                 630                 635                 640

Trp Leu Tyr Ser Ala Arg Gly Asp Phe Phe Arg Ala Thr Ser Arg Leu
                645                 650                 655

Thr Thr Asp Phe Arg Asn Ala Glu Lys Arg Asp Lys Phe Val Met Lys
            660                 665                 670

Lys Leu Asn Asp Arg Val Met Arg Val Glu Tyr Tyr Phe Leu Ser Pro

675                 680                 685

Tyr Val Ser Pro Lys Glu Ser Pro Phe Arg His Val Phe Trp Gly Ser
    690                 695                 700

Gly Ser His Thr Leu Ser Ala Leu Leu Glu Ser Leu Lys Leu Arg Arg
705                 710                 715                 720

Gln Asn Asn Ser Ala Phe Asn Glu Thr Leu Phe Arg Asn Gln Leu Ala
                725                 730                 735

Leu Ala Thr Trp Thr Ile Gln Gly Ala Ala Asn Ala Leu Ser Gly Asp
            740                 745                 750

Val Trp Asp Ile Asp Asn Glu Phe
        755                 760

<210>  7
<211>  43
<212>  DNA
<213>  Artificial Sequence

<220>
<223>  Primer hTfR5', synthetic sequence

<400>  7
ccgacgcgtc gccaccatga tggatcaagc tagatcagca ttc                    43

<210>  8
<211>  59
<212>  DNA
<213>  Artificial Sequence

<220>
<223>  Primer hTfR3', synthetic sequence

<400>  8
ataatgcggc cgcttaatga tgatgatgat gatgaaactc attgtcaatg tcccaaacg      59

<210>  9
<211>  113
<212>  PRT
<213>  Artificial Sequence

<220>
<223>  Amino acid sequence of the light-chain variable region of mouse
       anti-hTfR antibody No. 3

<400>  9

Asp Ile Val Met Thr Gln Thr Thr Leu Ser Leu Pro Val Ser Leu Gly
1               5                   10                  15

Asp Gln Ala Ser Ile Ser Cys Arg Ser Ser Gln Ser Leu Val His Ser

20                  25                  30

Asn Gly Asn Thr Tyr Leu His Trp Tyr Leu Gln Lys Pro Gly Gln Ser
        35                  40                  45

Pro Lys Leu Leu Ile Tyr Lys Val Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Leu Gly Val Phe Phe Cys Ser Gln Ser
                85                  90                  95

Thr His Val Pro Trp Thr Phe Gly Gly Gly Thr Lys Leu Glu Ile Lys
            100                 105                 110

Arg

<210>  10
<211>  118
<212>  PRT
<213>  Artificial Sequence

<220>
<223>  Amino acid sequence of the heavy-chain variable region of mouse
       anti-hTfR antibody No. 3

<400>  10

Glu Val Gln Leu Gln Glu Ser Gly Ala Glu Leu Val Arg Pro Gly Thr
1               5                   10                  15

Ser Val Lys Ile Ser Cys Lys Ala Ser Gly Tyr Thr Phe Met Asn Tyr
            20                  25                  30

Trp Leu Gly Trp Val Lys Gln Arg Pro Gly His Gly Leu Glu Trp Ile
        35                  40                  45

Gly Asp Ile Tyr Pro Gly Gly Asp Tyr Pro Thr Tyr Ser Glu Lys Phe
    50                  55                  60

Lys Val Lys Ala Ile Leu Thr Ala Asp Thr Ser Ser Ser Ser Val Tyr
65                  70                  75                  80

Leu His Leu Ser Ser Leu Thr Ser Glu Asp Ser Ala Val Tyr Phe Cys
                85                  90                  95

Ala Arg Ser Gly Asn Tyr Asp Glu Val Ala Tyr Trp Gly Gln Gly Thr

            100                 105                 110

Leu Val Thr Val Ser Thr
        115

<210> 11
<211> 11
<212> PRT
<213> Artificial Sequence

<220>
<223> Amino acid sequence 1 of CDR1 in the light chain of mouse
anti-hTfR antibody No. 3

<400> 11

Gln Ser Leu Val His Ser Asn Gly Asn Thr Tyr
1               5                   10

<210> 12
<211> 16
<212> PRT
<213> Artificial Sequence

<220>
<223> Amino acid sequence 2 of CDR1 in the light chain of mouse
anti-hTfR antibody No. 3

<400> 12

Arg Ser Ser Gln Ser Leu Val His Ser Asn Gly Asn Thr Tyr Leu His
1               5                   10                  15

<210> 13
<211> 6
<212> PRT
<213> Artificial Sequence

<220>
<223> Amino acid sequence 1 of CDR2 in the light chain of mouse
anti-hTfR antibody No. 3

<400> 13

Lys Val Ser Asn Arg Phe
1               5

<210> 14
<211> 7
<212> PRT
<213> Artificial Sequence

<220>
<223> Amino acid sequence 2 of CDR2 in the light chain of mouse
anti-hTfR antibody No. 3

<400> 14

Lys Val Ser Asn Arg Phe Ser
1               5


<210>  15
<211>  9
<212>  PRT
<213>  Artificial Sequence

<220>
<223>  Amino acid sequence of CDR3 in the light chain of mouse anti-hTfR
       antibody No. 3

<400>  15

Ser Gln Ser Thr His Val Pro Trp Thr
1               5


<210>  16
<211>  5
<212>  PRT
<213>  Artificial Sequence

<220>
<223>  Amino acid sequence 1 of CDR1 in the heavy chain of mouse
       anti-hTfR antibody No. 3

<400>  16

Asn Tyr Trp Leu Gly
1               5


<210>  17
<211>  8
<212>  PRT
<213>  Artificial Sequence

<220>
<223>  Amino acid sequence 2 of CDR1 in the heavy chain of mouse
       anti-hTfR antibody No. 3

<400>  17

Gly Tyr Ser Phe Thr Asn Tyr Trp
1               5


<210>  18
<211>  8
<212>  PRT
<213>  Artificial Sequence

<220>
<223>  Amino acid sequence 1 of CDR2 in the heavy chain of mouse
       anti-hTfR antibody No. 3

<400>  18

Ile Tyr Pro Gly Gly Asp Tyr Pro
1               5

<210>  19
<211>  17
<212>  PRT
<213>  Artificial Sequence

<220>
<223>  Amino acid sequence 2 of CDR2 in the heavy chain of mouse
       anti-hTfR antibody No. 3

<400>  19

Asp Ile Tyr Pro Gly Gly Asp Tyr Pro Thr Tyr Ser Glu Lys Phe Lys
1               5                   10                  15

Val

<210>  20
<211>  9
<212>  PRT
<213>  Artificial Sequence

<220>
<223>  Amino acid sequence 1 of CDR3 in the heavy chain of mouse
       anti-hTfR antibody No. 3

<400>  20

Ser Gly Asn Tyr Asp Glu Val Ala Tyr
1               5

<210>  21
<211>  11
<212>  PRT
<213>  Artificial Sequence

<220>
<223>  Amino acid sequence 2 of CDR3 in the heavy chain of mouse
       anti-hTfR antibody No. 3

<400>  21

Ala Arg Ser Gly Asn Tyr Asp Glu Val Ala Tyr
1               5                   10

<210>  22
<211>  112
<212>  PRT
<213>  Artificial Sequence

<220>
<223>  Amino acid sequence of the light-chain variable region of
       humanized anti-hTfR antibody No. 3

<400>  22

Asp Ile Val Met Thr Gln Thr Pro Leu Ser Leu Ser Val Thr Pro Gly
1               5                   10                  15

Gln Pro Ala Ser Ile Ser Cys Arg Ser Ser Gln Ser Leu Val His Ser
            20                  25                  30

Asn Gly Asn Thr Tyr Leu His Trp Tyr Leu Gln Lys Pro Gly Gln Ser
        35                  40                  45

Pro Gln Leu Leu Ile Tyr Lys Val Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Ser Gln Ser
                85                  90                  95

Thr His Val Pro Trp Thr Phe Gly Gln Gly Thr Lys Val Glu Ile Lys
            100                 105                 110

<210> 23
<211> 118
<212> PRT
<213> Artificial Sequence

<220>
<223> Amino acid sequence of the heavy-chain variable region of
humanized anti-hTfR antibody No. 3

<400> 23

Glu Val Gln Leu Val Gln Ser Gly Ala Glu Val Lys Lys Pro Gly Glu
1               5                   10                  15

Ser Leu Lys Ile Ser Cys Lys Gly Ser Gly Tyr Ser Phe Thr Asn Tyr
            20                  25                  30

Trp Leu Gly Trp Val Arg Gln Met Pro Gly Lys Gly Leu Glu Trp Met
        35                  40                  45

Gly Asp Ile Tyr Pro Gly Gly Asp Tyr Pro Thr Tyr Ser Glu Lys Phe
    50                  55                  60

Lys Val Gln Val Thr Ile Ser Ala Asp Lys Ser Ile Ser Thr Ala Tyr
65                  70                  75                  80

Leu Gln Trp Ser Ser Leu Lys Ala Ser Asp Thr Ala Met Tyr Tyr Cys
                85                  90                  95

Ala Arg Ser Gly Asn Tyr Asp Glu Val Ala Tyr Trp Gly Gln Gly Thr
            100                 105                 110

Leu Val Thr Val Ser Ser
        115

<210>  24
<211>  219
<212>  PRT
<213>  Artificial Sequence

<220>
<223>  Amino acid sequence of the light-chain of humanized  anti-hTfR
       antibody No. 3

<400>  24

Asp Ile Val Met Thr Gln Thr Pro Leu Ser Leu Ser Val Thr Pro Gly
1               5                   10                  15

Gln Pro Ala Ser Ile Ser Cys Arg Ser Ser Gln Ser Leu Val His Ser
            20                  25                  30

Asn Gly Asn Thr Tyr Leu His Trp Tyr Leu Gln Lys Pro Gly Gln Ser
        35                  40                  45

Pro Gln Leu Leu Ile Tyr Lys Val Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Ser Gln Ser
                85                  90                  95

Thr His Val Pro Trp Thr Phe Gly Gln Gly Thr Lys Val Glu Ile Lys
            100                 105                 110

Arg Thr Val Ala Ala Pro Ser Val Phe Ile Phe Pro Pro Ser Asp Glu
        115                 120                 125

Gln Leu Lys Ser Gly Thr Ala Ser Val Val Cys Leu Leu Asn Asn Phe
    130                 135                 140

Tyr Pro Arg Glu Ala Lys Val Gln Trp Lys Val Asp Asn Ala Leu Gln
145                 150                 155                 160

Ser Gly Asn Ser Gln Glu Ser Val Thr Glu Gln Asp Ser Lys Asp Ser
                165                 170                 175

Thr Tyr Ser Leu Ser Ser Thr Leu Thr Leu Ser Lys Ala Asp Tyr Glu
            180                 185                 190

Lys His Lys Val Tyr Ala Cys Glu Val Thr His Gln Gly Leu Ser Ser
        195                 200                 205

Pro Val Thr Lys Ser Phe Asn Arg Gly Glu Cys
    210                 215

<210>  25
<211>  445
<212>  PRT
<213>  Artificial Sequence

<220>
<223>  Amino acid sequence of the heavy-chain of humanized  anti-hTfR
       antibody No. 3 (IgG4)

<400>  25

Glu Val Gln Leu Val Gln Ser Gly Ala Glu Val Lys Lys Pro Gly Glu
1               5                   10                  15

Ser Leu Lys Ile Ser Cys Lys Gly Ser Gly Tyr Ser Phe Thr Asn Tyr
            20                  25                  30

Trp Leu Gly Trp Val Arg Gln Met Pro Gly Lys Gly Leu Glu Trp Met
        35                  40                  45

Gly Asp Ile Tyr Pro Gly Gly Asp Tyr Pro Thr Tyr Ser Glu Lys Phe
    50                  55                  60

Lys Val Gln Val Thr Ile Ser Ala Asp Lys Ser Ile Ser Thr Ala Tyr
65                  70                  75                  80

Leu Gln Trp Ser Ser Leu Lys Ala Ser Asp Thr Ala Met Tyr Tyr Cys
                85                  90                  95

Ala Arg Ser Gly Asn Tyr Asp Glu Val Ala Tyr Trp Gly Gln Gly Thr
            100                 105                 110

Leu Val Thr Val Ser Ser Ala Ser Thr Lys Gly Pro Ser Val Phe Pro
        115                 120                 125

Leu Ala Pro Cys Ser Arg Ser Thr Ser Glu Ser Thr Ala Ala Leu Gly
    130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
        180                 185                 190

Ser Leu Gly Thr Lys Thr Tyr Thr Cys Asn Val Asp His Lys Pro Ser
    195                 200                 205

Asn Thr Lys Val Asp Lys Arg Val Glu Ser Lys Tyr Gly Pro Pro Cys
    210                 215                 220

Pro Pro Cys Pro Ala Pro Glu Phe Leu Gly Gly Pro Ser Val Phe Leu
225                 230                 235                 240

Phe Pro Pro Lys Pro Lys Asp Thr Leu Met Ile Ser Arg Thr Pro Glu
                245                 250                 255

Val Thr Cys Val Val Val Asp Val Ser Gln Glu Asp Pro Glu Val Gln
        260                 265                 270

Phe Asn Trp Tyr Val Asp Gly Val Glu Val His Asn Ala Lys Thr Lys
    275                 280                 285

Pro Arg Glu Glu Gln Phe Asn Ser Thr Tyr Arg Val Val Ser Val Leu
    290                 295                 300

Thr Val Leu His Gln Asp Trp Leu Asn Gly Lys Glu Tyr Lys Cys Lys
305                 310                 315                 320

Val Ser Asn Lys Gly Leu Pro Ser Ser Ile Glu Lys Thr Ile Ser Lys
                325                 330                 335

Ala Lys Gly Gln Pro Arg Glu Pro Gln Val Tyr Thr Leu Pro Pro Ser
        340                 345                 350

Gln Glu Glu Met Thr Lys Asn Gln Val Ser Leu Thr Cys Leu Val Lys
    355                 360                 365

Gly Phe Tyr Pro Ser Asp Ile Ala Val Glu Trp Glu Ser Asn Gly Gln
    370                 375                 380

Pro Glu Asn Asn Tyr Lys Thr Thr Pro Pro Val Leu Asp Ser Asp Gly
385                 390                 395                 400

Ser Phe Phe Leu Tyr Ser Arg Leu Thr Val Asp Lys Ser Arg Trp Gln
                405                 410                 415

Glu Gly Asn Val Phe Ser Cys Ser Val Met His Glu Ala Leu His Asn

                    420                         425                         430

His Tyr Thr Gln Lys Ser Leu Ser Leu Ser Pro Gly Lys
            435                         440                         445

<210>  26
<211>  740
<212>  DNA
<213>  Artificial Sequence

<220>
<223>  Nucleic acid sequence encoding the amino acid sequence of the
       light-chain of humanized  anti-hTfR antibody No. 3, synthetic
       sequence

<400>  26
acgcgtgccg ccaccatggg ctggagctgg attctgctgt tcctcctgag cgtgacagca        60

ggagtgcaca gcgacatcgt gatgacccag actcccctga gcctgagcgt gacacctggc       120

cagcctgcca gcatcagctg cagaagctct cagagcctgg tgcacagcaa cggcaacacc       180

tacctgcact ggtatctgca gaagcccggc cagagccctc agctgctgat ctacaaggtg       240

tccaacagat tcagcggcgt gcccgacaga ttctccggca gcggctctgg caccgacttc       300

accctgaaga tttccagagt ggaagccgag gacgtgggcg tgtactactg cagccagagc       360

acccacgtgc cctggacatt cggccagggc accaaggtgg aaatcaagag aaccgtggcc       420

gctcccagcg tgttcatctt cccacctagc gacgagcagc tgaagtccgg cacagcctct       480

gtcgtgtgcc tgctgaacaa cttctacccc cgcgaggcca aggtgcagtg gaaggtggac       540

aacgccctgc agagcggcaa cagccaggaa agcgtgaccg agcaggactc caaggacagc       600

acctacagcc tgagcagcac cctgaccctg agcaaggccg actacgagaa gcacaaggtg       660

tacgcctgcg aagtgaccca ccagggcctg tctagccccg tgaccaagag cttcaacaga       720

ggcgagtgct aagcggccgc                                                   740

<210>  27
<211>  1330
<212>  PRT
<213>  Artificial Sequence

<220>
<223>  Amino acid sequence of the fusion protein of the heavy chain of
       humanized  anti-hTfR antibody No. 3 (IgG4) and hGAA

<400>  27

Glu Val Gln Leu Val Gln Ser Gly Ala Glu Val Lys Lys Pro Gly Glu
1               5                   10                  15

Ser Leu Lys Ile Ser Cys Lys Gly Ser Gly Tyr Ser Phe Thr Asn Tyr
            20                  25                  30

Trp Leu Gly Trp Val Arg Gln Met Pro Gly Lys Gly Leu Glu Trp Met
        35                  40                  45

Gly Asp Ile Tyr Pro Gly Gly Asp Tyr Pro Thr Tyr Ser Glu Lys Phe
    50                  55                  60

Lys Val Gln Val Thr Ile Ser Ala Asp Lys Ser Ile Ser Thr Ala Tyr
65                  70                  75                  80

Leu Gln Trp Ser Ser Leu Lys Ala Ser Asp Thr Ala Met Tyr Tyr Cys
                85                  90                  95

Ala Arg Ser Gly Asn Tyr Asp Glu Val Ala Tyr Trp Gly Gln Gly Thr
            100                 105                 110

Leu Val Thr Val Ser Ser Ala Ser Thr Lys Gly Pro Ser Val Phe Pro
        115                 120                 125

Leu Ala Pro Cys Ser Arg Ser Thr Ser Glu Ser Thr Ala Ala Leu Gly
    130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190

Ser Leu Gly Thr Lys Thr Tyr Thr Cys Asn Val Asp His Lys Pro Ser
        195                 200                 205

Asn Thr Lys Val Asp Lys Arg Val Glu Ser Lys Tyr Gly Pro Pro Cys
    210                 215                 220

Pro Pro Cys Pro Ala Pro Glu Phe Leu Gly Gly Pro Ser Val Phe Leu
225                 230                 235                 240

Phe Pro Pro Lys Pro Lys Asp Thr Leu Met Ile Ser Arg Thr Pro Glu
                245                 250                 255

Val Thr Cys Val Val Val Asp Val Ser Gln Glu Asp Pro Glu Val Gln
            260                 265                 270

Phe Asn Trp Tyr Val Asp Gly Val Glu Val His Asn Ala Lys Thr Lys
        275                 280                 285

Pro Arg Glu Glu Gln Phe Asn Ser Thr Tyr Arg Val Val Ser Val Leu
    290                 295                 300

Thr Val Leu His Gln Asp Trp Leu Asn Gly Lys Glu Tyr Lys Cys Lys
305                 310                 315                 320

Val Ser Asn Lys Gly Leu Pro Ser Ser Ile Glu Lys Thr Ile Ser Lys
                325                 330                 335

Ala Lys Gly Gln Pro Arg Glu Pro Gln Val Tyr Thr Leu Pro Pro Ser
            340                 345                 350

Gln Glu Glu Met Thr Lys Asn Gln Val Ser Leu Thr Cys Leu Val Lys
        355                 360                 365

Gly Phe Tyr Pro Ser Asp Ile Ala Val Glu Trp Glu Ser Asn Gly Gln
    370                 375                 380

Pro Glu Asn Asn Tyr Lys Thr Thr Pro Pro Val Leu Asp Ser Asp Gly
385                 390                 395                 400

Ser Phe Phe Leu Tyr Ser Arg Leu Thr Val Asp Lys Ser Arg Trp Gln
                405                 410                 415

Glu Gly Asn Val Phe Ser Cys Ser Val Met His Glu Ala Leu His Asn
            420                 425                 430

His Tyr Thr Gln Lys Ser Leu Ser Leu Ser Pro Gly Lys Gly Ser Ala
        435                 440                 445

His Pro Gly Arg Pro Arg Ala Val Pro Thr Gln Cys Asp Val Pro Pro
    450                 455                 460

Asn Ser Arg Phe Asp Cys Ala Pro Asp Lys Ala Ile Thr Gln Glu Gln
465                 470                 475                 480

Cys Glu Ala Arg Gly Cys Cys Tyr Ile Pro Ala Lys Gln Gly Leu Gln
                485                 490                 495

Gly Ala Gln Met Gly Gln Pro Trp Cys Phe Phe Pro Pro Ser Tyr Pro
            500                 505                 510

Ser Tyr Lys Leu Glu Asn Leu Ser Ser Ser Glu Met Gly Tyr Thr Ala
        515                 520                 525

Thr Leu Thr Arg Thr Thr Pro Thr Phe Phe Pro Lys Asp Ile Leu Thr

530                 535                 540

Leu Arg Leu Asp Val Met Met Glu Thr Glu Asn Arg Leu His Phe Thr
545                 550                 555                 560

Ile Lys Asp Pro Ala Asn Arg Arg Tyr Glu Val Pro Leu Glu Thr Pro
                565                 570                 575

Arg Val His Ser Arg Ala Pro Ser Pro Leu Tyr Ser Val Glu Phe Ser
            580                 585                 590

Glu Glu Pro Phe Gly Val Ile Val His Arg Gln Leu Asp Gly Arg Val
        595                 600                 605

Leu Leu Asn Thr Thr Val Ala Pro Leu Phe Phe Ala Asp Gln Phe Leu
    610                 615                 620

Gln Leu Ser Thr Ser Leu Pro Ser Gln Tyr Ile Thr Gly Leu Ala Glu
625                 630                 635                 640

His Leu Ser Pro Leu Met Leu Ser Thr Ser Trp Thr Arg Ile Thr Leu
                645                 650                 655

Trp Asn Arg Asp Leu Ala Pro Thr Pro Gly Ala Asn Leu Tyr Gly Ser
            660                 665                 670

His Pro Phe Tyr Leu Ala Leu Glu Asp Gly Gly Ser Ala His Gly Val
        675                 680                 685

Phe Leu Leu Asn Ser Asn Ala Met Asp Val Val Leu Gln Pro Ser Pro
    690                 695                 700

Ala Leu Ser Trp Arg Ser Thr Gly Gly Ile Leu Asp Val Tyr Ile Phe
705                 710                 715                 720

Leu Gly Pro Glu Pro Lys Ser Val Val Gln Gln Tyr Leu Asp Val Val
                725                 730                 735

Gly Tyr Pro Phe Met Pro Pro Tyr Trp Gly Leu Gly Phe His Leu Cys
            740                 745                 750

Arg Trp Gly Tyr Ser Ser Thr Ala Ile Thr Arg Gln Val Val Glu Asn
        755                 760                 765

Met Thr Arg Ala His Phe Pro Leu Asp Val Gln Trp Asn Asp Leu Asp
    770                 775                 780

Tyr Met Asp Ser Arg Arg Asp Phe Thr Phe Asn Lys Asp Gly Phe Arg
785                 790                 795                 800

Asp Phe Pro Ala Met Val Gln Glu Leu His Gln Gly Gly Arg Arg Tyr
                805                 810                 815

Met Met Ile Val Asp Pro Ala Ile Ser Ser Ser Gly Pro Ala Gly Ser
            820                 825                 830

Tyr Arg Pro Tyr Asp Glu Gly Leu Arg Arg Gly Val Phe Ile Thr Asn
        835                 840                 845

Glu Thr Gly Gln Pro Leu Ile Gly Lys Val Trp Pro Gly Ser Thr Ala
    850                 855                 860

Phe Pro Asp Phe Thr Asn Pro Thr Ala Leu Ala Trp Trp Glu Asp Met
865                 870                 875                 880

Val Ala Glu Phe His Asp Gln Val Pro Phe Asp Gly Met Trp Ile Asp
                885                 890                 895

Met Asn Glu Pro Ser Asn Phe Ile Arg Gly Ser Glu Asp Gly Cys Pro
            900                 905                 910

Asn Asn Glu Leu Glu Asn Pro Pro Tyr Val Pro Gly Val Val Gly Gly
        915                 920                 925

Thr Leu Gln Ala Ala Thr Ile Cys Ala Ser Ser His Gln Phe Leu Ser
    930                 935                 940

Thr His Tyr Asn Leu His Asn Leu Tyr Gly Leu Thr Glu Ala Ile Ala
945                 950                 955                 960

Ser His Arg Ala Leu Val Lys Ala Arg Gly Thr Arg Pro Phe Val Ile
                965                 970                 975

Ser Arg Ser Thr Phe Ala Gly His Gly Arg Tyr Ala Gly His Trp Thr
            980                 985                 990

Gly Asp Val Trp Ser Ser Trp Glu  Gln Leu Ala Ser Ser  Val Pro Glu
        995                 1000                1005

Ile Leu  Gln Phe Asn Leu Leu  Gly Val Pro Leu Val  Gly Ala Asp
    1010                1015                1020

Val Cys  Gly Phe Leu Gly Asn  Thr Ser Glu Glu Leu  Cys Val Arg
    1025                1030                1035

Trp Thr Gln Leu Gly Ala Phe Tyr Pro Phe Met Arg Asn His Asn
    1040                1045                1050

Ser Leu Leu Ser Leu Pro Gln Glu Pro Tyr Ser Phe Ser Glu Pro
    1055                1060                1065

Ala Gln Gln Ala Met Arg Lys Ala Leu Thr Leu Arg Tyr Ala Leu
    1070                1075                1080

Leu Pro His Leu Tyr Thr Leu Phe His Gln Ala His Val Ala Gly
    1085                1090                1095

Glu Thr Val Ala Arg Pro Leu Phe Leu Glu Phe Pro Lys Asp Ser
    1100                1105                1110

Ser Thr Trp Thr Val Asp His Gln Leu Leu Trp Gly Glu Ala Leu
    1115                1120                1125

Leu Ile Thr Pro Val Leu Gln Ala Gly Lys Ala Glu Val Thr Gly
    1130                1135                1140

Tyr Phe Pro Leu Gly Thr Trp Tyr Asp Leu Gln Thr Val Pro Ile
    1145                1150                1155

Glu Ala Leu Gly Ser Leu Pro Pro Pro Pro Ala Ala Pro Arg Glu
    1160                1165                1170

Pro Ala Ile His Ser Glu Gly Gln Trp Val Thr Leu Pro Ala Pro
    1175                1180                1185

Leu Asp Thr Ile Asn Val His Leu Arg Ala Gly Tyr Ile Ile Pro
    1190                1195                1200

Leu Gln Gly Pro Gly Leu Thr Thr Thr Glu Ser Arg Gln Gln Pro
    1205                1210                1215

Met Ala Leu Ala Val Ala Leu Thr Lys Gly Gly Glu Ala Arg Gly
    1220                1225                1230

Glu Leu Phe Trp Asp Asp Gly Glu Ser Leu Glu Val Leu Glu Arg
    1235                1240                1245

Gly Ala Tyr Thr Gln Val Ile Phe Leu Ala Arg Asn Asn Thr Ile
    1250                1255                1260

Val Asn Glu Leu Val Arg Val Thr Ser Glu Gly Ala Gly Leu Gln
    1265                1270                1275

Leu Gln Lys Val Thr Val Leu Gly Val Ala Thr Ala Pro Gln Gln
    1280                1285                1290

Val Leu Ser Asn Gly Val Pro Val Ser Asn Phe Thr Tyr Ser Pro
    1295                1300                1305

Asp Thr Lys Val Leu Asp Ile Cys Val Ser Leu Leu Met Gly Glu
    1310                1315                1320

Gln Phe Leu Val Ser Trp Cys
    1325                1330

<210> 28
<211> 4076
<212> DNA
<213> Artificial Sequence

<220>
<223> Nucleic acid sequence encoding the amino acid sequence of the
fusion protein of the heavy chain of humanized anti-hTfR
antibody No. 3 (IgG4) and hGAA, synthetic sequence

<400> 28
acgcgtgccg ccaccatggg ctggagctgg attctgctgt tcctcctgag cgtgacagca 60

ggagtgcaca gcgaggtgca actagtgcag tctggagcag aggtgaaaaa gcccggggag 120

tctctgaaga tttcctgtaa gggttctgga tacagcttta ccaactactg gctgggatgg 180

gtgcgccaga tgcccgggaa aggcctggag tggatggggg acatctaccc cggcggagac 240

taccctacat acagcgagaa gttcaaggtc caggtcacca tctcagccga caagtccatc 300

agcaccgcct acctgcagtg gagcagcctg aaggcctcgg acaccgccat gtattactgt 360

gcgagatcag gcaattacga cgaagtggcc tactggggcc aaggaaccct ggtcaccgtc 420

tcctcagcta gcaccaaggg cccatcggtc ttccccctgg cgccctgctc caggagcacc 480

tccgagagca cagccgccct gggctgcctg gtcaaggact acttccccga accggtgacg 540

gtgtcgtgga actcaggcgc cctgaccagc ggcgtgcaca ccttcccggc tgtcctacag 600

tcctcaggac tctactccct cagcagcgtg gtgaccgtgc cctccagcag cttgggcacg 660

aagacctaca cctgcaacgt agatcacaag cccagcaaca ccaaggtgga caagagagtt 720

gagtccaaat atggtccccc atgcccacca tgcccagcac ctgagttcct ggggggtcca 780

tcagtcttcc tgttcccccc aaaacccaag gacactctca tgatctcccg gacccctgag 840

gtcacgtgcg tggtggtgga cgtgagccag gaagaccccg aggtccagtt caactggtac 900

gtggatggcg tggaggtgca taatgccaag acaaagccgc gggaggagca gttcaacagc 960

acgtaccgtg tggtcagcgt cctcaccgtc ctgcaccagg actggctgaa cggcaaggag 1020

tacaagtgca aggtctccaa caaaggcctc ccgtcctcca tcgagaaaac catctccaaa 1080

gccaaagggc agccccgaga gccacaggtg tacaccctgc ccccatccca ggaggagatg 1140

accaagaacc aggtcagcct gacctgcctg gtcaaaggct tctaccccag cgacatcgcc 1200

gtggagtggg agagcaatgg gcagccggag aacaactaca agaccacgcc tcccgtgctg 1260

gactccgacg gctccttctt cctctacagc aggctcaccg tggacaagag caggtggcag 1320

gaggggaatg tcttctcatg ctccgtgatg catgaggctc tgcacaacca ctacacacag 1380

aagagcctct ccctgtctcc gggtaaagga tctgctcacc ctggacggcc tagggccgtg 1440

cctacccagt gcgacgtgcc tcctaattcc aggttcgact gcgcccccga taaagccatc 1500

acccaggaac agtgtgaggc caggggctgt tgctatattc ccgccaagca gggcctgcag 1560

ggcgctcaga tgggccagcc ctggtgcttc ttccccccct cctatccctc ctacaagctg 1620

gaaaacctgt cctcctccga gatgggctac acagccaccc tcacaaggac aacccccacc 1680

ttcttcccca aggacatcct caccctgcgg ctggatgtga tgatggagac cgagaaccgg 1740

ctgcacttca ccatcaagga ccctgccaat cggcggtacg aggtgcctct ggaaaccccc 1800

cgggtgcact ccagggctcc ttcccctctg tatagcgtgg agtttagcga ggagcccttc 1860

ggcgtgatcg tgcaccggca gctcgacgga agggtgctgc tgaacaccac cgtcgccccc 1920

ctgttcttcg ccgatcagtt cctccagctg tccacctccc tgccctccca gtacattacc 1980

ggcctggctg agcacctgag ccccctgatg ctgtccacca gctggacaag gatcaccctg 2040

tggaatcggg acctcgctcc cacccctgga gccaatctgt acggcagcca ccccttctac 2100

ctggccctcg aagatggagg cagcgctcat ggcgtgttcc tgctgaattc caacgctatg 2160

gacgtggtgc tccaaccctc ccctgccctg tcctggagga gcacaggcgg cattctggac 2220

gtctacattt tcctcggccc cgagcctaaa tccgtcgtcc agcagtacct ggacgtggtg 2280

ggctaccctt ttatgcctcc ttactggggc ctgggctttc acctgtgtag gtggggctat 2340

tccagcaccg ctattacacg gcaggtcgtc gaaaacatga cacgggccca ctttcccctg 2400

gacgtccaat ggaatgacct ggactacatg gactcccggc gggacttcac attcaataag 2460

gacggcttcc gggacttccc tgccatggtg caggagctgc atcagggagg ccggaggtac 2520

atgatgattg tggaccctgc tatttccagc tccggacctg ctggctccta ccggccctac 2580

gacgagggac tgcggagggg cgtcttcatc acaaacgaaa ccggtcagcc cctgattgga 2640

aaggtgtggc ccggttctac agctttcccc gactttacaa accccacagc cctggcttgg 2700

tgggaggaca tggtggccga gtttcacgac caggtgcctt tcgacggcat gtggatcgat 2760

atgaacgagc cttccaactt tatcaggggc agcgaggatg gctgccccaa caacgagctc 2820

gagaatcccc cctacgtccc cggagtcgtg ggaggcacac tccaggctgc cacaatctgc 2880

gccagctccc atcagttcct gagcacccat tacaacctgc ataacctgta cggcctcacc 2940

gaagccatcg ccagccatag ggctctggtg aaggccaggg gaacacggcc tttcgtgatc     3000

tccaggagca catttgccgg ccacggcagg tacgctggcc actggacagg cgatgtctgg     3060

tcctcctggg agcaactggc ttccagcgtc cccgagatcc tccagttcaa cctgctgggc     3120

gtgcccctgg tgggcgccga cgtgtgcggc tttctcggca acacctccga ggagctgtgt     3180

gtgcggtgga cccagctggg cgccttctat ccctttatgc ggaatcacaa cagcctgctg     3240

tccctgcctc aggaacccta cagcttcagc gaacctgctc agcaggccat gcggaaggcc     3300

ctgaccctcc ggtatgccct gctgccccac ctctataccc tgtttcatca ggctcatgtg     3360

gctggcgaga cagtggctag gcctctgttc ctggagtttc ccaaggattc cagcacatgg     3420

acagtggacc atcagctgct gtggggcgag gctctgctga tcacacccgt gctgcaggcc     3480

ggcaaagccg aggtgacagg ctatttcccc ctgggaacct ggtacgacct ccagacagtg     3540

cccattgagg ccctgggttc tctccctcct cctcctgccg ctcccaggga acctgctatc     3600

cactccgaag gccagtgggt gaccctgcct gctcccctgg acacaatcaa cgtccacctg     3660

cgggctggct acattattcc tctccaggga cccggcctga caaccacaga gagcaggcag     3720

cagcccatgg ccctggctgt cgctctgaca aaaggaggcg aggcccgggg agagctcttc     3780

tgggatgacg gcgagagcct ggaggtcctg gagaggggag cctataccca ggtgatcttc     3840

ctcgcccgga acaacaccat tgtgaacgag ctggtgaggg tcacatccga aggagccgga     3900

ctgcaactgc agaaagtgac agtgctgggc gtcgccaccg ctcctcaaca ggtgctgtcc     3960

aacggcgtgc ccgtctccaa ctttacctac agccccgaca ccaaggtgct ggacatttgt     4020

gtgtccctgc tgatgggcga acagtttctg gtgtcctggt gttaataagc ggccgc     4076

<210>  29
<211>  845
<212>  PRT
<213>  Artificial Sequence

<220>
<223>  Amino acid sequence of the fusion protein of the heavy chain of
       humanized  anti-hTfR antibody No. 3 (IgG4) and h alfa-galA

<400>  29

Glu Val Gln Leu Val Gln Ser Gly Ala Glu Val Lys Lys Pro Gly Glu
1               5                   10                  15

Ser Leu Lys Ile Ser Cys Lys Gly Ser Gly Tyr Ser Phe Thr Asn Tyr
            20                  25                  30

Trp Leu Gly Trp Val Arg Gln Met Pro Gly Lys Gly Leu Glu Trp Met
        35                  40                  45

Gly Asp Ile Tyr Pro Gly Gly Asp Tyr Pro Thr Tyr Ser Glu Lys Phe
    50                  55                  60

Lys Val Gln Val Thr Ile Ser Ala Asp Lys Ser Ile Ser Thr Ala Tyr
65                  70                  75                  80

Leu Gln Trp Ser Ser Leu Lys Ala Ser Asp Thr Ala Met Tyr Tyr Cys
                85                  90                  95

Ala Arg Ser Gly Asn Tyr Asp Glu Val Ala Tyr Trp Gly Gln Gly Thr
            100                 105                 110

Leu Val Thr Val Ser Ser Ala Ser Thr Lys Gly Pro Ser Val Phe Pro
        115                 120                 125

Leu Ala Pro Cys Ser Arg Ser Thr Ser Glu Ser Thr Ala Ala Leu Gly
    130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190

Ser Leu Gly Thr Lys Thr Tyr Thr Cys Asn Val Asp His Lys Pro Ser
        195                 200                 205

Asn Thr Lys Val Asp Lys Arg Val Glu Ser Lys Tyr Gly Pro Pro Cys
    210                 215                 220

Pro Pro Cys Pro Ala Pro Glu Phe Leu Gly Gly Pro Ser Val Phe Leu
225                 230                 235                 240

Phe Pro Pro Lys Pro Lys Asp Thr Leu Met Ile Ser Arg Thr Pro Glu
                245                 250                 255

Val Thr Cys Val Val Val Asp Val Ser Gln Glu Asp Pro Glu Val Gln
            260                 265                 270

Phe Asn Trp Tyr Val Asp Gly Val Glu Val His Asn Ala Lys Thr Lys
        275                 280                 285

Pro Arg Glu Glu Gln Phe Asn Ser Thr Tyr Arg Val Val Ser Val Leu
    290                 295                 300

Thr Val Leu His Gln Asp Trp Leu Asn Gly Lys Glu Tyr Lys Cys Lys
305                 310                 315                 320

Val Ser Asn Lys Gly Leu Pro Ser Ser Ile Glu Lys Thr Ile Ser Lys
                325                 330                 335

Ala Lys Gly Gln Pro Arg Glu Pro Gln Val Tyr Thr Leu Pro Pro Ser
            340                 345                 350

Gln Glu Glu Met Thr Lys Asn Gln Val Ser Leu Thr Cys Leu Val Lys
        355                 360                 365

Gly Phe Tyr Pro Ser Asp Ile Ala Val Glu Trp Glu Ser Asn Gly Gln
    370                 375                 380

Pro Glu Asn Asn Tyr Lys Thr Thr Pro Pro Val Leu Asp Ser Asp Gly
385                 390                 395                 400

Ser Phe Phe Leu Tyr Ser Arg Leu Thr Val Asp Lys Ser Arg Trp Gln
                405                 410                 415

Glu Gly Asn Val Phe Ser Cys Ser Val Met His Glu Ala Leu His Asn
            420                 425                 430

His Tyr Thr Gln Lys Ser Leu Ser Leu Ser Pro Gly Lys Gly Ser Leu
        435                 440                 445

Asp Asn Gly Leu Ala Arg Thr Pro Thr Met Gly Trp Leu His Trp Glu
    450                 455                 460

Arg Phe Met Cys Asn Leu Asp Cys Gln Glu Glu Pro Asp Ser Cys Ile
465                 470                 475                 480

Ser Glu Lys Leu Phe Met Glu Met Ala Glu Leu Met Val Ser Glu Gly
                485                 490                 495

Trp Lys Asp Ala Gly Tyr Glu Tyr Leu Cys Ile Asp Asp Cys Trp Met
            500                 505                 510

Ala Pro Gln Arg Asp Ser Glu Gly Arg Leu Gln Ala Asp Pro Gln Arg
        515                 520                 525

Phe Pro His Gly Ile Arg Gln Leu Ala Asn Tyr Val His Ser Lys Gly
    530                 535                 540

Leu Lys Leu Gly Ile Tyr Ala Asp Val Gly Asn Lys Thr Cys Ala Gly
545                 550                 555                 560

Phe Pro Gly Ser Phe Gly Tyr Tyr Asp Ile Asp Ala Gln Thr Phe Ala
                565                 570                 575

Asp Trp Gly Val Asp Leu Leu Lys Phe Asp Gly Cys Tyr Cys Asp Ser
            580                 585                 590

Leu Glu Asn Leu Ala Asp Gly Tyr Lys His Met Ser Leu Ala Leu Asn
        595                 600                 605

Arg Thr Gly Arg Ser Ile Val Tyr Ser Cys Glu Trp Pro Leu Tyr Met
    610                 615                 620

Trp Pro Phe Gln Lys Pro Asn Tyr Thr Glu Ile Arg Gln Tyr Cys Asn
625                 630                 635                 640

His Trp Arg Asn Phe Ala Asp Ile Asp Asp Ser Trp Lys Ser Ile Lys
                645                 650                 655

Ser Ile Leu Asp Trp Thr Ser Phe Asn Gln Glu Arg Ile Val Asp Val
            660                 665                 670

Ala Gly Pro Gly Gly Trp Asn Asp Pro Asp Met Leu Val Ile Gly Asn
        675                 680                 685

Phe Gly Leu Ser Trp Asn Gln Gln Val Thr Gln Met Ala Leu Trp Ala
    690                 695                 700

Ile Met Ala Ala Pro Leu Phe Met Ser Asn Asp Leu Arg His Ile Ser
705                 710                 715                 720

Pro Gln Ala Lys Ala Leu Leu Gln Asp Lys Asp Val Ile Ala Ile Asn
                725                 730                 735

Gln Asp Pro Leu Gly Lys Gln Gly Tyr Gln Leu Arg Gln Gly Asp Asn
            740                 745                 750

Phe Glu Val Trp Glu Arg Pro Leu Ser Gly Leu Ala Trp Ala Val Ala
        755                 760                 765

Met Ile Asn Arg Gln Glu Ile Gly Gly Pro Arg Ser Tyr Thr Ile Ala
    770                 775                 780

Val Ala Ser Leu Gly Lys Gly Val Ala Cys Asn Pro Ala Cys Phe Ile
785                 790                 795                 800

Thr Gln Leu Leu Pro Val Lys Arg Lys Leu Gly Phe Tyr Glu Trp Thr

805                 810                 815

Ser Arg Leu Arg Ser His Ile Asn Pro Thr Gly Thr Val Leu Leu Gln
            820                 825                 830

Leu Glu Asn Thr Met Gln Met Ser Leu Lys Asp Leu Leu
        835                 840                 845

<210>  30
<211>  20
<212>  DNA
<213>  Artificial Sequence

<220>
<223>  Primer Hyg-Sfi5', synthetic sequence

<400>  30
gaggccgcct cggcctctga                                                   20

<210>  31
<211>  29
<212>  DNA
<213>  Artificial Sequence

<220>
<223>  Primer Hyg-BstX3', synthetic sequence

<400>  31
aaccatcgtg atgggtgcta ttcctttgc                                         29

<210>  32
<211>  15669
<212>  DNA
<213>  Artificial Sequence

<220>
<223>  Nucelic acid sequence of GAA knockout vector, synthetic sequence

<400>  32
ggcggccgct tgatacctta caactacatg aaggctcata accatctgta cagatacagt      60

gtactcatat acataaaata aataattttt taaaaaagaa gccgaagctg agcgtggtgg     120

cacatgcctt taaccccagc acttgggagg cagaggcagg tagatttctg agttccaggc     180

cagcctggtc tatagagtga gttccaggac agccagggct gtacacagaa accctgtctc     240

ggaaaaacaa aaaaagaaaa agaaaaacat tcccatcact taacttttct aaaagattta     300

ttatgtatac aacattctgc ctgcacttac gtctgcaggc cagaagaggg caccagatct     360

cattatagat agtcatgagc caccaggtgg ttgctgggat ttgaactcag gacctctgga     420

agagcagcca gtgctctcaa cctctgagcc atctctccag cccacgtaat tgttttaaga     480

agccaaaatt ctcaccatac tgtctcccct ctcctagatc aaagatcctg ctagtaagcg     540

ctacgaagtg cccctggaga ccccacgtgt gctgagccag gcaccatccc cactttacag     600

cgtggaattc tcagaggaac cctttggagt gatcgttcgt aggaagcttg gtggccgagt 660

gttgtgagtt ggttacctga ggctttgact ccaccctgag tggcccctta gggtcagagc 720

ttctgcaatc agcaaacctg tctacctcat tccaaaccct gcctttttct gtcttgtttt 780

ctgttgctgt ggtaaacacg aacagaagcc acgtggggaa gagaaagtgg tttacctcac 840

aggttctagg ccatgctcca tcactgccgg ggcaggaact gaagcagagg ccatggagga 900

gtgctgttta ctgccttagt cctcatggct cacttagctt aagttcttat ccagcccaga 960

actaccagcc ctagggtgtt accaccctca gtgagctagg ccccacccac aacgaactag 1020

gccccaccca cagtgagcta ggccccaccc acaacaagct aggccccacc cacagtgtgc 1080

taggccctcc cacatcagtc aaaataaaaa atactccctc aggttactta cagaccaatc 1140

taatggaagt atgttctcag tggagagtca ttccctcttc tagatggccc tcgcttgtat 1200

caagtgatca cccagttctt ggcttccagc ttcctcattg ggtcccagag gttccctctg 1260

tggcttccca gtgaggagtg gtgcctccca ggagagacag gcagtgtgtg gagatgaccc 1320

accacagagg atgatccatc caagtgccag ggggactagg ggaagacagt tgtccgtcca 1380

cagtggcgcc ccctgcaggc ggcacactct gccagccgcc tctcttaagc cttgtgtttc 1440

tccaaggtcc atgtccccca gacaggacct catgctggta cccctgtgcc ctctgacctg 1500

gggtgacatg ggaccccctt gggcatgcct gctccggctt ctcccactgt cttcagatgc 1560

tgagagcctc tgggctctat gtggccgttg tcccagggtt gtaatctggc gtccccccac 1620

aggctgaaca caaccgtggc ccccctgttc ttcgctgacc agttcctgca gctgtccact 1680

tccctgccct cccagcacat cacaggcctg ggggaacacc tcagcccact catgctcagc 1740

accgactggg ctcgtatcac cctctggaac cgggacacac caccctcggt aagagacggg 1800

cagtgggtgg gagggtggag gctgggatcc tgagtgcaag gcgttgtctc ttgtcacccc 1860

agcaaggtac caacctctac gggtcacatc ctttctacct ggcactggag gacggtggct 1920

tggctcacgg tgtcttcttg ctaaacagca atgccatggg tgagtcacca gactggggct 1980

cctcagcggt cacttccgtg tgagagcttg cccgctccag agtctgcctg tctccaagcg 2040

acctccgtgg agagagtccc gtgagcagag tccccctcat cctcggaacc taaggaggtc 2100

ctgggtgatt ctccaggctc acatgttcac gtccttcagg ccttgctatg ttgagctcag 2160

tacagctgcc tcaggcccac cctgaggaca tctccccaca attcatattg gaagggcgcg 2220

ccataacttc gtataatgta tgctatacga agttatagcc acactccacc cacaggttga 2280

gtcccccatc tgtggggcac ttgttcctga gcccaaacac ttctttccag atgtcatcct 2340

gcaacccagc ccagccctaa cctggaggtc aacgggcggg tgaaattcta ccgggtaggg 2400

gaggcgcttt tcccaaggca gtctggagca tgcgctttag cagccccgct gggcacttgg 2460

cgctacacaa gtggcctctg gcctcgcaca cattccacat ccaccggtag gcgccaaccg 2520

gctccgttct ttggtggccc cttcgcgcca ccttctactc ctcccctagt caggaagttc 2580

ccccccgccc cgcagctcgc gtcgtgcagg acgtgacaaa tggaagtagc acgtctcact 2640

agtctcgtgc agatggacag caccgctgag caatggaagc gggtaggcct ttggggcagc 2700

ggccaatagc agctttgctc cttcgctttc tgggctcaga ggctgggaag gggtgggtcc 2760

gggggcgggc tcaggggcgg gctcaggggc ggggcgggcg cccgaaggtc ctccggaggc 2820

ccggcattct gcacgcttca aaagcgcacg tctgccgcgc tgttctcctc ttcctcatct 2880

ccgggccttt cgacctgcag ccaatatggg atcggccatt gaacaagatg gattgcacgc 2940

aggttctccg gccgcttggg tggagaggct attcggctat gactgggcac aacagacaat 3000

cggctgctct gatgccgccg tgttccggct gtcagcgcag gggcgcccgg ttctttttgt 3060

caagaccgac ctgtccggtg ccctgaatga actgcaggac gaggcagcgc ggctatcgtg 3120

gctggccacg acgggcgttc cttgcgcagc tgtgctcgac gttgtcactg aagcgggaag 3180

ggactggctg ctattgggcg aagtgccggg gcaggatctc ctgtcatctc accttgctcc 3240

tgccgagaaa gtatccatca tggctgatgc aatgcggcgg ctgcatacgc ttgatccggc 3300

tacctgccca ttcgaccacc aagcgaaaca tcgcatcgag cgagcacgta ctcggatgga 3360

agccggtctt gtcgatcagg atgatctgga cgaagagcat caggggctcg cgccagccga 3420

actgttcgcc aggctcaagg cgcgcatgcc cgacggcgat gatctcgtcg tgacccatgg 3480

cgatgcctgc ttgccgaata tcatggtgga aaatggccgc ttttctggat tcatcgactg 3540

tggccggctg ggtgtggcgg accgctatca ggacatagcg ttggctaccc gtgatattgc 3600

tgaagagctt ggcggcgaat gggctgaccg cttcctcgtg ctttacggta tcgccgctcc 3660

cgattcgcag cgcatcgcct tctatcgcct tcttgacgag ttcttctgag gggatcgatc 3720

cgctgtaagt ctgcagaaat tgatgatcta ttaaacaata aagatgtcca ctaaaatgga 3780

agtttttcct gtcatacttt gttaagaagg gtgagaacag agtacctaca ttttgaatgg 3840

aaggattgga gctacggggg tgggggtggg gtgggattag ataaatgcct gctctttact 3900

gaaggctctt tactattgct ttatgataat gtttcatagt tggatatcat aatttaaaca 3960

agcaaaacca aattaagggc cagctcattc ctcccactca tgatctatag atctatagat 4020

ctctcgtggg atcattgttt ttctcttgat tcccactttg tggttctaag tactgtggtt 4080

tccaaatgtg tcagtttcat agcctgaaga acgagatcag cagcctctgt tccacataca 4140

cttcattctc agtattgttt tgccaagttc taattccatc agaagctatc ctggatgtgt 4200

atgtgttcct aggcccagag cccaagagcg ttgtgcaaca atacctggat gttgtgggta 4260

ggcataactt cgtataatgt atgctatacg aagttatgtc gacctggccc ctgcccactg 4320

agctcagacc cctgcccctt gctccttgcc ccatgtcccc agtccctgct cactcgcagt 4380

actggtcctc aggatacccc ttcatgcctc catactgggg cctcggcttc cacctctgcc 4440

gctggggcta ctcctcgacc gccattgtcc gccaggtagt ggagaacatg accaggacac 4500

acttcccgct ggtgagtggc agtcagggtg agggcggccc cagcttccgt gctcttggct 4560

cccgacctca gcatcagctc gcgtcctctg cttcaggacg tgcaatggaa tgacctggac 4620

tacatggacg cccgaagaga cttcaccttc aaccaggaca gctttgccga cttcccagac 4680

atggtgcggg agctgcacca ggatggccgg cgctacatga tgatcgtggt aagcaggttt 4740

gggcacagcc attgctgctg cttccgtgtg gcctcttcct cctacctgag cagtttccca 4800

ggacactctc tgatgtgtct gctgcccagg ctcctggaca cacccatgag ctcggggctc 4860

tttggttaac aggcgggatc cgcagaactg ctctgcctta aagcctgcgg caggggccgt 4920

aacgagctgg tactgagctg aggacctaga tgtggtttgt gcgtcctcct ggccctgctc 4980

ctctctcagg ccagctatgt ccatgtggga aggaaacata cttgccctgt gcctgtcccc 5040

gcagcaggaa ggtccaagaa gaccaaacag tcacagccat gggatgtcct tgaggggagg 5100

gagctaagcc ctggaagata cggctttgta aggaagggct acgactgcct cgccgccacc 5160

accaccacca ccaccaccac ggggcagtga atagtggtac aggcagtgag ggccacccag 5220

acattaggca agccctccac cactgagctg cactgccaac tctctttccg gtttttattt 5280

gaggcagggc cacaccagat tgcccagcca ggccttgaat ccataatcct cccacttccg 5340

cggtccaggc agctgggatg atgaacctgt atcaccagcc cccgtgcaga tttaagacag 5400

ggtctcctca tgtagctctg actgacccat ctgtcactgt atagagtaga ccaggctggc 5460

ctcaaactca cccgcctgcc tcttagtctc tcagcagctg gaactaaagg cgtgcgccac 5520

catgcctggg tgctttttat tcatctccat ttttaaaaag taagtggggc tggaaagatg 5580

gctgagcagt taaagcacac tgctctcgaa gaggactgga gttcggtgcc tgggacccac 5640

accaagcagc tgtacttgtc atttcagcag ctgctgctgg cctctgtggg tcacgtgcat 5700

tcgcgtgcgt gcgcgcgcgc gcacacacac acacagacac acacacacag acacacaagt 5760

aataataagc tggacagtgt ggtaaacacc tgtttttttt ttattattta tttatttatt 5820

tatttgagca cactggagct gtcttcagac acaccagaag agggcatcag atcccattac 5880

agatgtttgt gagctaccat gtggttttgg ggatttgaac tcaggacctc tggaagagca 5940

gcagtcagtg ctcttaacct ctgagccatc tccccagcgc atacagtgaa cacctttaat 6000

cacagctctc gggaggcaga ggcaggtgca tctcttgagt tctgtgccaa cccggtctac 6060

agagtgagtt tcaaggcagc caagaatata caaagaaacc ccatctttaa aattaaaaat 6120

aaaataaaaa gagcaaatat aagcaactgc tgtaactctg gaaagcctga gaacaggagg 6180

atcacacgtc tagacttgct cacccctgct gcccccaaca ggcttcgcac agcctgacct 6240

atgccgcttt cctgtatagg ccatctctgc ctcggccagc tctcttctct gggacctgag 6300

cttctacctc tgccgctccg gctgctcctc cttctgggct ttggggctag gctcttcact 6360

gttggcttct ccccccagga ccctgccatc agcagcgcag gccctgctgg gagttacagg 6420

ccctacgacg agggtctgcg gaggggcgtg ttcatcacca acgagactgg gcagccgctg 6480

attgggaagg tagcgagggg gatgggagct gtggtggtgg tggtggtggt ggggtcctca 6540

ctgggggtaa ctcctgacag tcgcctgagg ttttctaagt ggagggtgtt ctcaggcatc 6600

ctgagtagct gggcccggct taagcctagc agacctttcg tgaggccttg gagattgggg 6660

ggaggggagt cttccttgtc cctgggggcc ttctgggagt tctgagtccc agcctcccca 6720

tcctaagcag ccctggccat gggagaagtg agggcttgtg tctggtggtc agggatcaat 6780

ggcactatct caacctcttt cccatgccct ggttcatggc tcatctgccc cacggcctta 6840

tgtgtagggc tggcctttgc tctcccaagg ctgaaggccc ctatgccctg aggcattgtt 6900

tgatattggg cttgaggctc tctgatatca tcattggctt agagcgaggc tgctgccttg 6960

cttgaggcat gaggcccaac agggctctgg cgcaggatct gtgtggtatt ggttcttccc 7020

tctgggctct tgttcaggtt tggcctggaa ccaccgcctt ccctgatttc accaaccctg 7080

agacccttga ctggtggcag gacatggtgt ctgagttcca cgcccaggtg cccttcgatg 7140

gcatgtggct cgtaagtgca gtcccatccc tccatgggta cacccccagc ctctggggac 7200

caacctagct cacatctctg cctcttccag gacatgaacg aaccgtccaa cttcgttaga 7260

ggctctcagc agggctgccc caacaatgaa ctggagaacc ccccctatgt gcccggtgag 7320

ccctgcttgc ctgcccggaa tggggatagt gctagagtta gtggcttaaa ccagggagtg 7380

tgcgcctctc tgtggtttgg gggatgtaaa gctctccatt tttttggccg tcaatagaca 7440

cagcagcttc gggacacaga acccaggagt gaagtcctac agtgtgtgac acagacatgg 7500

tcacagctag ccagtgcatc aggggaaggg ggttaggccc ggatgacaga cacttaggat 7560

gagtcaagag aaggtggatg tggagactgt aggtttagga ggaccgtctt tcgtacacct 7620

gtctttggac tagcagaagt gtatagtgat gtggggggat ggggatggga ccagtttcag 7680

gaggctccgc cctgggcaga acccaaggag gtgtcaatca aagcagaggg tgtgggccaa 7740

ggagagggca cagggaaccc tggagagagt tacacctaaa actagtcctc ctctcgccta 7800

ggggtggttg gcgggatctt gcaggcagcc accatctgtg cctccagcca ccaattcctc 7860

tccacacact acaacctcca caacctgtac ggcctcactg aagctatcgc ctccagcagg 7920

tgaggctctg ccggctctgc accagccagc ctgccctgca atcacagtgg gggtcgagag 7980

gtgggctggc tcatgtcagg gtcatgaaag gaagggaaag aggttaccat accacccatg 8040

ggccagcgat gcctgcacgt ctgctgaaat ggttgctgtt ttccccctcc cccccccagc 8100

atggagccgg cttctccccc ctatgcccag accagcccag atgttaagtg gggatgtcca 8160

aagggactcc atgtctctgg caaggcttag agtggtgact cacctgtaaa tgacttaaga 8220

gtcattgggg caggcctgac tgtacctagc atactttaaa gatggcctct ctttcccacc 8280

cattgtgatc tactaaacta tcagttcctc ttggccctga tccccttgct gctggccccc 8340

atgctctgtt ccagcacacg ggtgtgtatg acccaggtga gcccaggtct ctgtgttctc 8400

atcccactca gggccctggt caagactcgg ggaacacgac cctttgtgat ctcccgctca 8460

accttctcgg gccacggccg gtacgctggt cactggacag gggatgtgcg gagctcttgg 8520

gagcatcttg catactctgt gccaggtgag tacaagctgc ctgcagggct ctggggtctt 8580

ttctgggggt ctttgggctg tgggctcacc actgcagatg gtattgtgat agcttgggga 8640

catgcgccat ctcatgtggc acatgaagcc tgctgctggt ccttactcac ctgtgtcagc 8700

agcgcacctg tgcagtatgg ctcgcctggg cacctttctt ctcaggatcc ctgtgtctgt 8760

ttcaccctga ccactgcttg aaaagccctc agactctcgc cagagaccgg ttgttctctg 8820

gtcacctcat ccggagacac aggctagctg ttccacttgc ttttggggtt ttgtttgttt 8880

ttcaagacag ggtctctctc tctctctctc tgtatagcca tgaatgtcct ggaactggct 8940

ttgtagacca gtctggcctc aaattcatag aagtctgcct gcctctgcct cccaagtgct 9000

gggattaaag acgtttataa ccacagccca gctgcctgat ctacttgcaa aaaggagttt 9060

ggtttttatt ccaagaactt gaatgccatt ggcccatgtt aggtcagaaa gtcaggagcc 9120

ctagagaaca gactagggaa ggagagggag ccaggcctcc tcatgggcac ctaaggggcg 9180

tgactaagag tttggaacct tctaacttct ggaagactgg cttctgtata ggatatgctc 9240

acagacctat gaagggcccc agctggctga ctcctcagag gtggagctgg ctacctctgc 9300

ctcatgggct gcagctcact gcaggcaggc ctggtaggcc aggagccaga ctcagagcag 9360

ccttgcagag ctgagtctgg tctttctcct tctcttttgg cctcaggtct cttaatgtcc 9420

tgaggctgcc agagctgagg gcagctttct ggggacttaa tgtcatagga atccatctgt 9480

ggatagtgac tggaccatag gtctgcagcc aaggagtgac agagccacac ccccttgccc 9540

caaggtgtgg ctcggtgccc ctcacagcct tgggaaccct tcattctgct ctaggccctc 9600

tgggcgttgc tgctgggatg tatctccatc ctcacagggc tatcgtccca tgtgactttt 9660

aagtgtccca cgacattttc ttcaggacca catcaaaccc accttagtgc cattacatct 9720

ggagagctcc tgtttccaaa tgaggttaca gtcacacatc ctgcacccca gtatcttggg 9780

ctctgggcta ctctgctgcc cacaggcgtt aggacaaatg gaaagcagag aactatgggc 9840

tcttgcccgt caggccggga tggcaaggct gggttcaacc tgcccagcag gcctgtctgt 9900

ccagctgctg tcttggtaac ctggtaccac tctgcctctg tacccagaca tcctgcagtt 9960

caacctgctg ggcgtgcccc tggtcggggc ggacatctgc ggcttcatag gagacacgtc 10020

agaagagctg tgtgtgcgct ggacccagtt gggggccttc taccccttca tgcggaacca 10080

caatgacctg aatagcgtgg taggacggag tggcagggag tccccaccca cctagaccct 10140

ctcctggtta atcccatacc tgctggaaga gctgtcgacg aattctaccg ggtaggggag 10200

gcgcttttcc caaggcagtc tggagcatgc gctttagcag ccccgctggg cacttggcgc 10260

tacacaagtg gcctctggcc tcgcacacat tccacatcca ccggtaggcg ccaaccggct 10320

ccgttctttg gtggcccctt cgcgccacct tctactcctc ccctagtcag gaagttcccc 10380

cccgccccgc agctcgcgtc gtgcaggacg tgacaaatgg aagtagcacg tctcactagt 10440

ctcgtgcaga tggacagcac cgctgagcaa tggaagcggg taggcctttg gggcagcggc 10500

caatagcagc tttgctcctt cgctttctgg gctcagaggc tgggaagggg tgggtccggg 10560

ggcgggctca ggggcgggct caggggcggg gcgggcgcga aggtcctccc gaggcccggc 10620

attctcgcac gcttcaaaag cgcacgtctg ccgcgctgtt ctcctcttcc tcatctccgg 10680

gcctttcgac ctgcagcgac ccgcttaaca gcgtcaacag cgtgccgcag atcttggtgg 10740

cgtgaaactc ccgcacctct tcggccagcg ccttgtagaa gcgcgtatgg cttcgtaccc 10800

ctgccatcaa cacgcgtctg cgttcgacca ggctgcgcgt tctcgcggcc ataacaaccg 10860

acgtacggcg ttgcgccctc gccggcaaca aaaagccacg gaagtccgcc tggagcagaa 10920

aatgcccacg ctactgcggg tttatataga cggtccccac gggatgggga aaaccaccac 10980

cacgcaactg ctggtggccc tgggttcgcg cgacgatatc gtctacgtac ccgagccgat 11040

gacttactgg cgggtgttgg gggcttccga gacaatcgcg aacatctaca ccacacaaca 11100

ccgcctcgac cagggtgaga tatcggccgg ggacgcggcg gtggtaatga caagcgccca 11160

gataacaatg ggcatgcctt atgccgtgac cgacgccgtt ctggctcctc atatcggggg 11220

ggaggctggg agctcacatg ccccgccccc ggccctcacc ctcatcttcg accgccatcc 11280

catcgccgcc ctcctgtgct acccggccgc gcgatacctt atgggcagca tgacccccca 11340

ggccgtgctg gcgttcgtgg ccctcatccc gccgaccttg cccggcacaa acatcgtgtt 11400

gggggccctt ccggaggaca gacacatcga ccgcctggcc aaacgccagc gccccggcga 11460

gcggcttgac ctggctatgc tggccgcgat tcgccgcgtt tatgggctgc ttgccaatac 11520

ggtgcggtat ctgcagggcg gcgggtcgtg gcgggaggat tggggacagc tttcgggggc 11580

ggccgtgccg ccccagggtg ccgagcccca gagcaacgcg ggcccacgac cccatatcgg 11640

ggacacgtta tttaccctgt ttcgggcccc cgagttgctg gcccccaacg gcgacctgta 11700

taacgtgttt gcctgggctt tggacgtctt ggccaaacgc ctccgtccca tgcatgtctt 11760

tatcctggat tacgaccaat cgcccgccgg ctgccgggac gccctgctgc aacttacctc 11820

cgggatggtc cagacccacg tcaccacccc aggctccata ccgacgatct gcgacctggc 11880

gcgcacgttt gcccgggaga tgggggaggc taactgaaac acggaaggag acaataccgg 11940

aaggaacccg cgctatgacg gcaataaaaa gacagaataa aacgcacggg tgttgggtcg 12000

tttgttcata aacgcggggt tcggtcccag ggctggcact ctgtcgatac cccaccgaga 12060

ccccattggg accaatacgc ccgcgtttct tccttttccc caccccaacc cccaagttcg 12120

ggtgaaggcc cagggctcgc agccaacgtc ggggcggcaa gccctgccat agccacgggc 12180

cccgtgggtt agggacgggg tcccccatgg ggaatggttt atggttcgtg ggggttatta 12240

ttttgggcgt tgcgtggggt caggtccacg actggactga gcagacagac ccatggtttt 12300

tggatggcct gggcatggac cgcatgtact ggcgcgacac gaacaccggg cgtctgtggc 12360

tgccaaacac ccccgacccc caaaaaccac cgcgcggatt tctggcgccg ccggacgaac 12420

taaacctgac tacggcatct ctgccccttc ttcgctggta cgaggagcgc ttttgttttg 12480

tattggtcac cacggccgag tttccgcggg accccggcca ggacctgcag aaattgatga 12540

tctattaaac aataaagatg tccactaaaa tggaagtttt tcctgtcata ctttgttaag 12600

aagggtgaga acagagtacc tacattttga atggaaggat tggagctacg ggggtggggg 12660

tggggtggga ttagataaat gcctgctctt tactgaaggc tctttactat tgctttatga 12720

taatgtttca tagttggata tcataattta aacaagcaaa accaaattaa gggccagctc 12780

attcctccca ctcatgatct atagatctat agatctctcg tgggatcatt gtttttctct 12840

tgattcccac tttgtggttc taagtactgt ggtttccaaa tgtgtcagtt tcatagcctg 12900

aagaacgaga tcagcagcct ctgttccaca tacacttcat tctcagtatt gttttgccaa 12960

gttctaattc catcagaagc ttggcactgg ccgtcgtttt acaacgtcgt gactgggaaa 13020

accctggcgt tacccaactt aatcgccttg cagcacatcc ccctttcgcc agctggcgta 13080

atagcgaaga ggcccgcacc gatcgccctt cccaacagtt gcgcagcctg aatggcgaat 13140

ggcgcctgat gcggtatttt ctccttacgc atctgtgcgg tatttcacac cgcatatggt 13200

gcactctcag tacaatctgc tctgatgccg catagttaag ccagccccga cacccgccaa 13260

cacccgctga cgcgccctga cgggcttgtc tgctcccggc atccgcttac agacaagctg 13320

tgaccgtctc cgggagctgc atgtgtcaga ggttttcacc gtcatcaccg aaacgcgcga 13380

gacgaaaggg cctcgtgata cgcctatttt tataggttaa tgtcatgata ataatggttt 13440

cttagacgtc aggtggcact tttcggggaa atgtgcgcgg aacccctatt tgtttatttt 13500

tctaaataca ttcaaatatg tatccgctca tgagacaata accctgataa atgcttcaat 13560

aatattgaaa aaggaagagt atgagtattc aacatttccg tgtcgccctt attccctttt 13620

ttgcggcatt ttgccttcct gtttttgctc acccagaaac gctggtgaaa gtaaaagatg 13680

ctgaagatca gttgggtgca cgagtgggtt acatcgaact ggatctcaac agcggtaaga 13740

tccttgagag ttttcgcccc gaagaacgtt ttccaatgat gagcactttt aaagttctgc 13800

tatgtggcgc ggtattatcc cgtattgacg ccgggcaaga gcaactcggt cgccgcatac 13860

actattctca gaatgacttg gttgagtact caccagtcac agaaaagcat cttacggatg 13920

gcatgacagt aagagaatta tgcagtgctg ccataaccat gagtgataac actgcggcca 13980

acttacttct gacaacgatc ggaggaccga aggagctaac cgcttttttg cacaacatgg 14040

gggatcatgt aactcgcctt gatcgttggg aaccggagct gaatgaagcc ataccaaacg 14100

acgagcgtga caccacgatg cctgtagcaa tggcaacaac gttgcgcaaa ctattaactg 14160

gcgaactact tactctagct tcccggcaac aattaataga ctggatggag gcggataaag 14220

ttgcaggacc acttctgcgc tcggcccttc cggctggctg gtttattgct gataaatctg 14280

gagccggtga gcgtgggtct cgcggtatca ttgcagcact ggggccagat ggtaagccct 14340

cccgtatcgt agttatctac acgacgggga gtcaggcaac tatggatgaa cgaaatagac 14400

agatcgctga gataggtgcc tcactgatta agcattggta actgtcagac caagtttact 14460

catatatact ttagattgat ttaaaacttc atttttaatt taaaaggatc taggtgaaga 14520

tcctttttga taatctcatg accaaaatcc cttaacgtga gttttcgttc cactgagcgt 14580

cagaccccgt agaaaagatc aaaggatctt cttgagatcc tttttttctg cgcgtaatct 14640

gctgcttgca aacaaaaaaa ccaccgctac cagcggtggt ttgtttgccg gatcaagagc 14700

taccaactct ttttccgaag gtaactggct tcagcagagc gcagatacca aatactgtcc 14760

ttctagtgta gccgtagtta ggccaccact tcaagaactc tgtagcaccg cctacatacc 14820

tcgctctgct aatcctgtta ccagtggctg ctgccagtgg cgataagtcg tgtcttaccg 14880

ggttggactc aagacgatag ttaccggata aggcgcagcg gtcgggctga acggggggtt 14940

cgtgcacaca gcccagcttg gagcgaacga cctacaccga actgagatac ctacagcgtg 15000

agctatgaga aagcgccacg cttcccgaag ggagaaaggc ggacaggtat ccggtaagcg 15060

gcagggtcgg aacaggagag cgcacgaggg agcttccagg gggaaacgcc tggtatcttt 15120

atagtcctgt cgggtttcgc cacctctgac ttgagcgtcg atttttgtga tgctcgtcag 15180

gggggcggag cctatggaaa aacgccagca acgcggcctt tttacggttc ctggcctttt 15240

gctggccttt tgctcacatg ttctttcctg cgttatcccc tgattctgtg gataaccgta 15300

ttaccgcctt tgagtgagct gataccgctc gccgcagccg aacgaccgag cgcagcgagt 15360

cagtgagcga ggaagcggaa gagcgcccaa tacgcaaacc gcctctcccc gcgcgttggc 15420

cgattcatta atgcagctgg cacgacaggt ttcccgactg gaaagcgggc agtgagcgca 15480

acgcaattaa tgtgagttag ctcactcatt aggcacccca ggctttacac tttatgcttc 15540

cggctcgtat gttgtgtgga attgtgagcg gataacaatt tcacacagga aacagctatg 15600

accatgatta cgccaacgct cgaaattaac cctcactaaa gggaacaaaa gctggagctc 15660

caccgcggt 15669